(19) Europäisches Patentamt
European Patent Office
Office européen des brevets

(11) EP 4 644 376 A1

(12) # EUROPEAN PATENT APPLICATION
published in accordance with Art. 153(4) EPC

(43) Date of publication:
**05.11.2025 Bulletin 2025/45**

(21) Application number: **24736960.6**

(22) Date of filing: **23.02.2024**

(51) International Patent Classification (IPC):
***C07D 215/56*** (2006.01) ***C07D 471/04*** (2006.01)
***A61K 31/47*** (2006.01) ***A61K 31/435*** (2006.01)
***A61P 31/00*** (2006.01) ***A61P 35/00*** (2006.01)

(52) Cooperative Patent Classification (CPC):
**A61K 31/435; A61K 31/444; A61K 31/47; A61K 31/4709; A61K 31/506; A61K 31/53; A61K 31/5377; A61K 31/541; A61P 31/00; A61P 31/04; A61P 35/00; A61P 35/02; C07D 215/56; C07D 401/04; C07D 417/04;** (Cont.)

(86) International application number:
**PCT/CN2024/078241**

(87) International publication number:
**WO 2024/141126 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.12.2022 CN 202211674621**
**25.12.2023 CN 202311795403**

(71) Applicant: **Alphamol Science Ltd (Shanghai)**
**Shanghai 201210 (CN)**

(72) Inventors:
- **YUAN, Shuguang**
**Shenzhen, Guangdong 518000 (CN)**
- **LI, Haijian**
**Shenzhen, Guangdong 518000 (CN)**
- **PAN, Qing**
**Shenzhen, Guangdong 518000 (CN)**
- **QIN, Taisheng**
**Shenzhen, Guangdong 518000 (CN)**

(74) Representative: **Vossius & Partner Patentanwälte Rechtsanwälte mbB**
**Siebertstraße 3**
**81675 München (DE)**

Remarks:
A request for restoration of the right of priority by the EPO as designated Office has been granted (R. 49ter.2 PCT, Art. 122 EPC).

(54) **NEW PHENYLQUINOLONE COMPOUND HAVING ANTIBACTERIAL AND ANTI-CANCER FUNCTIONS AND PREPARATION THEREOF**

(57) Disclosed in the present invention are a new phenylquinolone compound having antibacterial and anti-cancer functions and the preparation thereof, which belong to the technical field of medicines. Provided in the present invention is a phenylquinolone compound as represented by formula (I) having antibacterial and anti-cancer activities or a pharmaceutically acceptable salt, a stereoisomer, a deuterated compound or a prodrug thereof. Also provided in the present invention is an antibacterial composition containing one or more of the compounds of formula (I) or salts thereof. The present invention further comprises a method for treating and preventing bacterial infections and the growth of tumor cells by means of administering several effective doses of the compounds of formula (I) or salts thereof.

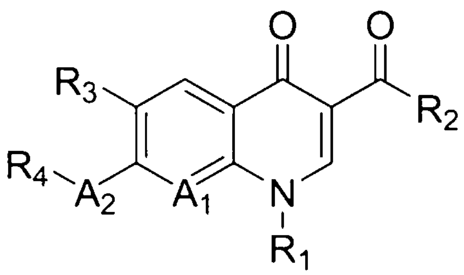

(I)

EP 4 644 376 A1

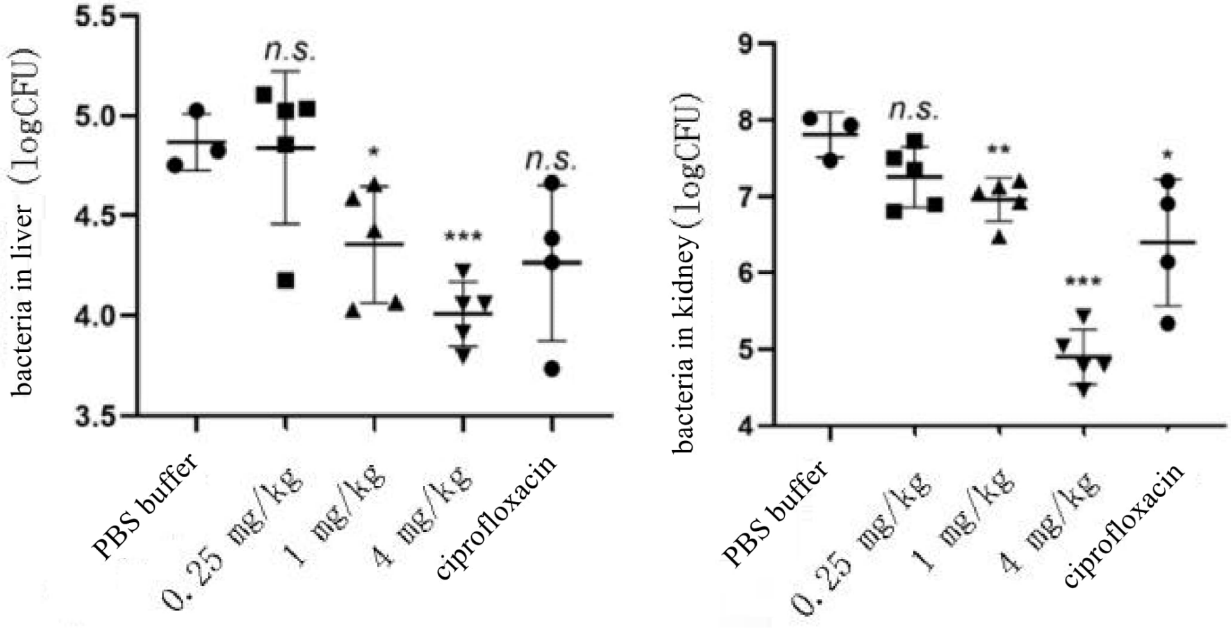


Fig. 4

(52) Cooperative Patent Classification (CPC): (Cont.)
**C07D 471/04**

## Description

### Technical Field

The present invention belongs to the field of medical technology, and specifically relates to a novel phenylquinolone compound with antibacterial and anticancer functions and the preparation thereof.

### Background

Since the first antibiotic, penicillin, was discovered, the industry of antibacterial has experienced rapid growth. However, resistance of bacteria resulting from the misuse of antibiotics, is emerging as one of the most serious threats to global public health.

About 30% of patients infected with drug-resistant *Staphylococcus aureus* (MRSA) eventually faces death, which is higher than the mortality rate of HIV. Carbapenem-resistant gram-negative super bacteria are listed as "high priority" pathogens, including *Acinetobacter baumannii, Pseudomonas aeruginosa*, and Enterobacter, while those listed as "high priority" are predominantly *Staphylococcus aureus* and Enterococci resistant to vancomycin or fluoroquinolone. It is reported that these multidrug-resistant (MDR) and extensively drug-resistant (XDR) bacteria have been be widely isolated, and they often exhibit insensitivity to multiple antibiotics, which not only leads to serious food safety concerns, but also has a serious impact on the effectiveness of anti-infection treatment for diseases. At present, the resistance of *Escherichia coli* and *Klebsiella pneumoniae* against ciprofloxacin, a fluoroquinolone antibiotic may reach 92.9% and 79.4%, respectively.

In addition, the mortality rate of cancer is also very high, and there is an urgent clinical need to provide an antibiotic that is effective for drug-resistant bacteria and suitable for cancer patients.

### Description of the invention

Description of the general formula

The present invention provides a phenylquinolone compound represented by formula (I), or a pharmaceutically acceptable salt, stereoisomer, deuteride, or prodrug thereof,

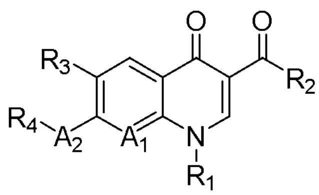

(I)

wherein

- $A_1$ is nitrogen atom N, or $CR_a$; in which $R_a$ is hydrogen atom, halogen atom (F, Cl, Br, I), C1-C3 alkyl, C1-C2 alkoxy, amino ($-NH_2$), nitro, or $R_1$ and $R_a$ are bonded to form a 6-membered heterocycle which is optionally substituted with short chain alkyl (C1-4 alkyl) or unsubstituted;
- $A_2$ is absent, methylene $-CH_2-$, alkenyl -CH=CH-, aminyl -NH- or oxygen atom -O-; when, $A_2$ is absent, then $R_4$-$A_2$- is $R_4$-;
- $R_1$ is hydrogen atom, cyano, C1-C2 short chain alkyl substituted with 0 or 2 halogen atom(s), C3-C6 cycloalkyl substituted with 0 or 1 halogen atom, phenyl substituted with 1 or 2 halogen atom(s), or 2-pyridinyl substituted with 1 to 3 substituent(s) selected from: halogen atom or amino ($-NH_2$); or, $R_1$ and $R_a$ are bonded to form a 6-membered heterocycle which is optionally substituted with short chain alkyl (C1-4 alkyl) or unsubstituted;
- $R_2$ is hydroxy, C1-C2 alkoxy, or $-NR_bR_c$; in which $R_b$, $R_c$ are each independently selected from hydrogen atom, C1-C2 alkyl or hydroxy, and are not both hydroxy;
- $R_3$ is halogen, nitro or C1-C2 alkoxy;
- $R_4$ is selected from:

  substituted or unsubstituted aryl; the substitution is mono- to tetra-substitution, including combinations of one or more of ortho-, meta- and para-substitutions; the substituent is selected from: halogen -X (F, Cl, Br, I), nitro $-NO_2$,

cyano -CN, hydroxylaminyl -NH-OH, C1-C3 alkoxy substituted with 0 to 3 halogen atom(s), C1-C9 alkyl substituted with 0 to 3 halogen atom(s), sulfinyl -SO-$R_d$, aminylsulfinyl -SO-$NR_eR_e'$, sulfonyl -$SO_2$-$R_f$, aminylsulfonyl -$SO_2$-$NR_gR_g'$, methyliminosulfinyl - S(=O)(=$NR_h$)$CH_3$, sulfenyl -S-$R_i$, aminyl -$NR_jR_j'$, carbamoyl -CO-$NR_kR_k'$, hydrazinyl -NH-$NR_lR_l'$, diazenyl -N=$NR_mR_m'$;

wherein, $R_d$ is selected from: C1-C9 linear or branched alkyl, C3-C6 cycloalkyl, halo C1-C9 alkyl, C1-C9 alkyl substituted with hydroxy, C1-C9 alkyl substituted with amino (-$NH_2$), C1-C9 alkyl substituted with -$SR_d''$, C2-C9 alkenyl, C2-C9 alkynyl, morpholine, piperazine, piperidine, $OR_d'$; $R_d'$, $R_d''$ are each independently selected from: hydrogen atom, C1-C9 linear or branched alkyl, C3-C6 cycloalkyl, or halo C1-C9 alkyl; C atom in the carbon chain of alkyl may be replaced by heteroatom (N, O, S) at any position as normal valence permits;

$R_e$, $R_e'$ are each independently selected from: hydrogen atom, C1-C9 linear or branched alkyl, C3-C6 cycloalkyl, halo C1-C9 alkyl, C1-C9 alkyl substituted with hydroxy, C1-C9 alkyl substituted with amino (-$NH_2$), C1-C9 alkyl substituted with-$SR_e''$, C2-C9 alkenyl, C2-C9 alkynyl, C1-C5 acyl (H or C1-C4 alkyl)-C(=O)-, halo C2-C5 acyl or C2-C5 alkoxycarbonyl; $R_e''$ is hydrogen atom, C1-C9 linear or branched alkyl, C3-C6 cycloalkyl, or halo C1-C9 alkyl; C atom in the carbon chain of alkyl may be replaced by heteroatom (N, O, S) at any position as normal valence permits;

$R_f$ is selected from: C1-C9 linear or branched alkyl, C3-C6 cycloalkyl, halo C1-C9 alkyl, C1-C9 alkyl substituted with hydroxy, C1-C9 alkyl substituted with amino (-$NH_2$), C1-C9 alkyl substituted with -$SR_f''$, morpholine, piperazine, piperidine, $OR_f'$; $R_f'$, $R_f''$ are each independently selected from: hydrogen atom, C1-C9 linear or branched alkyl, C3-C6 cycloalkyl, or halo C1-C9 alkyl; C atom in the carbon chain of alkyl may be replaced by heteroatom at any position as normal valence permits;

$R_g$, $R_g'$ are each independently selected from: hydrogen atom, C1-C25 linear or branched alkyl containing 0-10 unsaturated bond(s), C3-C6 cycloalkyl, halo C1-C9 alkyl, C1-C9 alkyl substituted with hydroxy, C1-C9 alkyl substituted with amino (-$NH_2$), C1-C9 alkyl substituted with -$SR_g''$, C2-C25alkenyl, C2-C9 alkynyl, C1-C5 acyl (H or C1-C4 alkyl)-C(=O)-, halo C2-C5 acyl or C2-C5 alkoxycarbonyl; $R_g''$ is hydrogen atom, C1-C9 linear or branched alkyl; C atom in the carbon chain of alkyl at any position may be replaced by heteroatom as normal valence permits; or-$NR_gR_g'$ is

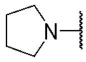,

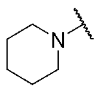, 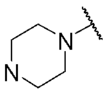, 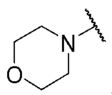, 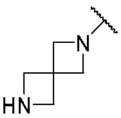 ;

$R_h$ is selected from: hydrogen atom, C1-C9 linear or branched alkyl containing 0-10 unsaturated bond(s), C3-C6 cycloalkyl, halo C1-C9 alkyl, C2-C9 alkenyl, C2-C9 alkynyl, C1-C5 acyl (H or C1-C4 alkyl)-C(=O)-, halo C2-C5 acyl or C2-C5 alkoxycarbonyl; C atom in the carbon chain of alkyl may be replaced by heteroatom at any position as normal valence permits;

$R_i$ is selected from: hydrogen atom, C1-C9 linear or branched alkyl, C3-C6 cycloalkyl, halo C1-C9 alkyl, C1-C9 alkyl substituted with hydroxy, C1-C5 acyl, halo C2-C5 acyl, -$NR_i'R_i''$; $R_i'$, $R_i''$ are each independently selected from hydrogen atom, C1-C9 linear or branched alkyl, C3-C6 cycloalkyl, halo C1-C9 alkyl, C1-C5 acyl (H or C1-C4 alkyl)-C(=O)-, halo C2-C5 acyl or C2-C5 alkoxycarbonyl; C atom in the carbon chain of alkyl may be replaced by heteroatom at any position as normal valence permits;

$R_j$, $R_j'$ are each independently selected from: hydrogen atom, C-C25 linear or branched alkyl containing 0-10 unsaturated bond(s), C3-C6 cycloalkyl, halo C1-C9 alkyl, C2-C9 alkenyl, C2-C9 alkynyl, C1-C25 acyl (H, C2-C9 alkenyl, or C1-C24 alkyl containing 0-10 unsaturated bond(s))-C(=O)- , halo C2-C5 acyl, C1-C3 alkoxycarbonyl, C1-C25 alkylsulfonyl or C2-C25 alkenyl sulfonyl; C atom in the carbon chain of alkyl may be replaced by heteroatom at any position as normal valence permits;

$R_k$, $R_k'$ are each independently selected from: hydrogen atom, C1-C9 linear or branched alkyl, C3-C6 cycloalkyl, halo C1-C9 alkyl, C1-C9 alkyl substituted with hydroxy, C2-C9 alkenyl, C2-C9 alkynyl, hydroxy, C1-C5 acyl (H or C1-C4 alkyl)-C(=O)-, halo C2-C5 acyl, C2-C5 alkoxycarbonyl or C1-C9 alkylsulfonyl; C atom in the carbon chain of alkyl may be replaced by heteroatom at any position as normal valence permits;

$R_l$, $R_l'$ are each independently selected from: hydrogen atom, C1-C9 linear or branched alkyl, C3-C6 cycloalkyl, halo C1-C9 alkyl, C2-C9 alkenyl, C2-C9 alkynyl, hydroxy, C1-C5 acyl (H or C1-C4 alkyl)-C(=O)-, halo C2-C5 acyl, or C2-C5 alkoxycarbonyl; C atom in the carbon chain of alkyl may be replaced by heteroatom at any position as normal valence permits;

$R_m$, $R_m$' are each independently selected from: hydrogen atom, phenyl, C1-C9 linear or branched alkyl, C3-C6 cycloalkyl, halo C1-C9 alkyl, C2-C9 alkenyl, C2-C9 alkynyl, hydroxy, C1-C5 acyl (H or C1-C4 alkyl)-C(=O)-, halo C2-C5 acyl, or C2-C5 alkoxycarbonyl; C atom in the carbon chain of alkyl may be replaced by heteroatom at any position as normal valence permits;

the aryl is selected from: phenyl, 2-pyridinyl, 3-pyridinyl, 4-pyridinyl,

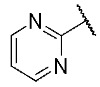 , 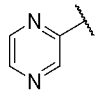 ,

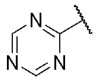 , 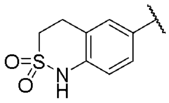 , 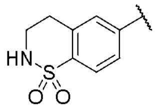 , 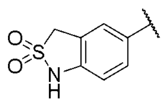 , 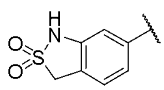 , 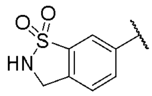 ,

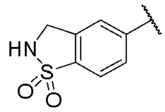 .

In an embodiment of the present invention, at least one substituent on the aryl in $R_4$ is selected from sulfinyl -SO-$R_d$, aminylsulfinyl -SO-$NR_eR_e$', sulfonyl -$SO_2$-$R_f$, aminylsulfonyl -$SO_2$-$NR_gR_g$', methyliminosulfinyl -S(=O)(=$NR_h$)$CH_3$, aminyl -$NR_jR_j$', hydrazinyl -NH-$NR_lR_l$', diazenyl - N=$NR_mR_m$'; wherein, $R_j$ is hydrogen atom, $R_j$' is halo C2-C5 acyl, C1-C25 alkylsulfonyl.

In an embodiment of the present invention, at least one substituent on the aryl in $R_4$ is selected from sulfinyl -SO-$R_d$, aminylsulfinyl -SO-$NR_eR_e$', sulfonyl -$SO_2$-$R_f$, aminylsulfonyl -$SO_2$-$NR_gR_g$', methyliminosulfinyl -S(=O)(=$NR_h$)$CH_3$, aminyl -$NR_jR_j$', hydrazinyl -NH-$NR_lR_l$', diazenyl - N=$NR_mR_m$'; wherein, $R_j$, $R_j$' are each independently selected from hydrogen atom, halo C2-C5 acyl, C1-C25 alkylsulfonyl, and $R_j$, $R_j$' are not both H.

In an embodiment of the present invention, $R_4$ is mono-substituted aryl, the substituent is selected from sulfinyl -SO-$R_d$, aminylsulfinyl -SO-$NR_eR_e$', sulfonyl -$SO_2$-$R_f$, aminylsulfonyl -$SO_2$-$NR_gR_g$', methyliminosulfinyl -S(=O)(=$NR_h$)$CH_3$, aminyl -$NR_jR_j$', hydrazinyl -NH-$NR_lR_l$', diazenyl - N=$NR_mR_m$';

alternatively, $R_4$ is aryl substituted with 2 to 4 substituents, at least one substituent is selected from sulfinyl-SO-$R_d$, aminylsulfinyl -SO-$NR_eR_e$', sulfonyl -$SO_2$-$R_f$, aminylsulfonyl -$SO_2$-$NR_gR_g$', methyliminosulfinyl -S(=O)(=$NR_h$)$CH_3$, aminyl -$NR_jR_j$', hydrazinyl -NH-$NR_lR_l$', diazenyl - N=$NR_mR_m$';

wherein, $R_j$, $R_j$' are each independently selected from hydrogen atom, halo C2-C5 acyl, C1-C25 alkylsulfonyl, and $R_j$, $R_j$' are not both H.

In an embodiment of the present invention, for $R_4$, the aryl is 4-pyridinyl, or

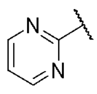 ;

and at least one substituent on the aryl is carbamoyl-CO-$NR_kR_k$'.

In an embodiment of the present invention, $R_4$ is specifically selected from:

(1) phenyl of formula II:

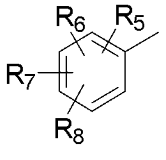 (II)

wherein $R_5$ is hydrogen atom, or a halogen atom, sulfinyl -SO-$R_d$, aminylsulfinyl -SO-$NR_eR_e$', sulfonyl -$SO_2$-$R_f$, aminylsulfonyl -$SO_2$-$NR_gR_g$', methyliminosulfinyl -S(=O)(=$NR_h$)$CH_3$, aminyl - $NR_jR_j$', hydrazinyl -NH-$NR_lR_l$',

diazenyl -N=$NR_mR_m$';
$R_6$, $R_7$ and $R_8$ are each independently selected from:

(a) hydrogen atom;
(b) halogen atom;
(c) nitro;
(d) cyano;
(e) hydroxylaminyl;
(f) C1-C3 alkoxy substituted with 0 to 3 halogen atom(s);
(g) C1-C9 alkyl substituted with 0 to 3 halogen atom(s);
(h) sulfinyl optionally substituted with C1-C9 linear or branched alkyl, C3-C6 cycloalkyl, C1-C9 haloalkyl, C1-C9 alkyl substituted with hydroxy, C1-C9 alkyl substituted with amino (-$NH_2$), C1-C9 alkyl substituted with -$SR_d$", C2-C9 alkenyl, C2-C9 alkynyl, morpholine, piperazine, piperidine, or $OR_d$'; wherein, $R_d$', $R_d$" are each independently selected from: hydrogen atom, C1-C9 linear or branched alkyl, C3-C6 cycloalkyl, or C1-C9 haloalkyl; C atom in the carbon chain of alkyl may be replaced by heteroatom at any position as normal valence permits;
(i) aminylsulfinyl optionally substituted with hydrogen atom, C1-C9 linear or branched alkyl, C3-C6 cycloalkyl, C1-C9 haloalkyl, C1-C9 alkyl substituted with hydroxy, C1-C9 alkyl substituted with amino (-$NH_2$), C1-C9 alkyl substituted with -$SR_e$", C2-C9 alkenyl, C2-C9 alkynyl, C1-C5 acyl, C2-C5 haloacyl or C2-C5 alkoxycarbonyl; $R_e$" is hydrogen atom, C1-C9 linear or branched alkyl, C3-C6 cycloalkyl, or halo C1-C9 alkyl; wherein, C atom in the carbon chain of alkyl may be replaced by heteroatom at any position as normal valence permits;
(j) sulfonyl optionally substituted with C1-C9 linear or branched alkyl, C3-C6 cycloalkyl, C1-C9 haloalkyl, C1-C9 alkyl substituted with hydroxy, C1-C9 alkyl substituted with amino (-$NH_2$), C1-C9 alkyl substituted with -$SR_f$", morpholine, piperazine, piperidine, or $OR_f$'; wherein, $R_f$', $R_f$" are each independently selected from: hydrogen atom, C1-C9 linear or branched alkyl, C3-C6 cycloalkyl, or C1-C9 haloalkyl; C atom in the carbon chain of alkyl may be replaced by heteroatom at any position as normal valence permits;
(k) aminylsulfonyl optionally substituted with hydrogen atom, C1-C25 linear or branched alkyl containing 0-10 unsaturated bond(s), C3-C6 cycloalkyl, C1-C9 haloalkyl, C1-C9 alkyl substituted with hydroxy, C1-C9 alkyl substituted with amino (-$NH_2$), C1-C9 alkyl substituted with -$SR_g$", C2-C25 alkenyl, C2-C9 alkynyl, C1-C5 acyl, C2-C5 haloacyl or C2-C5 alkoxycarbonyl; $R_g$" is hydrogen atom, C1-C9 linear or branched alkyl; wherein, C atom in the carbon chain of alkyl may be replaced by heteroatom at any position as normal valence permits; or-$NR_gR_g$' is

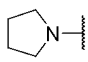, 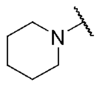, 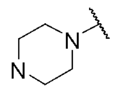,

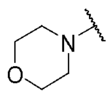, 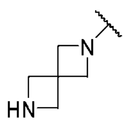;

(l) methyliminosulfinyl optionally substituted with hydrogen atom, C1-C9 linear or branched alkyl containing 0-10 unsaturated bond(s), C3-C6 cycloalkyl, C1-C9 haloalkyl, C2-C9 alkenyl, C2-C9 alkynyl, C1-C5 acyl, C2-C5 haloacyl or C2-C5 alkoxycarbonyl; wherein, C atom in the carbon chain of alkyl may be replaced by heteroatom at any position as normal valence permits;
(m) sulfenyl optionally substituted with hydrogen atom, C1-C9 linear or branched alkyl, C3-C6 cycloalkyl, C1-C9 haloalkyl, C1-C9 alkyl substituted with hydroxy, C1-C5 acyl, C2-C5 haloacyl or - $NR_i$'$R_i$"; wherein $R_i$', $R_i$" are each independently selected from hydrogen atom, C1-C9 linear or branched alkyl, C3-C6 cycloalkyl, C1-C9 haloalkyl, C1-C5 acyl, C2-C5 haloacyl or C2-C5 alkoxycarbonyl; C atom in the carbon chain of alkyl may be replaced by heteroatom at any position as normal valence permits;
(n) aminyl optionally substituted with hydrogen atom, C1-C25 linear or branched alkyl containing 0-10 unsaturated bond(s), C3-C6 cycloalkyl, C1-C9 haloalkyl, C2-C9 alkenyl, C2-C9 alkynyl, C1-C25 acyl (H, C2-C9 alkenyl, or C1-C24 alkyl containing 0-10 unsaturated bond(s))-C(=O)- , C2-C5 haloacyl, C1-C3 alkoxycarbonyl, C1-C25 alkylsulfonyl or C2-C25 alkenyl sulfonyl;
(o) carbamoyl optionally substituted with hydrogen atom, C1-C9 linear or branched alkyl, C3-C6 cycloalkyl,

C1-C9 haloalkyl, C2-C9 alkenyl, C2-C9 alkynyl, hydroxy, C1-C5 acyl, C2-C5 haloacyl, C2-C5 alkoxycarbonyl or C1-C9 alkylsulfonyl; wherein, C atom in the carbon chain of alkyl may be replaced by heteroatom at any position as normal valence permits;
(p) hydrazinyl;
(q) diazenyl;

**(2) 2-pyridinyl, substituted with 1 or 2 substituent(s) selected from:**

(a) hydrogen atom;
(b) halogen atom;
(c) hydroxylaminyl;
(d) sulfonyl optionally substituted with C1-C9 linear or branched alkyl, C3-C6 cycloalkyl, C1-C9 haloalkyl, C1-C9 alkyl substituted with hydroxy, C1-C9 alkyl substituted with amino ($-NH_2$), C1-C9 alkyl substituted with $-SR_f''$, morpholine, piperazine, piperidine, or $OR_f'$; wherein, $R_f'$, $R_f''$ are each independently selected from: hydrogen atom, C1-C9 linear or branched alkyl, C3-C6 cycloalkyl, or C1-C9 haloalkyl; C atom in the carbon chain of alkyl may be replaced by heteroatom at any position as normal valence permits;
(e) aminylsulfonyl optionally substituted with hydrogen atom, C1-C25 linear or branched alkyl containing 0-10 unsaturated bond(s), C3-C6 cycloalkyl, C1-C9 haloalkyl, C1-C9 alkyl substituted with hydroxy, C1-C9 alkyl substituted with amino ($-NH_2$), C1-C9 alkyl substituted with $-SR_g''$, C2-C25 alkenyl, C2-C9 alkynyl, C1-C5 acyl, C2-C5 haloacyl or C2-C5 alkoxycarbonyl; $R_g''$ is hydrogen atom, C1-C9 linear or branched alkyl; wherein, C atom in the carbon chain of alkyl may be replaced by heteroatom at any position as normal valence permits; or-$NR_gR_g'$ is

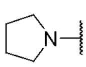, 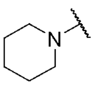, 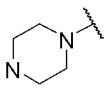,

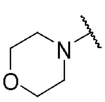, 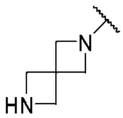;

(f) methyliminosulfinyl optionally substituted with hydrogen atom, C1-C9 linear or branched alkyl containing 0-10 unsaturated bond(s), C3-C6 cycloalkyl, C1-C9 haloalkyl, C2-C9 alkenyl, C2-C9 alkynyl, C1-C5 acyl, C2-C5 haloacyl or C2-C5 alkoxycarbonyl; wherein, C atom in the carbon chain of alkyl may be replaced by heteroatom at any position as normal valence permits;
(g) sulfenyl optionally substituted with hydrogen atom, C1-C9 linear or branched alkyl, C3-C6 cycloalkyl, C1-C9 haloalkyl, C1-C9 alkyl substituted with hydroxy, C1-C5 acyl, C2-C5 haloacyl or - $NR_i'R_i''$; wherein $R_i'$, $R_i''$ are each independently selected from hydrogen atom, C1-C9 linear or branched alkyl, C3-C6 cycloalkyl, C1-C9 haloalkyl, C1-C5 acyl, C2-C5 haloacyl or C2-C5 alkoxycarbonyl; C atom in the carbon chain of alkyl may be replaced by heteroatom at any position as normal valence permits;
(h) aminyl optionally substituted with hydrogen atom, C1-C25 linear or branched alkyl containing 0-10 unsaturated bond(s), C3-C6 cycloalkyl, C1-C9 haloalkyl, C2-C9 alkenyl, C2-C9 alkynyl, C1-C25 acyl (H, C2-C9 alkenyl, or C1-C24 alkyl containing 0-10 unsaturated bond(s))-C(=O)-, C2-C5 haloacyl, C1-C3 alkoxycarbonyl, C1-C25 alkylsulfonyl or C2-C25 alkenyl sulfonyl;
(i) carbamoyl optionally substituted with hydrogen atom, C1-C9 linear or branched alkyl, C3-C6 cycloalkyl, C1-C9 haloalkyl, C2-C9 alkenyl, C2-C9 alkynyl, hydroxy, C1-C5 acyl, C2-C5 haloacyl, C2-C5 alkoxycarbonyl or C1-C9 alkylsulfonyl; wherein, C atom in the carbon chain of alkyl may be replaced by heteroatom at any position as normal valence permits;
(j) hydrazinyl;
(k) diazenyl;

alternatively, at least one substituent is selected from sulfinyl $-SO-R_d$, aminylsulfinyl $-SO-NR_eR_e'$, sulfonyl $-SO_2-R_f$, aminylsulfonyl $-SO_2-NR_gR_g'$, methyliminosulfinyl $-S(=O)(=NR_h)CH_3$, aminyl - $NR_jR_j'$, hydrazinyl $-NH-NR_lR_l'$, diazenyl $-N=NR_mR_m'$;
**(3) 3-pyridinyl, substituted with 1 or 2 substituent(s) selected from:**

(a) hydrogen atom;
(b) halogen atom;
(c) hydroxylaminyl;
(h) sulfinyl optionally substituted with C1-C9 linear or branched alkyl, C3-C6 cycloalkyl, C1-C9 haloalkyl, C1-C9 alkyl substituted with hydroxy, C1-C9 alkyl substituted with amino ($-NH_2$), C1-C9 alkyl substituted with $-SR_d$", C2-C9 alkenyl, C2-C9 alkynyl, morpholine, piperazine, piperidine, or $OR_d$'; wherein, $R_d$', $R_d$" are each independently selected from: hydrogen atom, C1-C9 linear or branched alkyl, C3-C6 cycloalkyl, or C1-C9 haloalkyl; C atom in the carbon chain of alkyl may be replaced by heteroatom at any position as normal valence permits;
(i) aminylsulfinyl optionally substituted with hydrogen atom, C1-C9 linear or branched alkyl, C3-C6 cycloalkyl, C1-C9 haloalkyl, C1-C9 alkyl substituted with hydroxy, C1-C9 alkyl substituted with amino ($-NH_2$), C1-C9 alkyl substituted with $-SR_e$", C2-C9 alkenyl, C2-C9 alkynyl, C1-C5 acyl, C2-C5 haloacyl or C2-C5 alkoxycarbonyl; $R_e$" is hydrogen atom, C1-C9 linear or branched alkyl, C3-C6 cycloalkyl, or halo C1-C9 alkyl; wherein, C atom in the carbon chain of alkyl may be replaced by heteroatom at any position as normal valence permits;
(d) sulfonyl optionally substituted with C1-C9 linear or branched alkyl, C3-C6 cycloalkyl, C1-C9 haloalkyl, C1-C9 alkyl substituted with hydroxy, C1-C9 alkyl substituted with amino ($-NH_2$), C1-C9 alkyl substituted with $-SR_f$", morpholine, piperazine, piperidine, or $OR_f$'; wherein, $R_f$', $R_f$" are each independently selected from: hydrogen atom, C1-C9 linear or branched alkyl, C3-C6 cycloalkyl, or C1-C9 haloalkyl; C atom in the carbon chain of alkyl may be replaced by heteroatom at any position as normal valence permits;
(e) aminylsulfonyl optionally substituted with hydrogen atom, C1-C25 linear or branched alkyl containing 0-10 unsaturated bond(s), C3-C6 cycloalkyl, C1-C9 haloalkyl, C1-C9 alkyl substituted with hydroxy, C1-C9 alkyl substituted with amino ($-NH_2$), C1-C9 alkyl substituted with $-SR_g$", C2-C25 alkenyl, C2-C9 alkynyl, C1-C5 acyl, C2-C5 haloacyl or C2-C5 alkoxycarbonyl; $R_g$" is hydrogen atom, C1-C9 linear or branched alkyl; wherein, C atom in the carbon chain of alkyl may be replaced by heteroatom at any position as normal valence permits; or-$NR_gR_g$' is

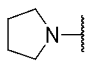, 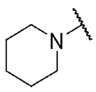, 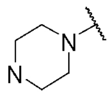,

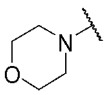, 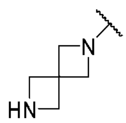;

(f) methyliminosulfinyl optionally substituted with hydrogen atom, C1-C9 linear or branched alkyl containing 0-10 unsaturated bond(s), C3-C6 cycloalkyl, C1-C9 haloalkyl, C2-C9 alkenyl, C2-C9 alkynyl, C1-C5 acyl, C2-C5 haloacyl or C2-C5 alkoxycarbonyl; wherein, C atom in the carbon chain of alkyl may be replaced by heteroatom at any position as normal valence permits;
(k) sulfenyl optionally substituted with hydrogen atom, C1-C9 linear or branched alkyl, C3-C6 cycloalkyl, C1-C9 haloalkyl, C1-C9 alkyl substituted with hydroxy, C1-C5 acyl, C2-C5 haloacyl or - $NR_i$'$R_i$"; wherein $R_i$', $R_i$" are each independently selected from hydrogen atom, C1-C9 linear or branched alkyl, C3-C6 cycloalkyl, C1-C9 haloalkyl, C1-C5 acyl, C2-C5 haloacyl or C2-C5 alkoxycarbonyl; C atom in the carbon chain of alkyl may be replaced by heteroatom at any position as normal valence permits;
(h) aminyl optionally substituted with hydrogen atom, C1-C25 linear or branched alkyl containing 0-10 unsaturated bond(s), C3-C6 cycloalkyl, C1-C9 haloalkyl, C2-C9 alkenyl, C2-C9 alkynyl, C1-C25 acyl (H, C2-C9 alkenyl or C1-C24 alkyl containing 0-10 unsaturated bond(s))-C(=O)-, C2-C5 haloacyl, C1-C3 alkoxycarbonyl, C1-C25 alkylsulfonyl or C2-C25 alkenylsulfonyl;
(i) carbamoyl optionally substituted with hydrogen atom, C1-C9 linear or branched alkyl, C3-C6 cycloalkyl, C1-C9 haloalkyl, C2-C9 alkenyl, C2-C9 alkynyl, hydroxy, C1-C5 acyl, C2-C5 haloacyl, C2-C5 alkoxycarbonyl or C1-C9 alkylsulfonyl; wherein, C atom in the carbon chain of alkyl may be replaced by heteroatom at any position as normal valence permits;
(j) hydrazinyl;
(k) diazenyl;

alternatively, at least one substituent is selected from sulfinyl $-SO-R_d$, aminylsulfinyl $-SO-NR_eR_e$', sulfonyl $-SO_2-R_f$, aminylsulfonyl $-SO_2-NR_gR_g$', methyliminosulfinyl $-S(=O)(=NR_h)CH_3$, aminyl - $NR_jR_j$', hydrazinyl $-NH-NR_lR_l$', diazenyl $-N=NR_mR_m$';

**(4) 4-pyridinyl, substituted with 1 or 2 substituent(s) selected from:**

(a) hydrogen atom;
(b) halogen atom;
(c) hydroxylaminyl;
(h) aminyl optionally substituted with C1-C25 alkyl, C3-C6 cycloalkyl, C1-C9 haloalkyl, C2-C9 alkenyl, C2-C9 alkynyl, C1-C25 acyl (H, C2-C9 alkenyl or C1-C24 alkyl containing 0-10 unsaturated bond(s))-C(=O)-, C2-C5 haloacyl, C1-C3 alkoxycarbonyl, C1-C25 alkylsulfonyl or C2-C25 alkenylsulfonyl;
(i) carbamoyl optionally substituted with hydrogen atom, C1-C9 linear or branched alkyl, C3-C6 cycloalkyl, C1-C9 haloalkyl, C2-C9 alkenyl, C2-C9 alkynyl, hydroxy, C1-C5 acyl, C2-C5 haloacyl, C2-C5 alkoxycarbonyl or C1-C9 alkylsulfonyl; wherein, C atom in the carbon chain of alkyl may be replaced by heteroatom at any position as normal valence permits;
(j) hydrazinyl;
(k) diazenyl;
alternatively, at least one substituent is selected from sulfinyl -SO-$R_d$, aminylsulfinyl -SO-$NR_eR_e$’, sulfonyl -$SO_2$-$R_f$, aminylsulfonyl -$SO_2$-$NR_gR_g$’, methyliminosulfinyl -S(=O)(=$NR_h$)$CH_3$, aminyl - $NR_jR_j$’, hydrazinyl -NH-$NR_lR_l$’, diazenyl -N=$NR_mR_m$’;

**(5) the groups of the following formulae:**

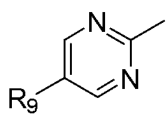
(III)

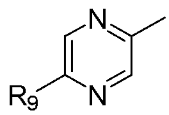
(IV)

wherein, $R_9$ is selected from:

(a) hydrogen atom;
(b) halogen atom;
(c) hydroxylaminyl;
(h) aminyl optionally substituted with hydrogen atom, C1-C25 linear or branched alkyl containing 0-10 unsaturated bond(s), C3-C6 cycloalkyl, C1-C9 haloalkyl, C2-C9 alkenyl, C2-C9 alkynyl, C1-C25 acyl (H, C2-C9 alkenyl, or C1-C24 alkyl containing 0-10 unsaturated bond(s))-C(=O)-, C2-C5 haloacyl, C1-C3 alkoxycarbonyl, C1-C25 alkylsulfonyl or C2-C25 alkenylsulfonyl;
(i) carbamoyl optionally substituted with hydrogen atom, C1-C9 linear or branched alkyl, C3-C6 cycloalkyl, C1-C9 haloalkyl, C2-C9 alkenyl, C2-C9 alkynyl, hydroxy, C1-C5 acyl, C2-C5 haloacyl, C2-C5 alkoxycarbonyl or C1-C9 alkylsulfonyl; wherein, C atom in the carbon chain of alkyl may be replaced by heteroatom at any position as normal valence permits;
(j) hydrazinyl;
(k) diazenyl;

alternatively, at least one substituent is selected from sulfinyl -SO-$R_d$, aminylsulfinyl -SO-$NR_eR_e$’, sulfonyl -$SO_2$-$R_f$, aminylsulfonyl -$SO_2$-$NR_gR_g$’, methyliminosulfinyl -S(=O)(=$NR_h$)$CH_3$, aminyl - $NR_jR_j$’, hydrazinyl -NH-$NR_lR_l$’, diazenyl -N=$NR_mR_m$’;
**(6) triazinyl:**

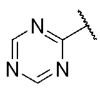
;

**(7) the groups of the following formulae:**

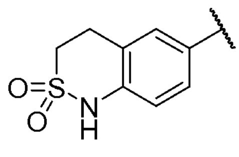 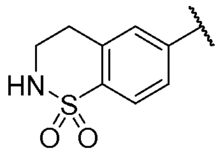

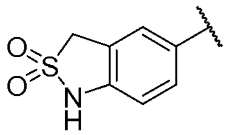 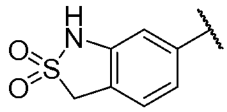 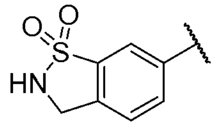 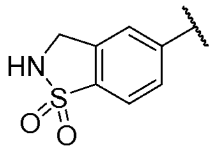 .

In an embodiment of the present invention, the C3-C6 cycloalkyl in $R_4$ may be further selected from C4-C5 heterocycloalkyl; specifically, for example:

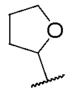 , 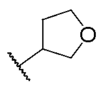 , 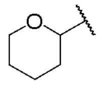 , 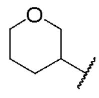 , 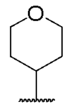 .

In an embodiment of the present invention, the number of heteroatom(s) in the 6-membered heterocycle is 1-4; the heteroatom is one or more of N, O, S. Specifically, the 6-membered heterocycle may be selected from:

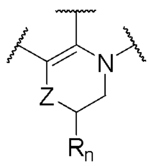 , 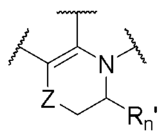 ;

wherein, Z is O or S; $R_n$, $R_n$' are each independently selected from C1-C4 alkyl, halogen.

In an embodiment of the present invention, the unsaturated bond includes carbon-carbon double bond and/or carbon-carbon triple bond.

In an embodiment of the present invention, said C2-C9 alkenyl refers to C2-C9 alkenyl containing 1-5 unsaturated double bond(s), for example, (H or C1-C7 alkyl containing 0-4 unsaturated double bond(s))-CH=CH-.

In an embodiment of the present invention, said C2-C25 alkenyl refers to C2-C25 alkenyl containing 1-10 unsaturated double bond(s), for example, (H or C1-C23 alkyl containing 0-9 unsaturated double bond(s))-CH=CH-.

In an embodiment of the present invention, said C2-C9 alkynyl refers to C2-C9 alkynyl containing 1-3 unsaturated bond(s), for example, (H or C1-C7 alkyl containing 0-2 unsaturated bond(s))-C≡C-. The unsaturated bond includes carbon-carbon double bond and/or carbon-carbon triple bond.

In an embodiment of the present invention, said C1-C5 acyl refers to (H or C1-C4 alkyl)-C(=O)-

In an embodiment of the present invention, said halo C2-C5 acyl refers to (halo C1-C4 alkyl)-C(=O)-.

In an embodiment of the present invention, said C2-C5 alkoxycarbonyl refers to (C2-C5 alkyl)-O-C(=O)-.

In an embodiment of the present invention, C1-C25 acyl refers to (H or C1-C24 alkyl containing 0-10 unsaturated double bond(s))-C(=O)-.

In an embodiment of the present invention, said C1-C25 alkylsulfonyl refers to (C1-C25 alkyl containing 0-10 unsaturated bond(s))-$SO_2$-.

In an embodiment of the present invention, said C2-C25 alkenylsulfonyl refers to (C2-C25 alkenyl containing 1-10 unsaturated double bond(s))-$SO_2$-.

In an embodiment of the present invention, the phenylquinolone compound further includes:

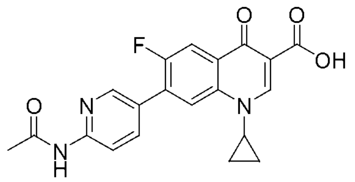 , 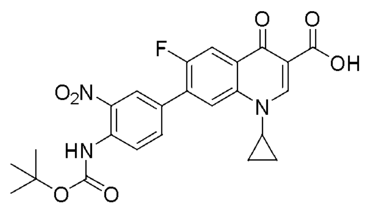 , 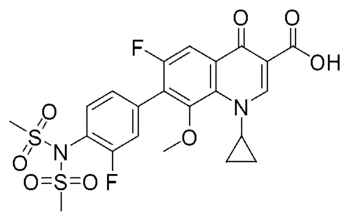 ,

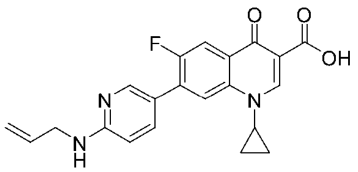 , 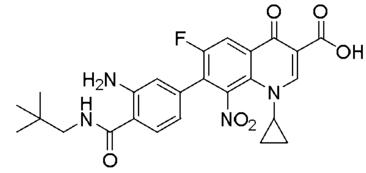 , 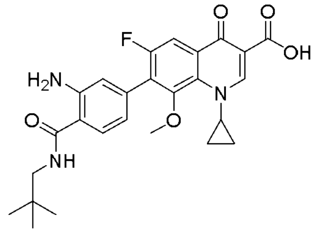 ,

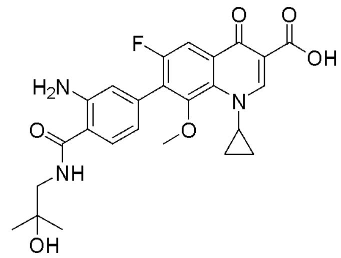 , 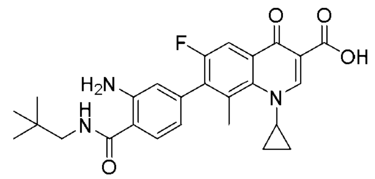 , 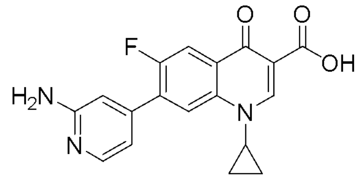 ,

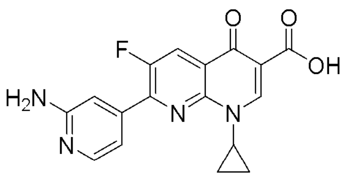 , 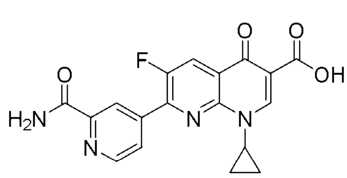 , 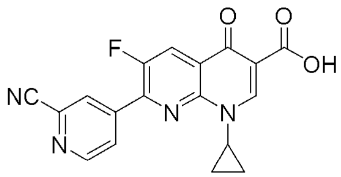 ,

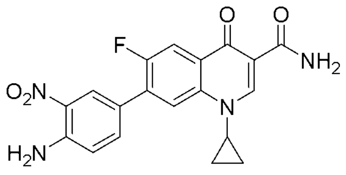 , 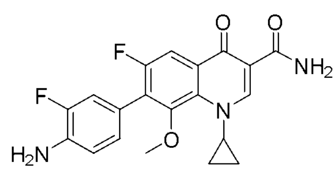 , 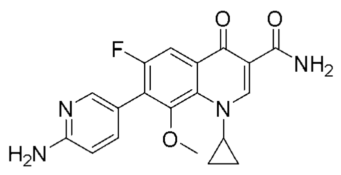 ,

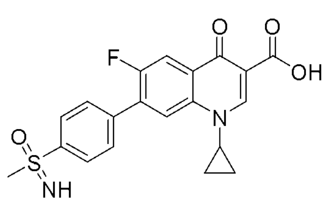 , 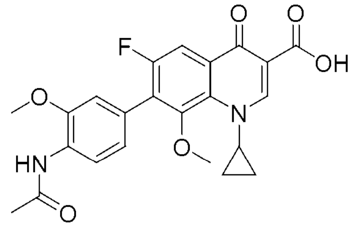 , 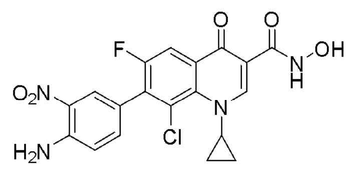 ,

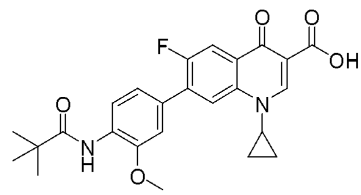 , 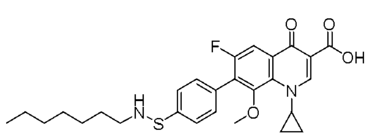 , 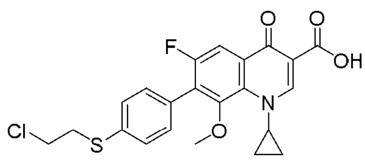 ,

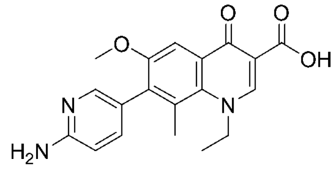 , 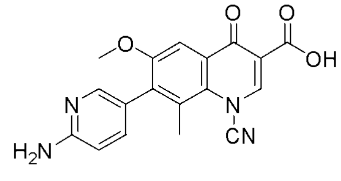 , 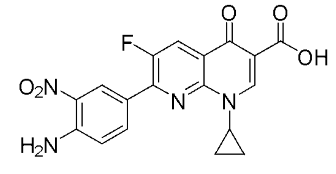 ,

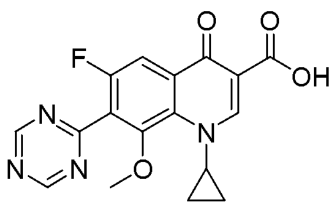,
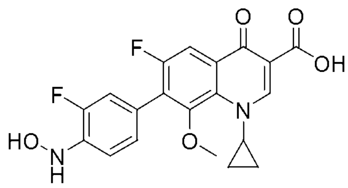,
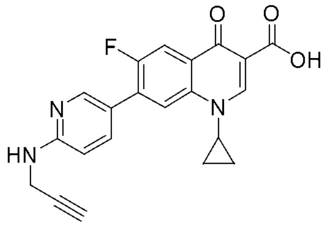,

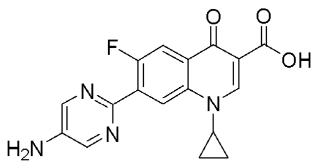,
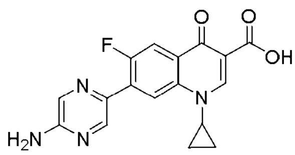,
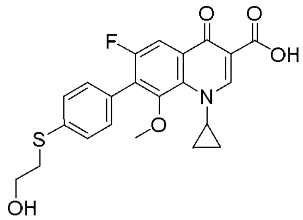,

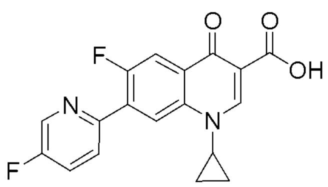,
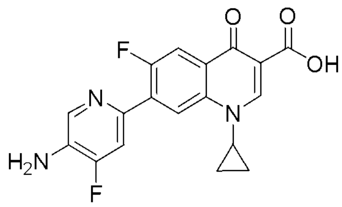,
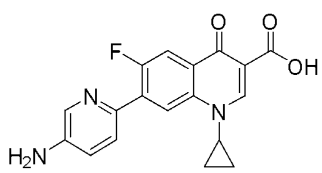,

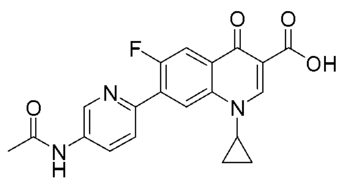,
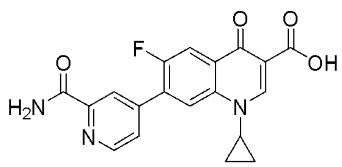,
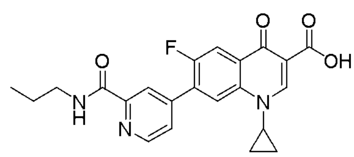,

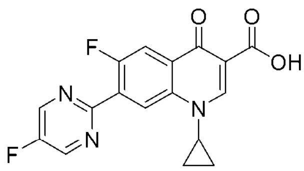,
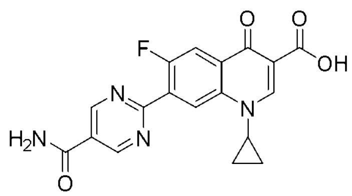,
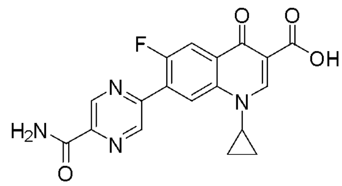,

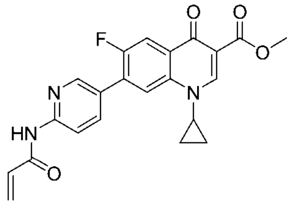,
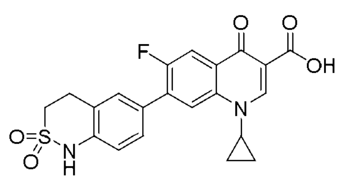,
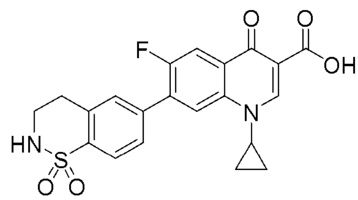,

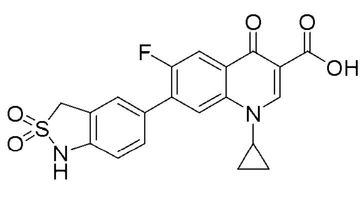,
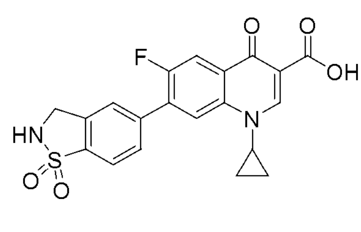,
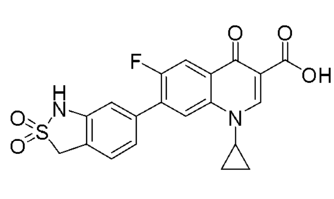,

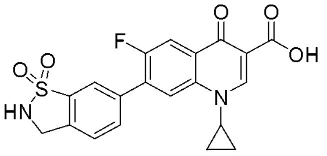,
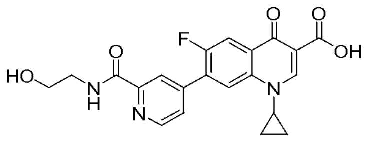,
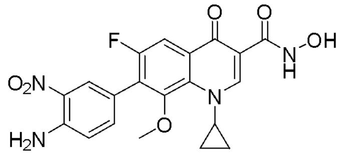,
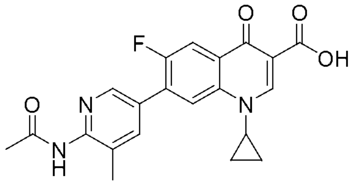,
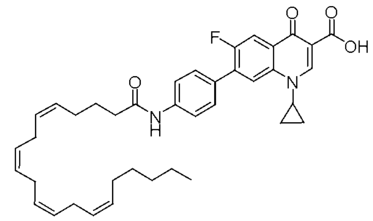,
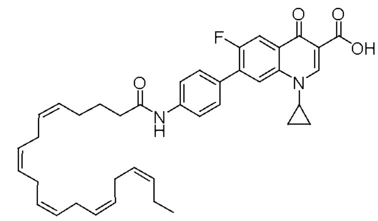,
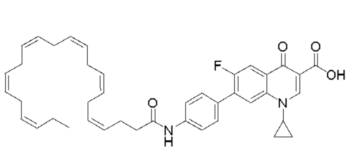,
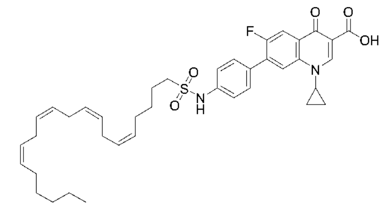,
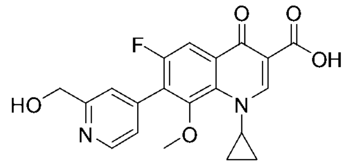,
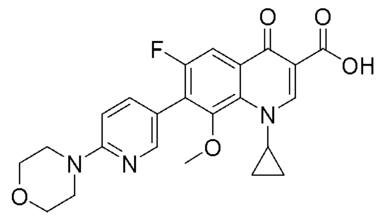,
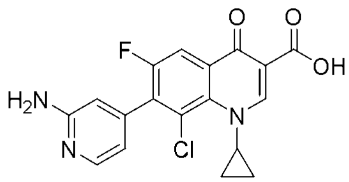.

In an embodiment of the present invention, the pharmaceutically acceptable salt includes inorganic salt or organic salt; wherein, the inorganic salt includes sodium salt, potassium salt, magnesium salt, hydrochloride, hydrobromide, hydroiodate, perchlorate, sulfate, bisulfate, nitrate, phosphate, acid phosphate; the organic salt is selected from formate, acetate, trifluoroacetate, propionate, pyruvate, hydroxyacetate, oxalate, malonate, succinate, glutarate, fumarate, maleate, lactate, malate, citrate, tartrate, methanesulfonate, ethanesulfonate, benzenesulfonate, salicylate, p-toluenesulfonate, ascorbate.

The present invention further provides an antibacterial agent comprising the above phenylquinolone compound or a pharmaceutically acceptable salt thereof.

The present invention further provides an antibacterial medicament comprising the above phenylquinolone compound or a pharmaceutically acceptable salt thereof.

The present invention further provides an anticancer medicament comprising the above phenylquinolone compound or a pharmaceutically acceptable salt thereof.

In an embodiment of the present invention, the above medicament further comprises one or more of the following pharmaceutical excipient(s): solvents, propellants, solubilizers, co-solvents, emulsifiers, color agents, adhesives, disintegrants, fillers, lubricants, wetting agents, osmotic regulators, stabilizers, glidants, flavoring agents, preservatives, suspending agents, coating materials, fragrances, anti-binders, integrators, permeation promoters, pH adjusters, buffers, plasticizers, surfactants, foaming agents, defoamers, thickeners, inclusion agents, moisturizers, flocculants and anti-flocculation agents, filter aids and release blockers.

In an embodiment of the present invention, the above medicament further comprises a pharmaceutical carrier selected from microcapsules, microspheres, nanoparticles and liposomes.

In an embodiment of the present invention, the dosage form of the above medicament includes injection, lyophilized powder for injection, suspension, implant, suppository, capsule, tablet, pill and oral liquid.

In an embodiment of the present invention, the cancer treated by the anticancer medicament includes: cervical cancer, Burkitt's lymphoma, acute T-lymphoblastic leukemia, non-small cell lung cancer, gastric cancer, colon cancer, pancreatic cancer, liver cancer, malignant melanoma, prostate cancer, ovarian adenocarcinoma, glioma, breast cancer, osteosarcoma, breast ductal carcinoma.

**Beneficial effects:**

The phenylquinolone compound or a pharmaceutically acceptable salt, stereoisomer, deuteride, or prodrug thereof of the present invention has excellent antibacterial activity and anticancer activity. The present invention provides a novel antibiotic medicament and treatment method that are effective against drug-resistant bacteria and is suitable for cancer patients.

**Description of the drawings**

Figure 1 is a line plot showing the result of drug resistance test of tested Example 34, ciprofloxacin and vancomycin against *Staphylococcus aureus* ATCC 43300 subtype.

Figure 2 shows photographs of skin infection in mice showing the effect of tested Examples on animal infection models, wherein (A) is normal saline group, (B) is low concentration group (4 μg/mL), (C) is medium concentration group (400 μg/mL), (D) is high concentration group (40000 μg/mL).

Figure 3 is a histogram of the effect of tested drugs on the weight of the kidney (left) and liver (right). Upon experiments, it is found that the related drug molecules had no significant effect on the weight of the kidney and liver, indicating that the safety of the related molecules is good, and the effect of the tested Examples on the liver and kidney of animals is shown.

Figure 4 is a histogram of bacterial colony number analysis of liver (left) and kidney (right) in animal models of systemic blood infections, showing the effect of tested Examples on colony numbers of liver and kidney of animals.

**Detailed embodiments**

In addition to the compound of above formula (I), the present invention further includes a compound of formula (I), in which variables (for example, $A_1$, $A_2$, $R_1$, $R_2$ and the like) have definitions other than the above definitions.

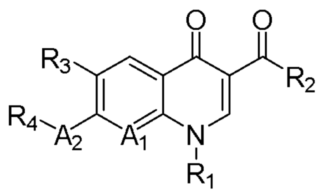

(I)

$A_1$ variable:

The present invention comprises a compound of formula (I) and a salt thereof, wherein $A_1$ is N atom or $CR_a$ group.

In certain embodiments, $R_1$ and $A_1$ are bonded to form a 6-membered heterocycle optionally substituted with short chain alkyl.

$A_2$ variable:

The present invention comprises a compound of formula (I) and a salt thereof, wherein $A_2$ is absent, $-CH_2-$, -CH=CH-, -O- or -NH-.

$R_1$ variable:

The present invention comprises a compound of formula (I) and a salt thereof, wherein $R_1$ is hydrogen, or $R_1$ is C1-C3 alkyl or C3-C6 cycloalkyl, each of them is substituted with 0-3 halogen(s).

In certain embodiments, $R_1$ is phenyl substituted with 0-2 halogen(s), or $R_1$ is 2-pyridinyl substituted with 0-3 substituent(s) independently selected from halogen and amino ($-NH_2$).

$R_2$ variable:

The present invention comprises a compound of formula (I) and a salt thereof, wherein $R_2$ is hydroxy, alkoxy, or

amide optionally substituted with short chain alkyl or hydroxy. Examples of such compound include but are not limited to a compound of formula (V) and a compound of formula (VI)

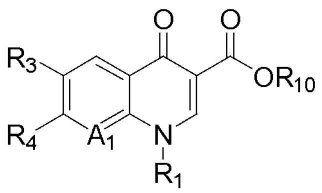

(V)

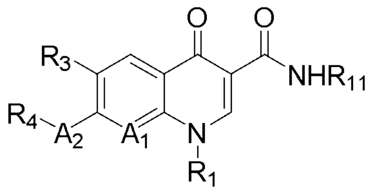

(VI)

Variables $A_1$, $A_2$, $R_a$, $R_1$, $R_3$, $R_4$ and $R_5$ shown in formula (V) and formula (VI) are as defined herein for these variables; $R_{10}$ is hydrogen atom or C1-C2 alkyl, $R_{11}$ is hydrogen atom, C1-C2 alkyl or hydroxy.

$R_3$ variable:

The present invention comprises a compound of formula (I) and a salt thereof, wherein $R_3$ is halogen, nitro or alkoxy.

$R_4$ variable:

Variable $R_4$ shown in formula (I) is as defined herein for the variable.

In certain embodiments, the present invention comprises a compound of formula (I), wherein $A_1$ and $R_1$ are bonded to form a 6-membered heterocycle optionally substituted with alkyl. Examples of such compound include but are not limited to a compound of formula (VII) and a compound of formula (VIII).

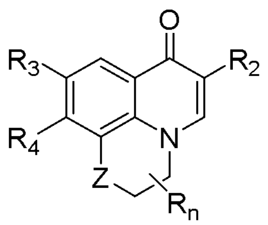

(VII)

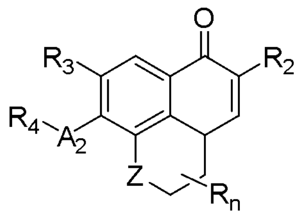

(VIII)

wherein, Z is O or S; $R_n$ is selected from C1-C4 alkyl and halogen.

As used herein, "halogen" denotes F atom, Cl atom, Br atom and I atom.

As used herein, "heteroatom" denotes N atom, O atom and S atom.

As used herein, "alkyl" refers to a branched or linear saturated aliphatic hydrocarbon group having 1-10 carbon atom(s). For example, "C1-C9 alkyl" includes a linear or branched alkyl having 1, 2, 3-8 or 9 carbon atom(s). For the alkyl, C atom in the carbon chain may be replaced by heteroatom at any position as normal valence permits. Examples of alkyl include but are not limited to methyl, ethyl, isopropyl and tert-butyl.

As used herein, "cycloalkyl" refers to saturated non-aromatic hydrocarbon cyclic group having 3-6 carbon atom(s). For the cycloalkyl, C atom in the carbon chain may be replaced by heteroatom at any position as normal valence permits. Examples of cycloalkyl include but are not limited to cyclopropyl, 2-fluorocyclopropyl, cyclobutyl, adamantanyl and the like. The cycloalkyl may include multiple spiro cycles or bridged cycles. The cycloalkyl may be mono-, di-, tri-, tetra-, penta-substituted at any position as normal valence permits.

As used herein, "alkenyl" refers to a linear, branched or cyclic non-aromatic hydrocarbon group having one or more C-C double bond(s) and having 2-25 C atoms. Up to four C-C double bonds may be present in these groups. For the alkenyl, C atom in the carbon chain may be replaced by heteroatom at any position as normal valence permits. For example, "C2-C7" alkenyl is defined as an alkenyl having 2-7 carbon atoms. Examples of alkenyl include but are not limited to vinyl, allyl, hexenyl, cyclopentadienyl and the like. Linear, branched or cyclic moiety of alkenyl may include double bond, and may be mono-, di-, tri-, tetra-, penta-substituted at any position as normal valence permits.

As used herein, "alkynyl" refers to a linear or branched hydrocarbon group containing at least one carbon-carbon triple bond. For example, "C2-C9 alkynyl" denotes an alkynyl containing 2-9 C atoms. For the alkynyl, C atom in the carbon chain may be replaced by heteroatom at any position as normal valence permits. Examples of alkynyl include but are not

limited to ethynyl, 2-propargyl and 2-butynyl. Linear or branched moiety of alkynyl may be mono-, di-, tri-, tetra-, penta-substituted at any position as normal valence permits.

As used herein, "alkoxy" refers to -O-alkyl containing a specific amount of C atom. For example, C2 alkoxy is -OCH2CH3. Examples of alkoxy include but are not limited to methoxy, ethoxy, n-propoxy, isopropoxy, n-butoxy, s-butoxy, t-butoxy, n-pentyloxy, s-pentyloxy, n-heptyloxy and n-octyloxy.

As used herein, "acyl" refers to a group which is attached via a carbonyl bridge connected with hydrogen, amino, hydroxylamine, alkyl, alkenyl, alkynyl, cycloalkyl, alkoxy, aryloxy or heterocycle. Examples of acyl include but are not limited to hydrogen (for example, acetaldehyde group), alkyloyl (for example, acetyl), alkenyloyl (for example, acryloyl), alkynyl (for example, ethynyl), cycloalkyl carbonyl (for example, cyclopentyl carbonyl), aryloyl (for example, benzoyl), alkoxycarbonyl (for example, methoxycarbonyl), aryloxycarbonyl and heterocycylcarbonyl (for example, epoxypropyl).

As used herein, "aryl" may be mono-, di-, tri-, tetra-, penta-substituted at any position as normal valence permits.

As used herein, "haloalkyl" refers to a branched or linear saturated aliphatic hydrocarbon group containing 1-6 carbon atoms, in which, C atom in the carbon chain may be substituted with halogen at any position as normal valence permits. Examples of haloalkyl include but are not limited to chloroethyl and trifluoromethyl.

A compound of formula (I) or a salt thereof has excellent activity against both gram-positive and gram-negative bacteria and can be used to treat a wide range of internal or external bacterial infections. A compound of formula (I) or a salt thereof can inhibit the growth of tumor cells and is suitable for the treatment of infections in cancer patients.

The described compounds may have various tautomers. The present invention is not limited to specific tautomers but includes all tautomers.

An atom in a compound of formula (I) may have corresponding isotopes. The present invention will include compounds comprising all isotopes. Isotopes include atoms having the same atomic number, but a different atomic mass, which include but are not limited to hydrogen isotopes deuterium and tritium, and carbon isotopes $^{11}C$, $^{13}C$ and $^{14}C$.

The "salt" of the compound includes inorganic and organic acid salts and base salts in the present invention. The salt of the compound can be synthesized by selecting a suitable base or acid according to the acidic or basic moiety of the compound itself. Generally, the method for preparing the salt is reacting the free acid form of the compound with a suitable base (such as sodium hydroxide, magnesium ethoxide, etc.), or reacting the free base form of the compound with a suitable acid (such as hydrochloric acid, acetic acid, etc.) to obtain the corresponding salt. Such reaction is usually carried out in an organic solvent or water, or in a mixture of an organic solvent and water. The salt of the compound also includes a solvate compound.

Preparation of the compounds:

All non-aqueous reactions were performed using oven-dried glassware under dry argon. Thin layer chromatography on glass plates coated with general reagent silica gel 60 was used to monitor the progress of reaction. Flash column chromatography was performed on general reagent silica gel. Mass spectrum of the compound was recorded using Agilent G7115A liquid phase-mass spectrometer. NMR spectrum was recorded using Bruker Avance 400 spectrometer at ambient temperature. Chemical shifts for $^1H$ were reported in parts per million ($\delta$) relative to external tetramethyl silane and were referenced to the signal of the residual protons in the deuterated solvent.

In the preparation of the compound of the present invention, different synthetic routes are applied. The compound of formula (I) can be synthesized by the following synthesis processes.

## Reaction formula 1:

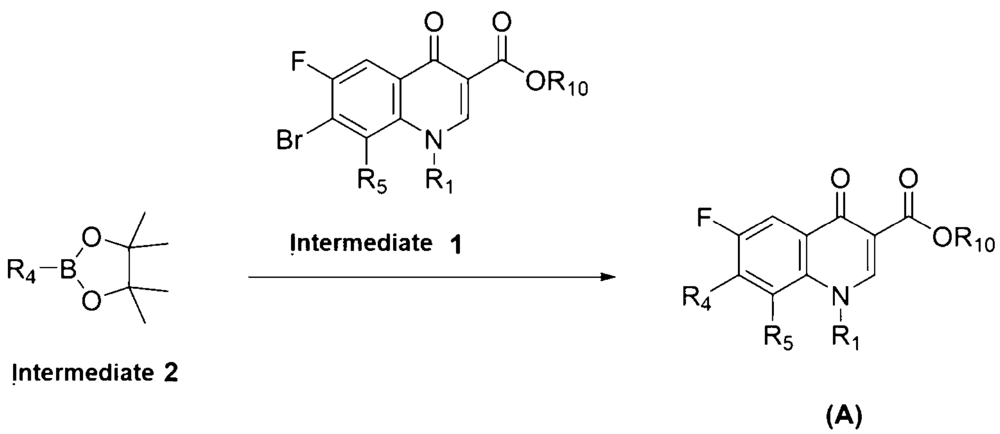

wherein, $R_{10}$ is short chain alkyl.

The synthesis is carried out by reacting intermediate 1 and intermediate 2 through Suzuki reaction in the presence of a base such as $K_2CO_3$, $Na_2CO_3$, $Cs_2CO_3$, etc., and a palladium catalyst such as 1,1-bis(diphenylphosphino) ferrocenedichloropalladium(II), palladium chloride (II) and palladium acetate (II), in a solvent such as DMF, DMSO, THF, dichloromethane, acetonitrile, etc. The reaction may be carried out at a normal atmosphere under an inert gas environment (including nitrogen, argon, etc.). The reaction is usually carried out at room temperature to about 150° C for about 16 to 24 hours.

**Reaction formula 2:**

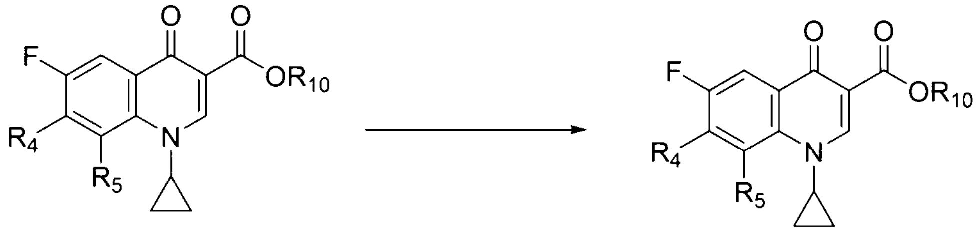

(A) (B)

$R_{10}$ = **short chain alkyl** $R_{10}$ = H

The compound of general formula (B) may be prepared by hydrolyzing compound (A). The hydrolysis of compound (1) may be carried out under conventional reaction conditions for hydrolysis, for example, in the presence of a basic compound such as LiOH, NaOH, KOH, etc., in a solvent including dichloromethane, an alcohol such as methanol, DMF, DMSO, THF, etc. The reaction is usually carried out at a room temperature to about 60° C for about 0.1 to 24 hours.

**Reaction formula3:**

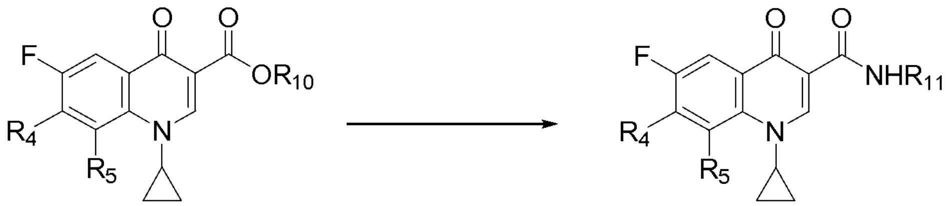

(B) (C)

The compound of formula (C) may be synthesized by a condensation reaction of a compound of formula (B). The compound of formula (B) may be reacted with a relevant amine in the presence of a condensing agent (e.g., DMAP, HATU, etc.) in a solvent including dichloromethane, DMF, THF, etc.

The reaction is usually carried out at room temperature for about 3 to 12 hours.

**Reaction formula4:**

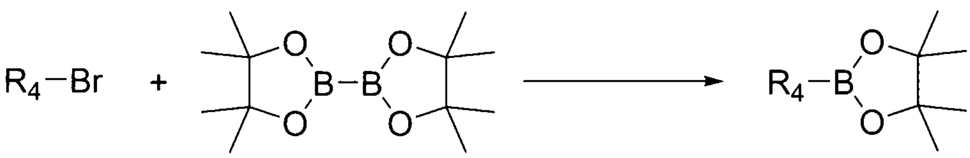

(D) (E) **Intermediate 2**

Intermediate 2 may be prepared by reacting compound (D) with a bis(pinacolato)diboron compound (E) in a typical inert solvent in the presence of a palladium catalyst and a base. The reaction is carried out at a normal atmosphere under an inert gas environment including nitrogen, argon, etc. The reaction may usually be carried out at room temperature to about 150° C for about 16 to 24 hours.

## Reaction formula5:

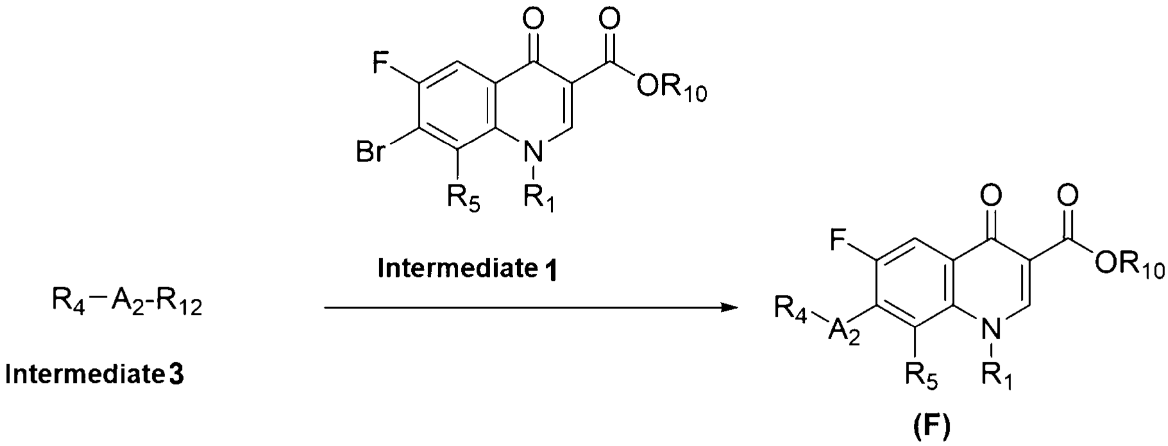

Intermediate 1

Intermediate 3

(F)

wherein, $R_{12}$ is hydrogen atom or halogen.

The of a compound of formula (F) is synthesized by reacting intermediate 3 and intermediate 1 through Buchwald reaction, Kumada reaction, Negishi reaction or Ullmann reaction in the presence of a base such as $K_2CO_3$, $Na_2CO_3$, $Cs_2CO_3$ etc., and in the presence of a copper catalyst such as CuI, Cu (I) or a palladium catalyst such as 1,1-bis(diphenylphosphino)ferrocenedichloropalladium(II), palladium chloride (II) and palladium acetate(II), in a solvent such as DMF, DMSO, THF, toluene, etc. The reaction may be carried out at a normal atmosphere under an inert gas environment including nitrogen, argon, etc. The reaction is usually carried out at room temperature to about 100-150°C for about 16 to 24 hours.

Specific preparation of related intermediates:

**Preparation of intermediate compound 7:**

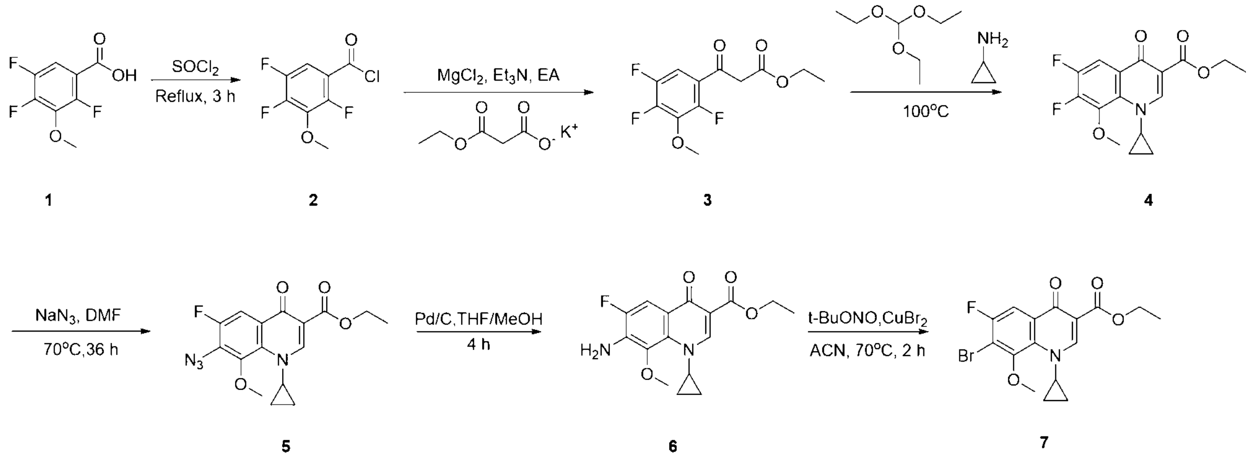


1 2 3 4 5 6 7

Preparation of 2,4,5-trifluoro-3-methoxybenzoyl chloride (**2**)

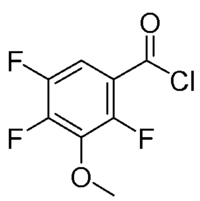

To 2, 4, 5-trifluoro-3-methoxybenzoic acid (**1**) (30.0 g, 145.5 mmol) was added a solution of $SOCl_2$ (180 mL), stirred at 85 °C for 3 hours. After cooling to room temperature, the solvent was removed by evaporation, dried under vacuum, to obtain a light yellow oil (32.0g, 142.5mmol, yield 97.9%), which is directly used in the next step without further purification.

Preparation of ethyl 3-oxo-3-(2,4,5-trifluoro-3-methoxyphenyl)propionate (**3**)

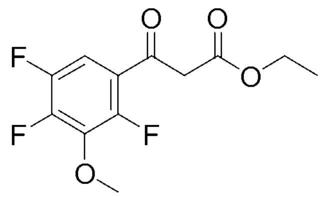

To a solution of potassium 3-ethoxy-3-oxo propionate (**2**) (48.5 g, 284.9 mmol) in EA (300 mL) was added $MgCl_2$ (33.9 g, 356.1 mmol). The mixture was stirred at room temperature for 30 minutes under nitrogen protection. Then, to the mixture was added trimethylamine (28.8 g, 284.6mmol), and stirred at room temperature for additional 30 minutes. To the mixture was added 2,4,5-trifluoro-3-methoxy benzoyl chloride (32.0 g, 142.5 mmol) dropwise, and stirred at 85 °C for 2 hours. After cooling to room temperature, the mixture was added water (150 ml) and acidified with HCl (2M) to (pH=1-2). The mixture was extracted with EA (200 mL). The organic layer was dried over $Na_2SO_4$ and filtered. The solvent was removed under reduced pressure to obtain a brown oil crude product (58.0 g).

Preparation of ethyl 1-cyclopropyl-6,7-difluoro-8-methoxy-4-oxo-1,4-dihydroquinolin-3-carboxylate (**4**)

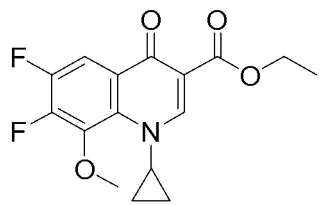

To a solution of ethyl 3-oxo-3-(2,4,5-trifluoro-3-methoxyphenyl)propionate (3) (58.0 g, 210.0 mmol) in $Ac_2O$ (64.3 g, 630.0 mmol) was added triethoxymethane (46.6 g, 314.4 mmol), stirred at 100 °C for 2 hours. The solvent was removed under reduced pressure to obtain a residue. The residue was dissolved in DMSO (400 ml) and was added cyclopropylamine (14.4 g, 252.2 mmol), stirred at room temperature for 2 hours. Then, to the mixture was added $K_2CO_3$ (43.4 g, 314.8 mmol), and stirred at 100°C for additional 1 hours. After cooling to room temperature, to the mixture was added water (500 mL) and stirred at for 10 minutes. The suspension was filtered to remove the filtrate. The solid obtained by filtering was washed with water (50 mL), and dried under vacuum to obtain a target product, as a white solid (21.0 g, 65.0 mmol, 45.6% yield, over two steps).

Preparation of ethyl 7-azido-1-cyclopropyl-6-fluoro-8-methoxy-4-oxo-1,4-dihydroquinolin-3-carboxylate (**5**)

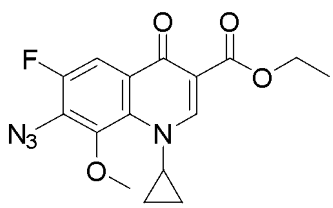

To a solution of ethyl 1-cyclopropyl-6,7-difluoro-8-methoxy-4-oxo-1,4-dihydroquinolin-3-carboxylate (**4**) (10.0 g, 30.9 mmol) in DMF (50 mL) was added $NaN_3$ (4.02 g, 61.8 mmol), stirred at 70 °C for 36 hours. After the reaction was

completed and cooled to room temperature, the mixture was filtered through celite. The filtrate was added to water (50 mL) and extracted with EA (70 mL x3). The organic phase was washed with water (50 ml x3) and brine (50 mL x3), dried over $Na_2SO_4$, filtered and concentrated under reduced pressure to obtain a crude product, as a brown solid (5.6 g).

Preparation of ethyl 7-amino-1-cyclopropyl-6-fluoro-8-methoxy-4-oxo-1,4-dihydroquinolin-3-carboxylate (**6**):

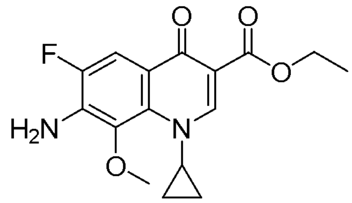

To a solution of ethyl 7-azido-1-cyclopropyl-6-fluoro-8-methoxy-4-oxo-1,4-dihydroquinolin-3-carboxylate (**5**) (5.6 g, crude product) in THF/MeOH (50 mL/10 mL) was added Pd/C (2.8 g, 10% by weight), charged with hydrogen for three times, and stirred at room temperature under hydrogen (ballon) for 4 hours. The reaction was filtered. The solvent was removed under reduced pressure. The residue was purified through silica gel column chromatography (DCM/MeOH=50/1-20/1), to obtain a white solid (3.8 g, 11.86 mmol, 38.3%yield, over two steps).

Preparation of ethyl 7-bromo-1-cyclopropyl-6-fluoro-8-methoxy-4-oxo-1,4-dihydroquinolin-3-carboxylate (**7**):

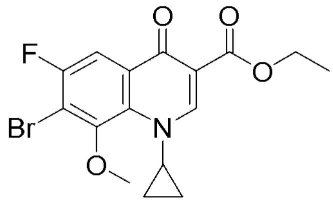

To a solution of ethyl 7-amino-1-cyclopropyl-6-fluoro-8-methoxy-4-oxo-1,4-dihydroquinolin-3-carboxylate (**6**) (3.80 g, 11.86 mmol) in ACN (50 mL) was added tert-butyl alcohol (1.84 g, 17.84 mmol) and $CuBr_2$ (5.30 g, 23.73 mmol), stirred at 70 °C for 2 hours. After cooling to room temperature, the mixture was diluted with saturated $NH_4Cl$ solution (25 mL), and extracted with EA (50 mL x3). The organic layer was washed with brine (30 mL x3), dried over $Na_2SO_4$, filtered and concentrated under reduced pressure to obtain a residue. The residue was dissolved in DCM/MTBE (10mL/610ml), and stirred at room temperature for 10 minutes. The suspension was filtered to remove the filtrate. The solid obtained by filtering was dried under vacuum to obtain a target product, as a yellow solid (2.0 g, 5.21 mmol, 43.9%).

ESI-MS calculated: 383.02, MS found: 386.1$[M+H]^+$.

$^1H$ NMR (400 MHz, MeOD) $\delta$ 8.77 (s, 1H), 7.92 (d, *J* = 8.5 Hz, 1H), 4.33 (q, *J* = 7.1 Hz, 2H), 4.12 (m, 1H), 3.94 (s, 3H), 1.37 (t, *J* = 7.1 Hz, 3H), 1.26 - 1.16 (m, 2H), 1.09 - 0.99 (m, 2H).

**Preparation of intermediate compound 10:**

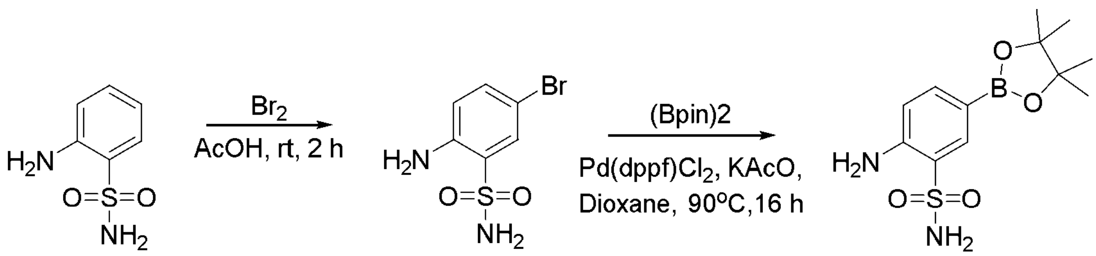


**8** **9** **10**

Preparation of 2-amino-5-bromobenesulfonamide (**9**)

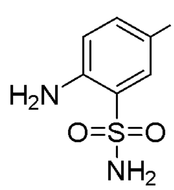

To a mixture of 2-aminobenesulfonamide (**7)** (1 g, 5.81 mmol) in AcOH (20 mL) was added $Br_2$ (928 mg, 5.81 mmol) at room temperature, stirred at room temperature for 2 hours. The reaction mixture was diluted with water (50 mL). The product was precipitated by the addition of water. The precipitated solid was collected by filtering and washed with water to obtain the title product, as a white solid (910 mg, 3.62 mmol 62.4%).

ESI-MS calculated: 249.94, MS found: 251.0[M+H]$^+$.

$^1$H NMR (400 MHz, DMSO-$d_6$) δ 7.64 - 7.61 (m, 1H), 7.41 - 7.35 (m, 3H), 6.78 (d, J = 8.8 Hz, 1H), 6.01 (s, 2H).

Preparation of 2-amino-5-(4,4,5,5-tetramethyl-1,3,2-dioxaborolan-2-yl)benesulfonamide (**10**)

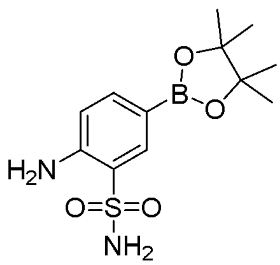

A mixture of 2-amino-5-bromobenesulfonamide (**8**) (800 mg, 3.18 mmol), $Pin_2B_2$ (1.62 g, 6.37 mmol), Pd (dppf) $Cl_2$ (233 mg, 0.32 mmol), potassium acetate (938 mg, 9.56 mmol) and 1.4-dioxane (20 mL) was stirred at 90 °C for 16 hours. After the reaction was completed, the reaction was diluted with water (30 ml) and extracted with ethyl acetate (20 mL x3). The organic layer was washed with saturated aqueous sodium chloride (30 mL), dried over anhydrous sodium sulfate, concentrated under reduced pressure, purified through silica gel column chromatography (PE:EA=1:1), to obtain the title product (760 mg, 2.54 mmol, 80.15% yield), as a yellow oil.

ESI-MS calculated: 298.12, MS found: 299.0[M+H]$^+$.

Preparation of intermediate compound 12:

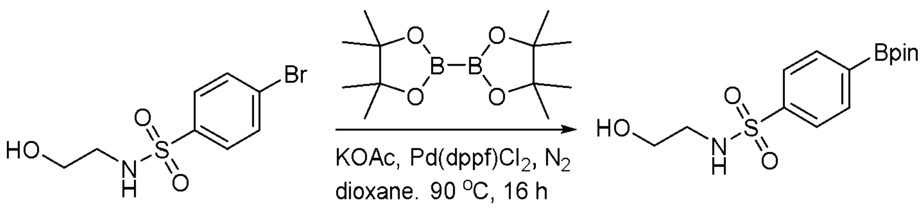


**11** **12**

Preparation of N-(2-hydroxyethyl)-4-(4,4,5,5-tetramethyl-1,3,2-dioxaborolan-2-yl)benzenesulfonamide (**12**)

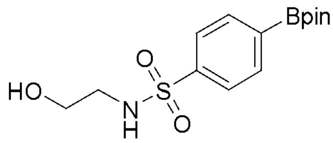

To a mixture of 4-bromo-N-(2-hydroxyethyl)benzenesulfonamide (**11**) (1 g, 3.57 mmol) in 1,4-dioxane (10 mL) was added $B_2Pin_2$ (1.2 g, 4.28 mmol), KOAc (1.05 g, 10.71 mmol) and Pd (dppf)$Cl_2$ (259.04 mg, 356.97 mmol) at room temperature. Then, the mixture was heated to 90°C and stirred for 16 hours. After the reaction was completed, the reaction was cooled to room temperature, concentrated under reduced pressure. The crude product was purified through column chromatography (DCM:MeOH=20:1), to obtain the title product (582 mg, 312.24 mmol, 58.2%), as a brown solid.

ESI-MS calculated: 327.20, MS found: 327.9[M+H]$^+$.

Through the similar preparation route as described above, a series of derivatives were obtained by using similar starting materials as substitutes.

Preparation of intermediate compound 14:

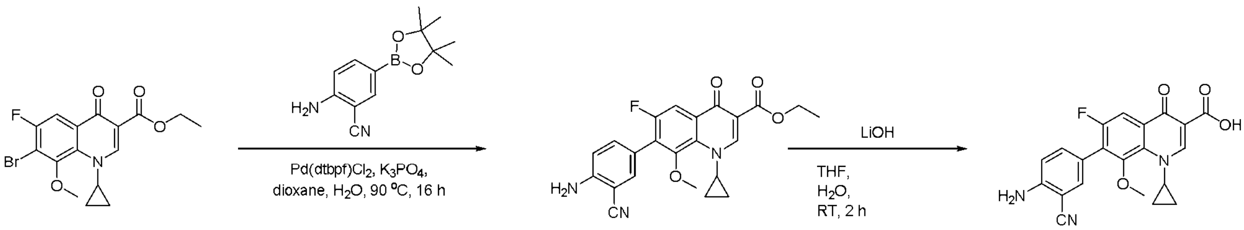


7 13 14

Preparation of ethyl 7-(4-amino-3-cyanophenyl)-1-cyclopropyl-6-fluoro-8-methoxy-4-oxo-1,4-dihydroquinolin-3-carboxylate (**13**)

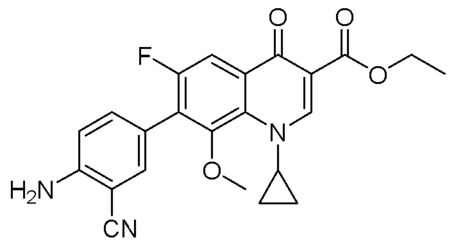

A mixture of ethyl 7-bromo-1-cyclopropyl-6-fluoro-8-methoxy-4-oxo-1,4-dihydroquinolin-3-carboxylate (200 mg, 561.57 umol), 2-amino-5-(4,4,5,5-tetramethyl-1,3,2-dioxaborolan-2-yl)benzonitrile (205.62 mg, 842.35 umol), Pd(dtbpf)$Cl_2$ (36.26 mg, 56.16 umol), $K_3PO_4$ (357.6 mg, 1.68 mmol) in 1,4-dioxane (5 mL) and $H_2O$ (1 mL) was stirred at 90°C for 16 h under nitrogen atmosphere. After cooling to room temperature, the reaction was concentrated under reduced pressure. The crude product was purified through silica gel column chromatography, eluting with DCM:MeOH=20:1, to obtain the product as a white solid (130 mg, 561.57 ummol, 54.93%). ESI-MS calculated: 421.14, MS found: 422.0[M+H]$^+$.

Preparation of 7-(4-amino-3-cyanophenyl)-1-cyclopropyl-6-fluoro-8-methoxy-4-oxo-1,4-dihydroquinolin-3-carboxylic acid (**14**)

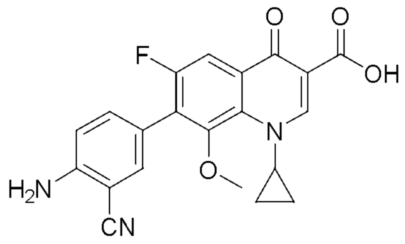

A mixture of ethyl 7-(4-amino-3-cyanophenyl)-1-cyclopropyl-6-fluoro-8-methoxy-4-oxo-1,4-dihydroquinolin-3-carboxylate (130 mg, 308.47 umol), LiOH (29.55 mg, 1.23 umol) in THF (3 mL) and $H_2O$ (1 mL) was stirred at room temperature for 2 hours. After the reaction was completed, the reaction was concentrated under reduced pressure. The crude product was purified through preparative liquid chromatography (0.1% $NH_3$-$H_2O$, $H_2O$) to obtain the product as a white solid (22.73 mg, 308.47 umol, 18.73%).

ESI-MS calculated: 393.11, MS found: 394.1$[M+H]^+$.

A series of derivatives can be obtained by the same preparation routes as described above by replacing similar starting materials.

The pharmaceutically acceptable salts, formed a part of the present invention, can be prepared via a method known in the art by treating a compound of formula I with a suitable acid or base in a suitable solvent.

The present invention is further illustrated by the following examples, which demonstrate some of several preferred examples of the present invention. These examples are provided only as representative examples and should not be understood as a limitation on the scope of the present invention in any way.

**Example 1 Preparation of ethyl 7-(4-amino-3-sulfonylphenyl)-1-cyclopropyl-6-fluoro-8-methoxy-4-oxo-1,4-dihydroquinolin-3-carboxylate**

The synthetic route is as follows:

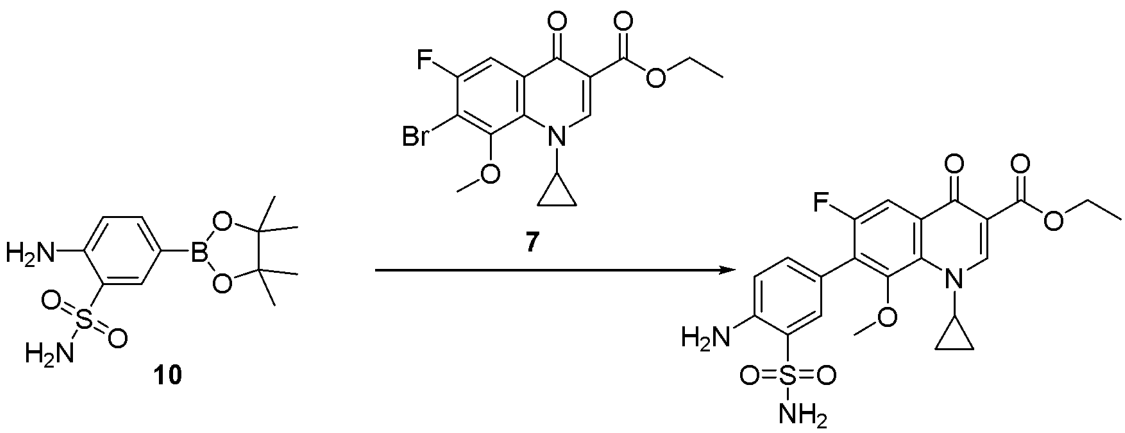

The specific process of preparation:

A mixture of 2-amino-5-(4,4,5,5,6-fluoro-8-methoxy-4-oxo-1,4-dioxabenzofuran-2-ylbenesulfonamide (**10**) (311 mg, 1.04 mmol), ethyl 7-bromo-1-cyclopropyl-6-fluoro-8-methoxy-4-oxo-1,4-dihydroquinoline-3-carboxylate (**7**) (200 mg, 0.522 mmol), Pd (dppf)$Cl_2$ (38 mg, 0.05 mmol), $K_3PO_4$ (333 mg, 10.57mmol) and 1.4-dioxane/$H_2O$=4/1 (10 mL) was stirred at 90°C for 16 hours. The reaction mixture was diluted with water (20 mL) and extracted with ethyl acetate (20 mL x3). The organic phase was washed with saturated aqueous sodium chloride (20mL), dried over anhydrous sodium sulfate. The solvent was removed under reduced pressure. The residue was purified through silica gel column chromatography (gradient of 1:0 to 1:9 PE:EA), to obtain a yellow solid (70 mg, 0.15 mmol, yield 28.23%).

MS calculated: 475.12, MS found: 476.0$[M+H]^+$.

**Example 2 7-(4-amino-3-sulfonylphenyl)-1-cyclopropyl-6-fluoro-8-methoxy-4-oxo-1,4-dihydroquinolin-3-carboxylic acid**

The synthetic route is as follows

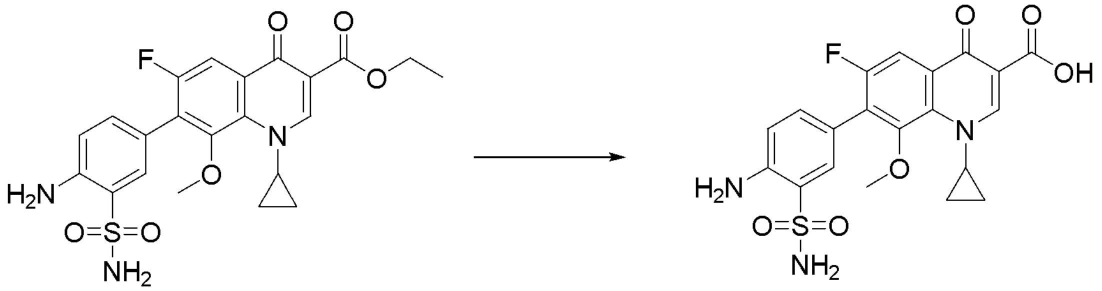

The specific process of preparation:

To THF/$H_2O$=2/1 (5mL) was added ethyl 7-(4-amino-3-sulfonylphenyl)-1-cyclopropyl-6-fluoro-8-methoxy-4-oxo-1,4-dihydroquinolin-3-carboxylate (70 mg, 0.15 mmol) (Example 1), and added LiOH (35 mg, 1.47 mmol). The mixture was stirred at room temperature for 1 hours. The mixture was concentrated under reduced pressure, diluted with water (20 mL). The mixture was adjusted with citric acid to PH=4-5, and extracted with ethyl acetate (20 mL x3). The combined organic extract was washed with saturated aqueous sodium chloride (20 mL), dried over anhydrous sodium sulfate and concentrated under reduced pressure. The residue was purified through prep-HPLC [water (0.1%FA)], to obtain the final product (18.96 mg, 0.042 mmol, 28.27%), as a yellow solid.

MS calculated: 447.09, MS found: 448.0 $[M+H]^+$.

$^1$H NMR (400 MHz, DMSO-d6) δ 14.72 (s, 1H), 8.79 (s, 1H), 7.94 - 7.82 (m, 1H), 7.75 (s, 1H), 7.48 - 7.32 (m, 3H), 6.97 (d, J = 8.5 Hz, 1H), 6.22 (s, 2H), 4.29 - 4.12 (m, 1H), 3.43 (s, 3H), 1.22 - 1.08 (m, 4H).

**Example 3 1-cyclopropyl-6-fluoro-7-(3-nitro-4-sulfamoy phenyl)-4-oxo-1,4-dihydroquinolin-3-carboxylic acid**

Referring to the preparation processes in Examples 1 and 2, the target compound with the following structure was obtained by using similar starting materials as substitutes:

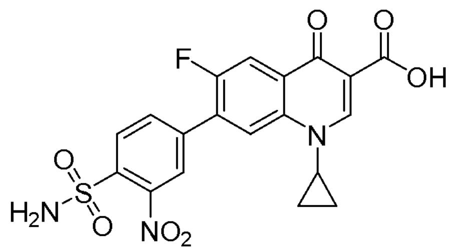

MS calculated: 448.05, MS found: 448.1 $[M+H]^+$.

$^1$H NMR (400 MHz, DMSO-d6) δ 14.73 (s, 1H), 8.81 (s, 1H), 8.51 (d, J = 6.4 Hz, 1H), 8.37 (s, 1H), 8.22 (d, J = 11.2 Hz, 3H), 8.01 (s, 2H), 4.08 - 3.75 (m, 1H), 1.37 - 1.21 (m, 4H).

**Example 4 8-chloro-1-cyclopropyl-6-fluoro-7-(3-nitro-4-sulfamoy phenyl)-4-oxo-1,4-dihydroquinolin-3-carboxylic acid**

Referring to the preparation processes in Examples 1 and 2, the target compound with the following structure was obtained by using similar starting materials as substitutes:

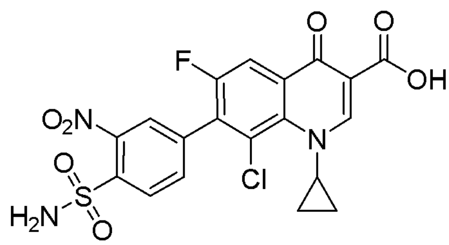

MS calculated: 482.01, MS found: 482.1 $[M+H]^+$.

$^1$H NMR (400 MHz, DMSO-d6) δ 14.11 (s, 1H), 8.93 (s, 1H), 8.27 - 8.17 (m, 3H), 8.02 (d, J = 8.2 Hz, 3H), 4.44 - 4.34

(m, 1H), 1.24 - 1.19 (m, 2H), 1.16 - 1.08 (m, 2H).

**Example 5 ethyl 1-cyclopropyl-6-fluoro-7-(4-(N-(2-hydroxyethyl)aminosulfonyl)phenyl)-8-methoxy-4-oxo-1,4-dihydroquinolin-3-carboxylate**

The synthetic route is as follows:

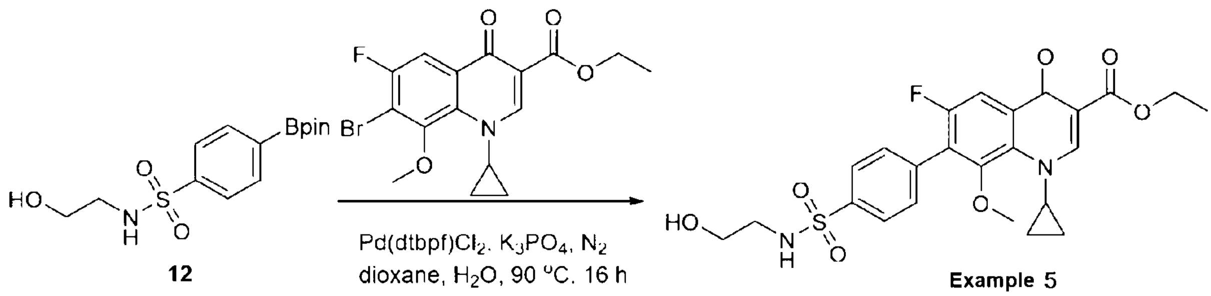


12

Example 5

To a mixture of N-(2-hydroxyethyl)-4-(4,4,5,5-tetramethyl-1,3,2-dioxaborolan-2-yl)benzolsulfonamide(12)(146 mg, 312.24 umol) in 1,4-dioxane (3 mL) and $H_2O$ (1 mL) was added ethyl 7-bromo-1-cyclopropyl-6-fluoro-8-methoxy-4-oxo-1,4-dihydroquinolin-3-carboxylate (7)(100 mg, 260.28 umol), $K_3PO_4$ (165.74 mg, 780.84 umol) and Pd(dtbpf)$Cl_2$ (16.81 mg, 26.03 umol) at room temperature. Then, the mixture was heated to 90°C and stirred for 16 hours. Then, the reaction solution was cooled to room temperature, concentrated under reduced pressure. The crude product was purified through silica gel column chromatography, eluted with a gradient of DCM:MeOH - 1:0 to 20:1, to obtain the product as a brown solid (66 mg, 130.81 umol, 35.18%).

MS calculated: 504.53, MS found: 505.1[M+H]$^+$.

$^1$H NMR (400 MHz, DMSO-d6) δ 8.58 (s, 1H), 7.96 (d, *J* = 8.3 Hz, 2H), 7.86 - 7.68 (m, 3H), 4.72 (t, *J* = 5.6 Hz, 1H), 4.25 (q, *J* = 7.1 Hz, 2H), 4.05 (dt, *J* = 6.4, 2.5 Hz, 1H), 3.47 - 3.23 (m, 5H), 2.88 (t, *J* = 6.1 Hz, 2H), 1.29 (t, *J* = 7.1 Hz, 3H), 1.36 - 1.03 (m, 7H).

**Example 6 1-cyclopropyl-6-fluoro-7-(4-(N-(2-hydroxyethyl)aminosulfonyl)phenyl)-8-methoxy-4-oxo-1,4-dihydroquinolin-3-carboxylic acid**

The synthetic route is as follows:

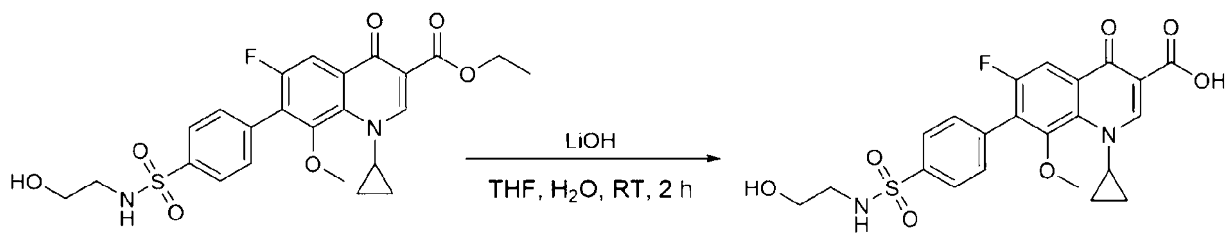


Example 5

Example 6

To a solution of ethyl 1-cyclopropyl-6-fluoro-7-(4-(N-(2-hydroxyethyl)aminosulfonyl)phenyl)-8-methoxy-4-oxo-1,4-dihydroquinolin-3-carboxylate (66 mg, 130.81 umol) in THF (3 mL) was added $H_2O$ (1 mL) and LiOH(12.53 mg, 523.26 umol). The reaction solution was stirred at room temperature for 2h. After the reaction was completed, the pH was adjusted to neutral. After diluting with DCM (10 mL), the reaction solution was washed with water (10 mL X 3). The organic phase was dried with anhydrous sodium sulfate, filtered, reduced under reduced pressure to obtain the product as a white solid (22 mg, 46.17 umol, 35.30%).

MS calculated: 476.11, MS found: 477.2 [M+H]$^+$.

$^1$H NMR (400 MHz, DMSO-d6) δ 14.61 (s, 1H), 8.82 (s, 1H), 7.98 (dd, *J* = 8.7, 4.7 Hz, 3H), 7.77 (dd, *J* = 13.3, 6.9 Hz, 3H), 4.72 (t, *J* = 5.2 Hz, 1H), 4.28 - 4.17 (m, 1H), 3.44 - 3.36 (m, 5H), 2.88 (q, *J* = 6.0 Hz, 2H), 1.23 - 1.12 (m, 4H).

**Example 7 7-(4-(N-(2-chloroethyl)aminosulfonyl)phenyl)-1-cyclopropyl-6-fluoro-8-methoxy-4-oxo-1,4-dihydroquinolin-3-carboxylic acid**

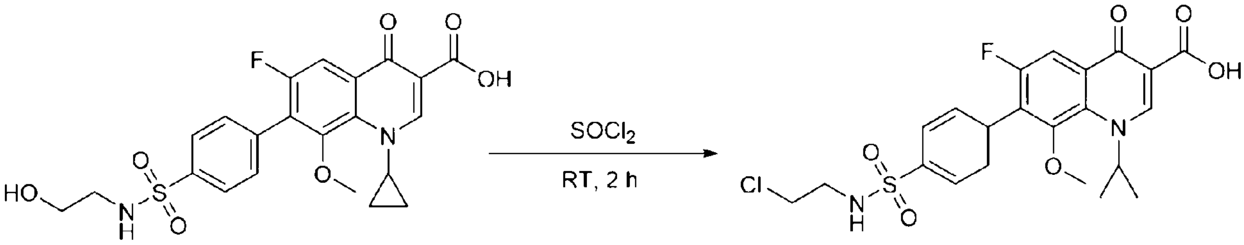


Example 6 Example 7

To a solution of 1-cyclopropyl-6-fluoro-7-(4-(N-(2-hydroxyethyl)aminosulfonyl)phenyl)-8-methoxy-4-oxo-1,4-dihydroquinolin-3-carboxylic acid (Example 6) (22 mg, 130.81 umol) in THF (3 mL) was added $H_2O$ (1 mL) and $SOCl_2$(5 mL) at room temperature. After MS confirmed the completion of the reaction, the reaction solution was concentrated under reduced pressure. The crude product was purified through preparative liquid chromatography (0.1% FA, $H_2O$) to obtain the product as a white solid (13.70 mg, 36.37 umol, 78.77%).

MS calculated: 494.07, MS found: 495.1 $[M+H]^+$.

$^1H$ NMR (400 MHz, DMSO-d6) δ 14.63 (s, 1H), 8.82 (s, 1H), 8.16 (d, *J* = 10.6 Hz, 1H), 7.98 (d, *J* = 11.1 Hz, 3H), 7.80 (d, *J* = 7.9 Hz, 2H), 4.21 (q, *J* = 5.8 Hz, 1H), 3.61 (t, *J* = 6.1 Hz, 2H), 3.39 (s, 3H), 3.21 - 3.17 (m, 2H), 1.20-1.13 (m, 4H).

**Example 8 7-(3-cyano-4-(2,2,2-trifluoroacetamido)phenyl)-1-cyclopropyl-6-fluoro-8-methoxy-4-oxo-1,4-dihydroquinolin-3-carboxylic acid**

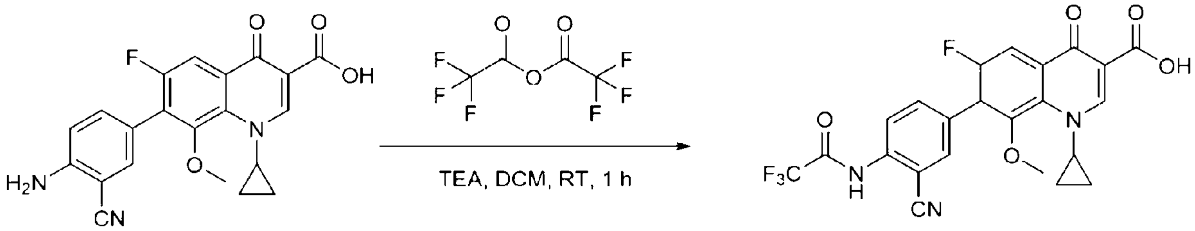


14 Example 8

To 7-(4-amino-3-cyanophenyl)-1-cyclopropyl-6-fluoro-8-methoxy-4-oxo-1,4-dihydroquinolin-3-carboxylic acid (14) (35 mg, 88.97 umol) in DCM (3 mL) was added 2,2,2-trifluoroacetic anhydride (28.03 mg, 133.46 umol) at TEA (13.51 mg, 133.46 umol) at room temperature. After stirring at room temperature for 1h, the mixture was concentrated under reduced pressure. The crude product was purified through preparative high performance liquid chromatography (0.1% FA, $H_2O$) to obtain the product as a white solid (17.35 mg, 88.97 umol, 39.85%).

MS calculated: 489.09, MS found: 489.8 $[M+H]^+$.

$^1H$ NMR (400 MHz, DMSO-d6) δ 14.60 (s, 1H), 11.94 (s, 1H), 8.82 (s, 1H), 8.21 (s, 1H), 8.06 - 7.91 (m, 2H), 7.81 (d, *J* = 8.4 Hz, 1H), 4.33 - 4.10 (m, 1H), 3.43 (s, 3H), 1.27 - 1.09 (m, 4H).

**Example 9 7-(3-cyano-4-(N-(tetrahydro-2H-pyran-4-yl)sulfamoyl)phenyl)-1-cyclopropyl-6-fluoro-4-oxo-1,4-dihydroquinolin-3-carboxylic acid**

Referring to the preparation processes in Examples 1 to 8, the target compound with the following structure was obtained by using similar starting materials as substitutes:

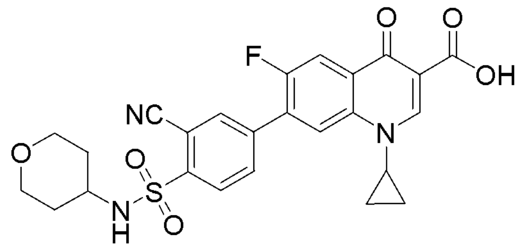

MS calculated: 511.12, MS found: 512.3 $[M+H]^+$.

$^1H$ NMR (400 MHz, DMSO-d6) δ 14.72 (s, 1H), 8.81 (s, 2H), 8.57 - 8.36 (m, 3H), 8.31 - 8.14 (m, 3H), 4.01 - 3.69 (m, 3H), 3.49 - 3.40 (m, 2H), 2.67 (s, 1H), 1.74 - 1.44 (m, 4H), 1.38 - 1.17 (m, 4H).

**Example 10 7-(3-cyano-4-(N-cyclohexylaminosulfonyl)phenyl)-1-cyclopropyl-6-fluoro-8-methoxy-4-oxo-1,4-dihydroquinolin-3-carboxylic acid**

Referring to the preparation processes in Examples 1 to 8, the target compound with the following structure was obtained by using similar starting materials as substitutes:

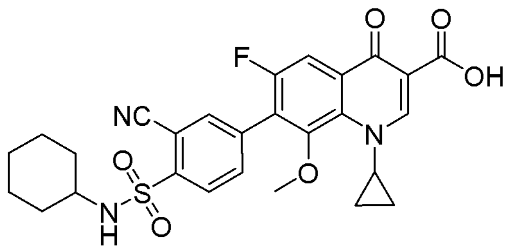

MS calculated: 539.15, MS found: 540.3 $[M+H]^+$.

$^1H$ NMR (400 MHz, DMSO-d6) δ 14.85 (s, 1H), 8.71 (s, 1H), 8.12 - 7.85 (m, 4H), 7.71 (dt, J = 7.4, 1.7 Hz, 1H), 7.26 (d, J = 12.1 Hz, 1H), 4.13 - 3.80 (m, 4H), 3.36 - 3.15 (m, 1H), 1.75 - 0.88 (m, 14H).

**Example 11 7-(4-amino-3-sulfonylphenyl)-1-cyclopropyl-6-fluoro-8-nitro-4-oxo-1,4-dihydroquinolin-3-carboxylic acid**

Referring to the preparation processes in Examples 1 to 8, the target compound with the following structure was obtained by using similar starting materials as substitutes:

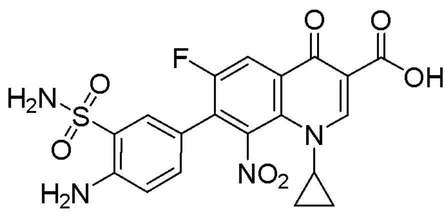

MS calculated: 462.06, MS found: 463.0 $[M+H]^+$.

$^1H$ NMR (400 MHz, MeOD) δ 8.99 (s, 1H), 8.42 (d, J = 8.5 Hz, 1H), 7.68 (s, 1H), 7.25 (d, J = 8.7 Hz, 1H), 6.93 (d, J = 8.2 Hz, 1H), 3.90- 3.77 (m, 1H), 1.21 - 1.08 (m, 4H).

**Example 12 7-(3,5-dimethyl-4-(piperidin-1-ylsulfonyl)phenyl)-1-ethyl-6-nitro-4-oxo-1,4-dihydro-1,8- naphthyridin-3-carboxylic acid**

Referring to the preparation processes in Examples 1 to 8, the target compound with the following structure was obtained by using similar starting materials as substitutes:

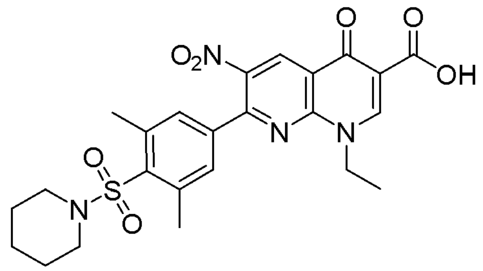

MS calculated: 514.15, MS found: 515.2 $[M+H]^+$.

$^1H$ NMR (400 MHz, DMSO-d6) δ 14.03 (s, 1H), 9.34 (s, 1H), 9.26 (s, 1H), 7.56 (s, 2H), 4.67 (q, $J$ = 7.0 Hz, 2H), 3.14 (s, 4H), 2.66 (s, 6H), 1.54 (s, 6H), 1.45 (t, $J$ = 7.1 Hz, 3H).

**Example 13 1-(6-amino-3,5-difluoropyridin-2-yl)-8-chloro-6-fluoro-4-oxo-7-(4-pyrrolidin-1-ylsulfonyl)phenyl)-1,4-dihydroquinolin-3-carboxylic acid**

Referring to the preparation processes in Examples 1 to 8, the target compound with the following structure was obtained by using similar starting materials as substitutes:

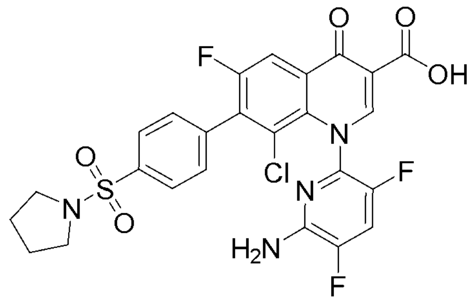

MS calculated: 578.06, MS found: 579.0 $[M+H]^+$.

$^1H$ NMR (400 MHz, MeOD) δ 8.63 (s, 1H), 8.25 (d, J = 9.0 Hz, 1H), 7.98 - 7.93 (m, 2H), 7.60 - 7.56 (m, 2H), 7.56 - 7.52 (m, 1H), 3.29 - 3.26 (m, 4H), 1.79 - 1.73 (m, 4H).

**Example 14 1-(6-amino-3,5-difluoropyridin-2-yl)-8-chloro-6-fluoro-4-oxo-7-(4-(pyrrolidin-1-ylsulfonyl)phenyl)-1,4-dihydroquinolin-3-carboxamide**

Referring to the preparation processes in Examples 1 to 8, the target compound with the following structure was obtained by using similar starting materials as substitutes:

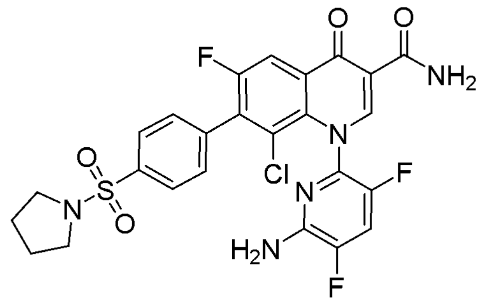

MS calculated: 577.08, MS found: 578.0 $[M+H]^+$.

$^1H$ NMR (400 MHz, DMSO-d6) δ 11.03 - 9.91 (m, 2H), 8.56 - 8.33 (m, 1H), 8.00 - 7.86 (m, 3H), 7.77 - 7.67 (m, 1H), 7.65 - 7.42 (m, 2H), 6.42 - 6.20 (m, 2H), 3.21 - 3.15 (m, 4H), 1.69 - 1.62 (m, 4H).

**Example 15 7-(4-(N-(2-chloroethyl)sulfamoyl)phenyl)-1-cyclopropyl-6-fluoro-4-oxo-1,4-dihydroquinolin-3-carboxylic acid**

Referring to the preparation processes in Examples 1 to 8, the target compound with the following structure was obtained by using similar starting materials as substitutes:

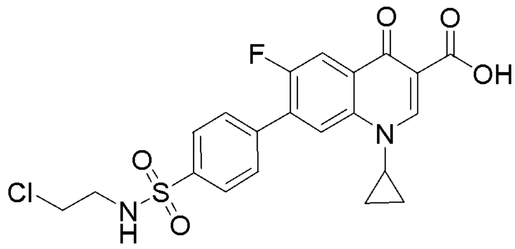

MS calculated: 464.06, MS found: 465.2 $[M+H]^+$.

$^1$H NMR (400 MHz, DMSO-d6) δ 14.76 (s, 1H), 8.81 (s, 1H), 8.44 (d, J = 6.4 Hz, 1H), 8.18 (dd, J = 8.0, 5.1 Hz, 2H), 7.99 (q, J = 8.5 Hz, 4H), 4.03 -3.93 (m, 1H), 3.63 (t, J = 6.1 Hz, 2H), 3.17 (q, J = 6.0 Hz, 2H), 1.39 - 1.28 (m, 2H), 1.29 - 1.21 (m, 2H).

**Example 16 1-cyclopropyl-6-fluoro-7-(4-(N-(2-hydroxyethyl)aminosulfonyl)phenyl)-4-oxo-1,4-dihydroquinolin-3-carboxylic acid**

Referring to the preparation processes in Examples 1 to 8, the target compound with the following structure was obtained by using similar starting materials as substitutes:

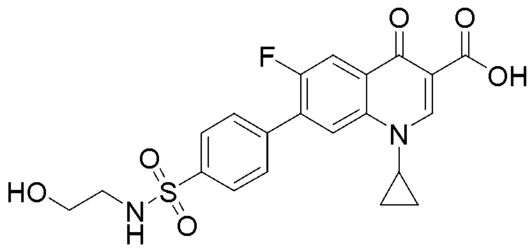

MS calculated: 446.09, MS found: 447.2 $[M+H]^+$.

$^1$H NMR (400 MHz, DMSO) δ 14.78 (s, 1H), 8.80 (s, 1H), 8.44 (d, *J* = 6.4 Hz, 1H), 8.18 (dd, *J* = 10.3, 2.0 Hz, 1H), 8.04 - 7.91 (m, 4H), 7.80 (t, *J* = 5.9 Hz, 1H), 4.73 (s, 1H), 4.05 - 3.84 (m, 1H), 3.43 (t, *J* = 6.3 Hz, 2H), 2.87 (q, *J*= 6.2 Hz, 2H), 1.38 - 1.21 (m, 4H).

**Example 17 6-fluoro-1-(4-fluorophenyl)-7-(4-(N-heptylsulfamoyl)phenyl)-4-oxo-1,4-dihydroquinolin-3-carboxylic acid**

Referring to the preparation processes in Examples 1 to 8, the target compound with the following structure was obtained by using similar starting materials as substitutes:

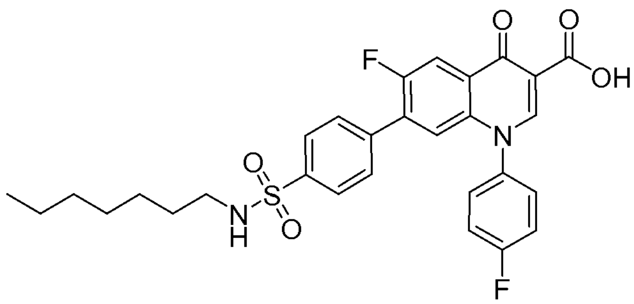

MS calculated: 554.17, MS found: 555.2 $[M+H]^+$.

**Example 18 1-cyclopropyl-6-fluoro-7-(4-(N-(2-hydroxyethyl)aminosulfonyl)phenyl)-4-oxo-1,4-dihydro-1,8-naphthyridine -3-carboxylic acid**

Referring to the preparation processes in Examples 1 to 8, the target compound with the following structure was obtained by using similar starting materials as substitutes:

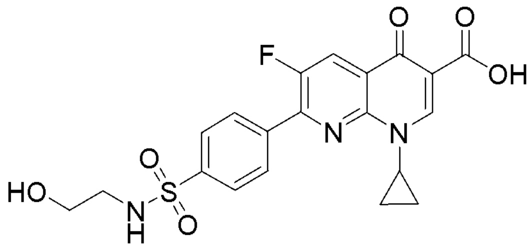

MS calculated: 447.09, MS found: 448.2 $[M+H]^+$.

$^1H$ NMR (400 MHz, DMSO-d6) δ 14.78 (s, 1H), 8.80 (s, 1H), 7.93 - 7.47 (m, 6H), 4.73 (s, 1H), 4.05 - 3.84 (m, 1H), 3.43 (t, *J* = 6.3 Hz, 2H), 2.87 (q, *J* = 6.2 Hz, 2H), 1.34 - 1.20 (m, 4H).

**Example 19 1-cyclopropyl-6-fluoro-4-oxo-7-(6-(propylsulfonamido)pyridin-3-yl)-1,4-dihydroquinolin-3-carboxylic acid**

Referring to the preparation processes in Examples 1 to 8, the target compound with the following structure was obtained by using similar starting materials as substitutes:

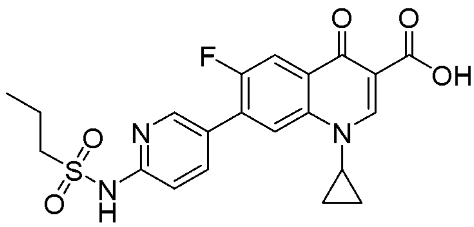

MS calculated: 445.11, MS found: 446.3$[M+H]^+$.

$^1H$ NMR (400 MHz, DMSO-d6) δ 15.08 - 14.64 (m, 1H), 11.92 - 10.78 (m, 1H), 8.78 (s, 1H), 8.58 (t, *J* = 6.2 Hz, 1H), 8.41 (t, *J* = 6.3 Hz, 1H), 8.21 (s, 1H), 8.18 - 8.10 (m, 1H), 8.09 - 8.02 (m, 1H), 7.07 (d, *J* = 8.8 Hz, 1H), 3.95 (ddd, *J* = 11.3, 7.2, 3.8 Hz, 1H), 3.43 (dd, *J* = 13.2, 4.9 Hz, 2H), 1.81 - 1.68 (m, 2H), 1.34 (dd, *J* = 7.4, 5.2 Hz, 2H), 1.25 - 1.23 (m, 2H), 0.99 (t, *J* = 7.4 Hz, 3H).

**Example 20 8-chloro-1-cyclopropyl-6-fluoro-4-oxo-7-(6-(N-pentylaminosulfonyl)pyridine-3-yl)-1,4-dihydroquinolin-3-carboxylic acid**

Referring to the preparation processes in Examples 1 to 8, the target compound with the following structure was obtained by using similar starting materials as substitutes:

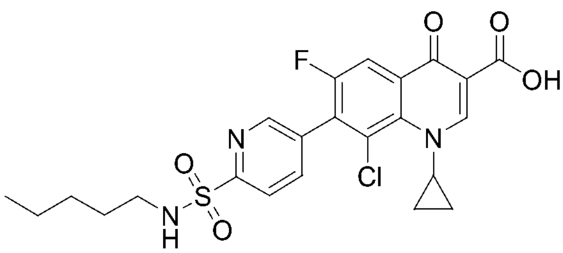

MS calculated: 507.10, MS found: 507.9 $[M+H]^+$.

$^1H$ NMR (400 MHz, DMSO-d6) δ 14.19 (s, 1H), 8.94 (s, 1H), 8.87 (s, 1H), 8.27 (dd, *J*= 8.0, 1.8 Hz, 1H), 8.22 (d, *J* = 8.6 Hz, 1H), 8.15 (d, *J* = 8.1 Hz, 1H), 8.00 (t, *J* = 5.8 Hz, 1H), 4.42 - 4.38 (m, 1H), 2.97 (dd, *J* = 13.1, 6.7 Hz, 2H), 1.62 - 0.79 (m, 13H).

**Example 21 1-cyclopropyl-6-fluoro-4-oxo-7-(6-(N-(2,2,2-trichloroacetyl)sulfamoyl)pyridin-3-yl)-1,4-dihydroquinolin-3-carboxylic acid**

Referring to the preparation processes in Examples 1 to 8, the target compound with the following structure was obtained by using similar starting materials as substitutes:

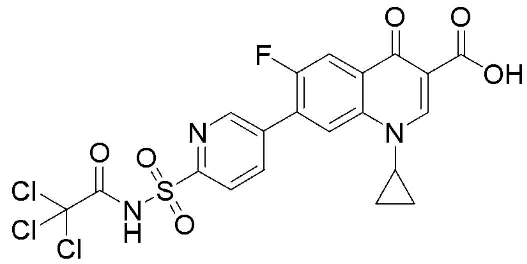

MS calculated: 546.96, MS found: 549.8 [M+H]$^+$.

$^1$H NMR (400 MHz, DMSO-d6) δ 8.95 (s, 1H), 8.81 (s, 1H), 8.51 (d, J = 6.4 Hz, 1H), 8.34 (d, J = 8.3 Hz, 1H), 8.21 (d, J = 10.2 Hz, 1H), 8.08 (d, J = 8.2 Hz, 1H), 4.03 - 3.92 (m, 1H), 1.39 - 1.33 (m, 2H), 1.27 - 1.19 (m, 2H).

**Example 22 1-cyclopropyl-6-fluoro-8-methoxy-4-oxo-7-(4-(N-pentylaminosulfonyl)phenyl)-1,4-dihydroquinolin-3-carboxylic acid**

Referring to the preparation processes in Examples 1 to 8, the target compound with the following structure was obtained by using similar starting materials as substitutes:

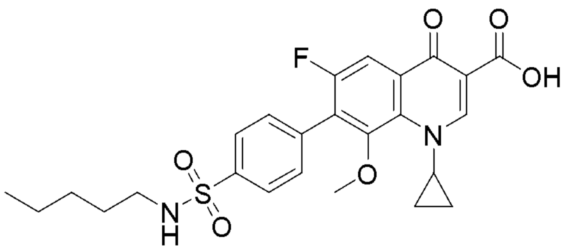

MS calculated: 502.16, MS found: 503.3 [M+H]$^+$.

$^1$H NMR (400 MHz, DMSO-d6) δ 14.64 (s, 1H), 8.82 (s, 1H), 7.97 (d, *J* = 8.4 Hz, 3H), 7.79 (d, *J* = 7.9 Hz, 2H), 7.74 (t, *J* = 5.8 Hz, 1H), 4.26 - 4.15 (m, 1H), 3.38 (s, 3H), 2.85 - 2.78 (m, 2H), 1.63 - 0.76 (m, 13H).

**Example 23 ethyl 1-ethyl-7-(4-(N-heptylsulfamoyl)-3,5-dimethylphenyl)-6-nitro-4-oxo-1,4-dihydro-1,8-naphthyridin-3-carboxylate**

Referring to the preparation processes in Examples 1 to 8, the target compound with the following structure was obtained by using similar starting materials as substitutes:

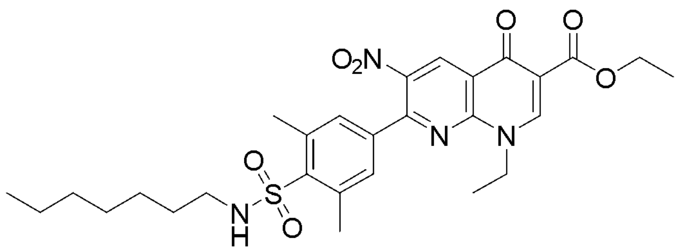

MS calculated: 572.23, MS found: 573.2 [M+H]$^+$.

$^1$H NMR (400 MHz, DMSO-d6) δ 9.06 (s, 1H), 8.98 (s, 1H), 7.69 (t, *J* = 5.7 Hz, 1H), 7.47 (s, 2H), 4.52 (q, *J* = 7.0 Hz, 2H), 4.28 (q, *J* = 7.1 Hz, 2H), 2.84 (dd, *J* = 12.9, 6.6 Hz, 2H), 2.66 (s, 6H), 1.40 (t, *J* = 7.1 Hz, 3H), 1.31 (t, *J* = 7.1 Hz, 5H), 1.24 - 1.14 (m, 8H), 0.81 (t, *J* = 7.0 Hz, 3H).

**Example 24 6-fluoro-1-(5-fluoropyridin-2-yl)-7-(4-(N-heptylsulfamoyl)phenyl)-4-oxo-1,4-dihydroquinolin-3-carboxylic acid**

Referring to the preparation processes in Examples 1 to 8, the target compound with the following structure was obtained by using similar starting materials as substitutes:

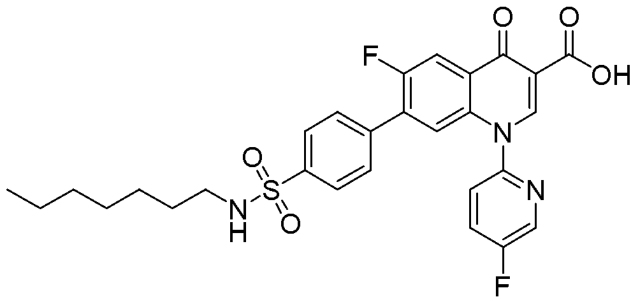

MS calculated: 555.16, MS found: 556.2 $[M+H]^+$.

$^1H$ NMR (400 MHz, DMSO-d6) δ 14.52 (s, 1H), 9.01 (s, 1H), 8.74 (d, *J* = 2.8 Hz, 1H), 8.26 (d, *J* = 10.2 Hz, 1H), 8.16 (td, *J* = 8.4, 2.7 Hz, 1H), 8.05 (dd, *J* = 8.8, 3.9 Hz, 1H), 7.88 (d, *J* = 8.3 Hz, 2H), 7.75 (d, *J* = 7.8 Hz, 2H), 7.69 (t, *J* = 5.8 Hz, 1H), 7.56 (d, *J* = 6.2 Hz, 1H), 2.76 (dd, *J* = 13.0, 6.6 Hz, 2H), 1.35 (dd, *J* = 13.0, 6.1 Hz, 2H), 1.21 (dd, *J* = 16.9, 9.4 Hz, 8H), 0.81 (t, *J* = 6.9 Hz, 3H).

**Example 25 6-fluoro-1-(2-fluoroethyl)-7-(4-(N-heptylsulfamoyl)phenyl)-4-oxo-1,4-dihydroquinolin-3-carboxylic acid**

Referring to the preparation processes in Examples 1 to 8, the target compound with the following structure was obtained by using similar starting materials as substitutes:

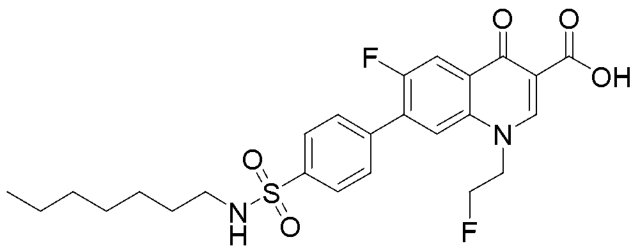

MS calculated: 506.17, MS found: 507.2 $[M+H]^+$.

$^1H$ NMR (400 MHz, DMSO-d6) δ 14.81 (s, 1H), 9.03 (s, 1H), 8.25 (s, 1H), 8.19 (d, J = 10.2 Hz, 1H), 7.95 (s, 4H), 7.73 (t, J = 5.6 Hz, 1H), 5.05 (d, J = 26.9 Hz, 2H), 4.93 (s, 1H), 4.81 (s, 1H), 2.79 (q, J = 6.6 Hz, 2H), 1.39 (q, J = 6.4 Hz, 2H), 1.28 - 1.14 (m, 8H), 0.83 (t, J = 6.9 Hz, 3H).

**Example 26 1-cyclopropyl-7-(4-(N-heptylsulfamoyl)phenyl)-6-nitro-4-oxo-1,4-dihydroquinolin-3-carboxylic acid**

Referring to the preparation processes in Examples 1 to 8, the target compound with the following structure was obtained by using similar starting materials as substitutes:

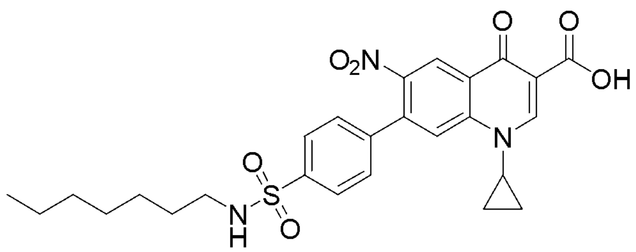

MS calculated: 527.17, MS found: 528.2 $[M+H]^+$.

$^1H$ NMR (400 MHz, DMSO-d6) δ 14.30 (s, 1H), 8.92 (s, 1H), 8.86 (s, 1H), 8.32 (s, 1H), 7.93 (d, *J*= 8.4 Hz, 2H), 7.79 - 7.69 (m, 3H), 3.92 (s, 1H), 2.81 (dd, *J* = 13.0, 6.8 Hz, 2H), 1.38 (d, *J*= 6.8 Hz, 2H), 1.33 - 1.28 (m, 2H), 1.26 - 1.19 (m, 10H), 0.84 (t, *J* = 6.9 Hz, 3H).

**Example 27 7-(4-(N-(6-aminohexyl)sulfamoyl)-3,5-dimethylphenyl)-1-cyclopropyl-6-fluoro-4-oxo-1,4-dihydroquinolin-3-carboxylic acid**

Referring to the preparation processes in Examples 1 to 8, the target compound with the following structure was obtained by using similar starting materials as substitutes:

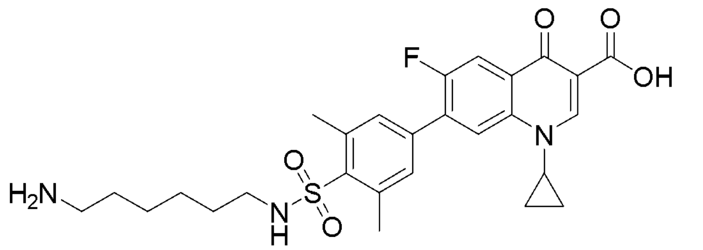

MS calculated: 529.20, MS found: 530.3 $[M+H]^+$.

$^1H$ NMR (400 MHz, DMSO-d6) δ 8.80 (s, 1H), 8.38 (d, *J*= 26.7 Hz, 3H), 8.11 (s, 1H), 7.56 (s, 3H), 4.05 - 3.85 (m, 1H), 2.82 (s, 2H), 2.70 (s, 8H), 1.48 - 1.30 (m, 6H), 1.21 (s, 6H).

**Example 28 ethyl 7-(4-(N-(6-aminohexyl)sulfamoyl)-3,5-dimethylphenyl)-1-cyclopropyl-6-fluoro-4-oxo-1,4-dihydroquinolin-3-carboxylate**

Referring to the preparation processes in Examples 1 to 8, the target compound with the following structure was obtained by using similar starting materials as substitutes:

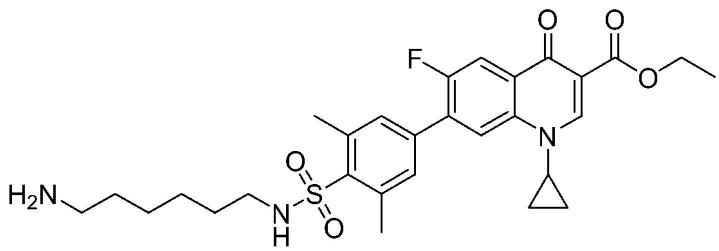

MS calculated: 557.24, MS found: 558.7 $[M+H]^+$.

$^1H$ NMR (400 MHz, DMSO-d6) δ 8.55 (s, 1H), 8.18 (d, *J* = 6.4 Hz, 1H), 7.99 (d, *J* = 10.6 Hz, 1H), 7.64 (dd, *J* = 13.3, 7.4 Hz, 3H), 7.54 (s, 2H), 4.24 (q, *J* = 7.1 Hz, 2H), 3.81 - 3.74 (m, 1H), 2.82 (dd, *J* = 13.0, 6.6 Hz, 2H), 2.77 - 2.72 (m, 2H), 2.70 (s, 6H), 1.47 (dd, *J* = 14.2, 7.6 Hz, 2H), 1.40 (dd, *J* = 13.6, 6.6 Hz, 2H), 1.32 - 1.21 (m, 9H), 1.14 (t, *J* = 7.7 Hz, 2H).

**Example 29 1-cyclopropyl-6-fluoro-7-(4-(N-(6-hydroxyhexyl)sulfamoyl)phenyl)-4-oxo-1,4-dihydroquinolin-3-carboxylic acid**

Referring to the preparation processes in Examples 1 to 8, the target compound with the following structure was obtained by using similar starting materials as substitutes:

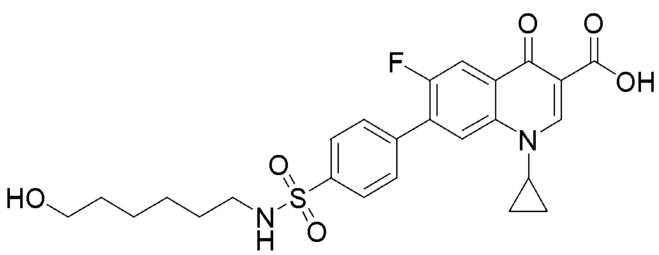

MS calculated: 502.16, MS found: 503.3 $[M+H]^+$.

$^1H$ NMR (400 MHz, DMSO-d6) δ 14.75 (s, 1H), 8.80 (s, 1H), 8.43 (d, *J* = 6.1 Hz, 1H), 8.18 (d, *J* = 10.2 Hz, 1H), 8.03 - 7.91 (m, 4H), 7.74 (t, *J* = 5.6 Hz, 1H), 4.30 (s, 1H), 3.97 (s, 1H), 3.34 (s, 2H), 2.91 - 2.70 (m, 2H), 1.43 - 1.18 (m, 12H).

**Example 30 1-cyclopropyl-6-fluoro-7-(4(N(5(methylthio)pentyl)sulfamoyl)phenyl)-4-oxo-1,4-dihydroquinolin-3-carboxylic acid**

Referring to the preparation processes in Examples 1 to 8, the target compound with the following structure was obtained by using similar starting materials as substitutes:

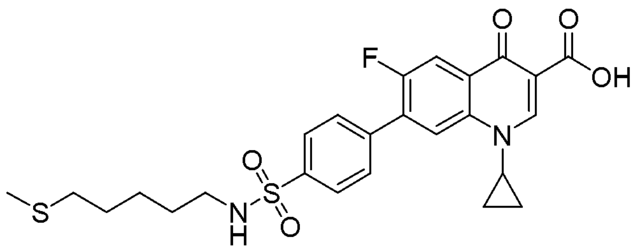

MS calculated: 518.13, MS found: 519.3 $[M+H]^+$.

$^1H$ NMR (400 MHz, DMSO-d6) δ 14.78 (s, 1H), 8.81 (s, 1H), 8.44 (d, J = 6.4 Hz, 1H), 8.19 (d, J = 10.3 Hz, 1H), 8.00 - 7.94 (m, 4H), 7.76 (t, J = 5.8 Hz, 1H), 4.00 - 3.94 (m, 1H), 2.82 (d, J = 6.4 Hz, 2H), 2.40 (t, J = 7.2 Hz, 2H), 2.00 (s, 3H), 1.65 - 1.09 (m, 10H).

**Example 31 1-cyclopropyl-6-fluoro-7-(5-(N-heptylsulfamoyl)pyridin-2-yl)-4-oxo-1,4-dihydroquinolin-3-carboxylic acid**

Referring to the preparation processes in Examples 1 and 2, the target compound with the following structure was obtained by using similar starting materials as substitutes:

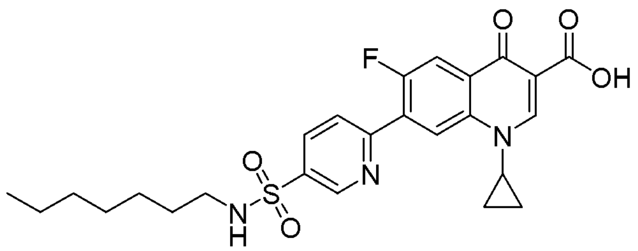

MS calculated: 501.17, MS found: 502.2 $[M+H]^+$.

$^1H$ NMR (400 MHz, DMSO-d6) δ 14.71 (s, 1H), 9.15 (d, *J* = 2.0 Hz, 1H), 8.86 (d, *J* = 6.4 Hz, 1H), 8.82 (s, 1H), 8.39 (dd, *J* = 8.0 Hz, 2.0 Hz, 1H), 8.22 (d, *J* = 10.8 Hz, 2H), 7.97 (t, *J* = 5.6 Hz, 1H), 4.02 - 3.90 (m, 1H), 2.87 (q, *J* = 6.8 Hz, 2H), 1.43-1.37 (m, 2H), 1.35-1.29 (m, 2H), 1.26-1.17 (m, 10H), 0.82 (t, *J* = 7.2 Hz, 3H).

**Example 32 7- (3-carbamoyl-4- (N-heptylsulfamoyl)phenyl)-1-cyclopropyl-6-fluoro-4-oxo-1,4-dihydroquinolin-3-carboxylic acid**

Referring to the preparation processes in Examples 1 to 8, the target compound with the following structure was obtained by using similar starting materials as substitutes:

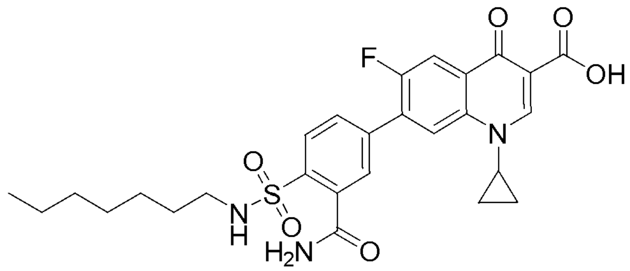

MS calculated: 543.18, MS found: 544.2 [M+H]$^+$.

$^1$H NMR (400 MHz, DMSO) δ 14.75 (s, 1H), 8.81 (s, 1H), 8.48 (s, 1H), 8.34 (s, 1H), 8.20 (d, J = 10.5 Hz, 1H), 8.07 - 8.00 (m, 2H), 7.93 (d, J = 12.1 Hz, 2H), 6.97 (t, J = 6.0 Hz, 1H), 4.00 - 3.94 (m, 1H), 2.89 (q, J = 6.5 Hz, 2H), 1.43 - 1.38 (m, 2H), 1.34 (d, J = 5.7 Hz, 2H), 1.25 - 1.15 (m, 10H), 0.81 (t, J = 6.8 Hz, 3H).

**Example 33 7-(3-amino-4-(N-heptylsulfonyl)phenyl)-1-cyclopropyl-6-fluoro-4-oxo-1,4-dihydroquinolin-3-carboxylic acid**

Referring to the preparation processes in Examples 1 to 8, the target compound with the following structure was obtained by using similar starting materials as substitutes:

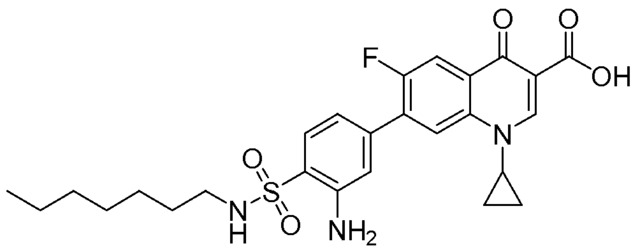

MS calculated: 515.19, MS found: 516.3 [M+H]$^+$.

$^1$H NMR (400 MHz, DMSO-d6) δ 8.79 (s, 1H), 8.34 (d, *J* = 5.6 Hz, 1H), 8.14 (d, *J* = 10.4 Hz, 1H), 7.66 (d, *J* = 8.0 Hz, 2H), 7.18 (s, 1H), 6.94 (d, J = 8.0 Hz, 1H), 6.17 (s, 2H), 4.00 - 3.90 (m, 1H), 2.78 (t, J = 6.8 Hz, 2H), 1.43-1.30 (m, 4H), 1.27-1.13 (m, 10H), 0.81 (t, J = 6.8 Hz, 3H).

**Example 34 8-chloro-1-cyclopropyl-6-fluoro-4-oxo-7-(6-(N-(4,4,4-trifluorobutyl)sulfamoyl)pyridin-3-yl)-1,4-dihydroquinolin-3-carboxylic acid**

Referring to the preparation processes in Examples 1 to 8, the target compound with the following structure was obtained by using similar starting materials as substitutes:

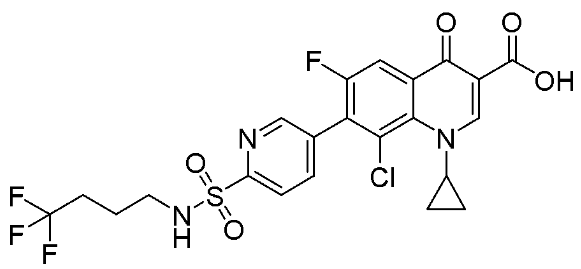

MS calculated: 547.06, MS found: 548.0 [M+H]$^+$.

$^1$H NMR (400 MHz, DMSO-d6) δ 14.15 (s, 1H), 8.93 (s, 1H), 8.88 (d, J = 2.0 Hz, 1H), 8.29 (dd, J = 8.1, 2.1 Hz, 1H), 8.22 (d, J = 8.6 Hz, 1H), 8.19 - 8.13 (m, 2H), 4.45 - 4.36 (m, 1H), 3.12 (t, J = 6.7 Hz, 2H), 2.32 - 2.22 (m, 2H), 1.67 - 1.59 (m, 2H), 1.24 - 1.19 (m, 2H), 1.18 - 1.12 (m, 2H).

**Example 35 1-cyclopropyl-6-fluoro-8-methoxy-7-(3-nitro-4-(2,2,2-triifluoroacetamido)phenyl)-4-oxo-1,4-dihydroquinolin-3-carboxylic acid**

Referring to the preparation processes in Examples 1 to 8, the target compound with the following structure was obtained by using similar starting materials as substitutes:

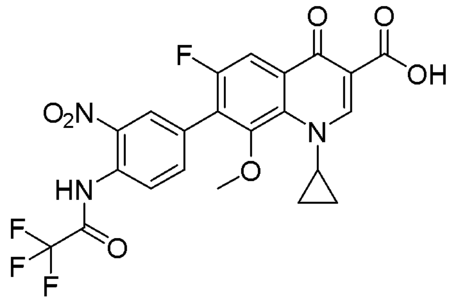

MS calculated: 509.08, MS found: 509.8 [M+H]$^+$.

$^1$H NMR (400 MHz, DMSO-d6) δ 14.61 (s, 1H), 11.91 (s, 1H), 8.82 (s, 1H), 8.26 (s, 1H), 7.99 (d, *J* = 9.1 Hz, 2H), 7.86 (d, *J*= 8.3 Hz, 1H), 4.27 - 4.14 (m, 1H), 3.46 (s, 3H), 1.17 (s, 4H).

According to similar processes, a series of compounds with the following structures were also synthesized in the present invention:

| Example No. | Structure | LC-MS | $^1$H NMR |
|---|---|---|---|
| **36** | 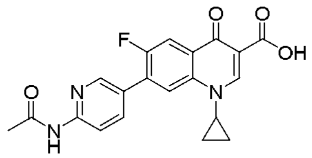 | 382.1 [M+H]$^+$ | $^1$H NMR (400 MHz, DMSO-d6) δ 14.83 (s, 1H), 10.76 (s, 1H), 8.79 (s, 1H), 8.69 (s, 1H), 8.43 (d, J = 6.3 Hz, 1H), 8.29 - 8.14 (m, 3H), 4.01 - 3.93 (m, 1H), 2.15 (s, 3H), 1.44 - 1.14 (m, 4H). |
| **37** | 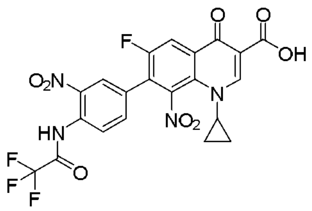 | 525.2 [M+H]$^+$ | $^1$H NMR (400 MHz, DMSO-d6) δ 13.90 (s, 1H), 11.94 (s, 1H), 8.90 (s, 1H), 8.49 (d, J = 8.4 Hz, 1H), 8.31 - 8.27 (m, 1H), 7.95 - 7.80 (m, 2H), 3.79 - 3.73 (m, 1H), 1.18 - 1.12 (m, 2H), 1.03 - 0.97 (m, 2H). |
| **38** | 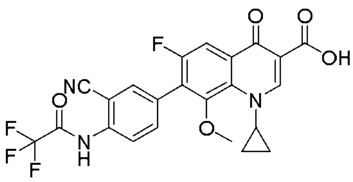 | 489.8 [M+H]$^+$ | $^1$H NMR (400 MHz, DMSO-d6) δ 14.60 (s, 1H), 11.94 (s, 1H), 8.82 (s, 1H), 8.21 (s, 1H), 8.04 - 7.91 (m, 2H), 7.81 (d, J = 8.4 Hz, 1H), 4.25 - 4.18 (m, 1H), 3.43 (s,3H), 1.27 - 1.09 (m, 4H). |
| **39** | 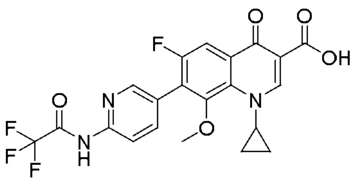 | 466.1 [M+H]$^+$ | $^1$H NMR (400 MHz, DMSO-d6) δ 14.62 (s, 1H), 12.24 (s, 1H), 8.82 (s, 1H), 8.65 (s, 1H), 8.17 (s, 2H), 7.99 (d, J = 9.1 Hz, 1H), 4.35 - 4.10 (m, 1H), 3.44 (s, 3H), 1.23 - 1.07 (m, 4H). |
| **40** | 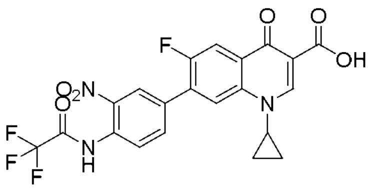 | 480.1 [M+H]$^+$ | $^1$H NMR (400 MHz, DMSO-d6) δ 14.77 (s, 1H), 11.95 (s, 1H), 8.81 (s, 1H), 8.51 (d, *J* = 6.4 Hz, 1H), 8.43 (s, 1H), 8.24-8.15 (m, 2H), 7.86 (d, *J* = 8.4 Hz, 1H), 4.03-3.92 (m, 1H), 1.37 - 1.32(m, 2H), 1.27-1.22 (m, 2H). |

(continued)

| Example No. | Structure | LC-MS | $^1$H NMR |
|---|---|---|---|
| **41** | 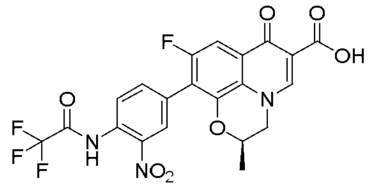 | 496.2 $[M+H]^+$ | $^1$H NMR (400 MHz, DMSO-d6) $\delta$ 14.94 (s, 1H), 11.86 (s, 1H), 8.96 (s, 1H), 8.27 (s, 1H), 7.98 (d, *J* = 8.5 Hz, 1H), 7.79 (dd, J = 20.2, 9.1 Hz, 2H), 4.75 (d, *J* = 11.7 Hz, 2H), 4.24 (dd, *J* = 13.9, 9.8 Hz, 1H), 1.39 (d, *J* = 6.3 Hz, 3H). |
| **42** | 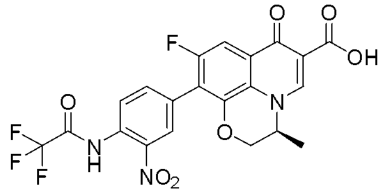 | 496.1 $[M+H]^+$ | $^1$H NMR (400 MHz, DMSO-d6) $\delta$ 14.93 (s, 1H), 11.91 (s, 1H), 9.11 (s, 1H), 8.28 (s, 1H), 8.00 (d, *J* = 8.3 Hz, 1H), 7.80 (dd, *J* = 9.0, 7.4 Hz, 2H), 5.01 (d, *J* = 6.8 Hz, 1H), 4.59 (dd, *J* = 11.6, 1.7 Hz, 1H), 4.47 (dd, *J* = 11.7, 2.5 Hz, 1H), 1.49 (d, *J* = 6.8 Hz, 3H). |
| **43** | 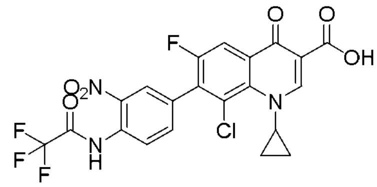 | 514.0 $[M+H]^+$ | $^1$H NMR (400 MHz, DMSO-d6) $\delta$ 14.21 (s, 1H), 11.93 (s, 1H), 8.94 (s, 1H), 8.28 (d, *J* = 2.0 Hz, 1H), 8.19 (d, *J* = 8.4 Hz, 1H), 7.95 (dd, *J* = 8.4 Hz, 1.6 Hz, 1H), 7.87 (d, *J* = 8.4 Hz, 1H), 4.47-4.35 (m, 1H), 1.26-1.12 (m, 4H). |
| **44** | 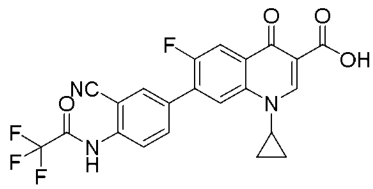 | 460.1 $[M+H]^+$ | $^1$H NMR (400 MHz, DMSO-d6) $\delta$ 14.79 (s, 1H), 11.98 (s, 1H), 8.80 (s, 1H), 8.49 (d, *J* = 6.4 Hz, 1H), 8.35 (s, 1H), 8.18 (s, 1H), 8.13 (s, 1H), 7.82 (d, *J* = 8.0 Hz, 1H), 4.01 - 3.95 (m, 1H), 1.36 -1.20 (m, 4H). |
| **45** | 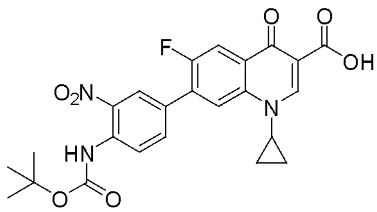 | 484.3 $[M+H]^+$ | $^1$H NMR (400 MHz, DMSO-d6) $\delta$ 14.75 (s, 1H), 9.07 (s, 1H), 8.82 (s, 1H), 8.55 (d, J = 6.0 Hz, 1H), 8.45 (d, J = 8.4 Hz, 1H), 8.23 (d, J = 10.0 Hz, 1H), 8.13 (d, J = 8.0 Hz, 1H), 7.64 (s, 2H), 4.02 - 3.89 (m, 1H), 1.38 - 1.32 (m, 2H), 1.28 - 1.21 (m, 2H). |
| **46** | 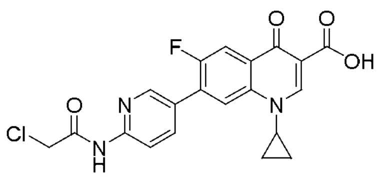 | 415.9 $[M+H]^+$ | $^1$H NMR (400 MHz, DMSO-d6) $\delta$ 14.82 (s, 1H), 11.09 (s, 1H), 8.84 - 8.68 (m, 2H), 8.45 (d, J = 6.3 Hz, 1H), 8.30 - 8.07 (m, 3H), 4.42 (s, 2H), 4.09 - 3.89 (m, 1H), 1.39 - 1.16 (m, 4H). |
| **47** | 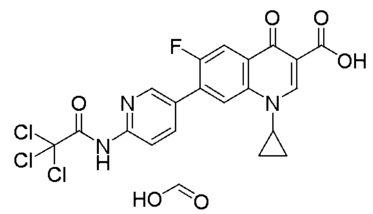 | 486.1 $[M+H]^+$ | $^1$H NMR (400 MHz, DMSO-d6) $\delta$ 14.82 (s, 1H), 11.81 (s, 1H), 8.81 (d, J = 5.9 Hz, 2H), 8.48 (d, J = 6.1 Hz, 1H), 8.30 (d, J = 9.1 Hz, 1H), 8.23 - 8.14 (m, 2H), 8.07 (d, J = 8.6 Hz, 1H), 4.01 - 3.95 (m, 1H), 1.40 - 1.22 (m, 4H). |
| **48** | 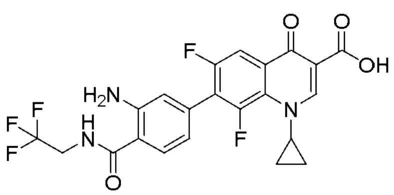 | 482.3 $[M+H]^+$ | $^1$H NMR (400 MHz, DMSO-d6) $\delta$ 14.50 (s, 1H), 8.95 (s, 1H), 8.78 (s, 1H), 8.03 (dd, J = 9.1, 1.5 Hz, 1H), 7.70 (d, J = 8.3 Hz, 1H), 6.90 (s, 1H), 6.69 (d, J = 11.1 Hz, 3H), 4.21 - 3.96 (m, 3H), 1.32 - 1.07 (m, 4H). |

(continued)

| Example No. | Structure | LC-MS | $^1$H NMR |
|---|---|---|---|
| **49** | 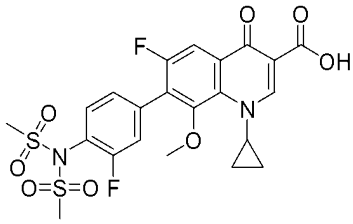 | 542.9 $[M+H]^+$ | $^1$H NMR (400 MHz, DMSO-d6) δ 14.56 (s, 1H), 8.83 (s, 1H), 8.01 - 7.81 (m, 2H), 7.73 (d, J = 10.6 Hz, 1H), 7.53 (d, J = 8.2 Hz, 1H), 4.27 - 4.12 (m, 1H), 3.60 (s, 6H), 3.43 (s, 3H), 1.33 - 1.06 (m, 4H). |
| **50** | 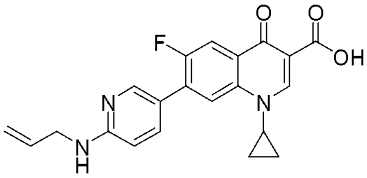 | 380.3 $[M+H]^+$ | $^1$H NMR (400 MHz, DMSO-d6) δ 8.56 (s, 1H), 8.31 (s, 1H), 8.18 (d, J = 6.5 Hz, 1H), 8.06 (s, 1H), 7.98 (d, J = 10.7 Hz, 1H), 7.54 (s, 1H), 6.94 (d, J = 9.0 Hz, 1H), 6.10 - 5.97 (m, 1H), 5.47 (d, J = 17.3 Hz, 1H), 5.26 (d, J = 12.0 Hz, 1H), 4.74 (d, J = 5.1 Hz, 2H), 3.76 - 3.73 (m, 1H), 1.30 - 1.26 (m, 2H), 1.17 - 1.12 (m, 2H). |
| **51** | 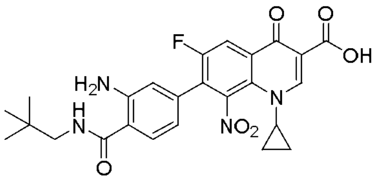 | 497.1 $[M+H]^+$ | $^1$H NMR (400 MHz, DMSO-d6) δ 13.92 (s, 1H), 8.87 (s, 1H), 8.41 (d, J = 8.3 Hz, 1H), 8.31 (t, J = 6.2 Hz, 1H), 7.59 (d, J = 8.1 Hz, 1H), 6.68 (s, 1H), 6.49 (d, J = 9.4 Hz, 3H), 3.82 - 3.68 (m, 1H), 3.08 (d, J = 6.3 Hz, 2H), 1.21 - 1.09 (m, 2H), 1.05 - 0.84 (m, 11H). |
| **52** | 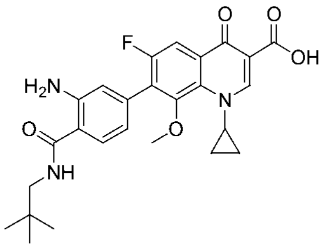 | 482.2 $[M+H]^+$ | $^1$H NMR (400 MHz, DMSO-d6) δ 14.68 (s, 1H), 8.79 (s, 1H), 8.31 - 8.24 (m, 1H), 7.96 - 7.83 (m, 1H), 7.65 (d, J = 8.1 Hz, 1H), 6.84 (s, 1H), 6.67 (d, J = 7.8 Hz, 1H), 6.44 (s, 2H), 4.26 - 4.10 (m, 2H), 3.48 (s, 3H), 3.10 (d, J = 6.3 Hz, 2H), 1.19 - 1.10 (m, 4H), 0.92 (s, 9H). |
| **53** | 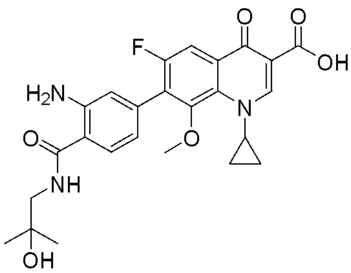 | 484.7 $[M+H]^+$ | $^1$H NMR (400 MHz, DMSO-d6) δ 14.68 (s, 1H), 8.81 (s, 1H), 8.12 (t, J = 6.0 Hz, 1H), 7.91 (d, J = 9.0 Hz, 1H), 7.67 (d, J = 8.2 Hz, 1H), 6.85 (s, 1H), 6.67 (d, J = 8.0 Hz, 1H), 6.48 (s, 2H), 4.56 (s, 1H), 4.27 - 4.14 (m, 1H), 3.49 (s, 3H), 3.25 (d, J = 6.1 Hz, 2H), 1.19 - 1.10 (m, 10H). |
| **54** | 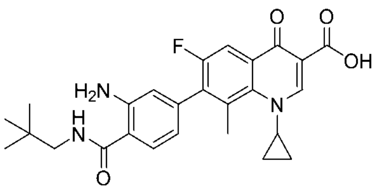 | 466.2 $[M+H]^+$ | $^1$H NMR (400 MHz, DMSO-d6) δ 14.70 (s, 1H), 8.90 (s, 1H), 8.31 - 8.22 (m, 1H), 8.01 - 7.91 (m, 1H), 7.69 - 7.60 (m, 1H), 6.72 - 6.66 (m, 1H), 6.54 - 6.48 (m, 1H), 6.46 - 6.36 (m, 2H), 4.47 - 4.33 (m, 1H), 3.09 (s, 2H), 2.64 (s, 3H), 1.25 - 1.20 (m, 2H), 1.07 - 1.03 (m, 2H), 0.92 (s, 9H). |
| **55** | 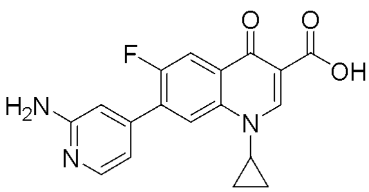 | 340.0 $[M+H]^+$ | $^1$H NMR (400 MHz, DMSO-d6) δ 14.80 (s, 1H), 8.80 (s, 1H), 8.35 (d, J = 6.8 Hz, 1H), 8.16 (d, J = 10.4 Hz, 1H), 8.09 (d, J = 5.2 Hz, 1H), 6.79 (d, J = 5.2 Hz, 1H), 6.74 (s, 1H), 6.21 (s, 2H), 3.99 - 3.91 (m, 1H), 1.40 - 1.14 (m, 4H). |

(continued)

| Example No. | Structure | LC-MS | $^1$H NMR |
|---|---|---|---|
| **56** | 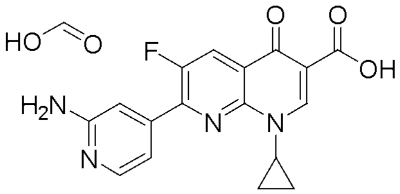 | 341.3 $[M+H]^+$ | $^1$H NMR (400 MHz, DMSO-d6) δ 14.33 (s, 1H), 8.88 (s, 1H), 8.61 (d, J = 10.5 Hz, 1H), 8.14 (d, J = 5.3 Hz, 1H), 7.25 - 7.16 (m, 2H), 6.29 (s, 2H), 3.95 - 3.86 (m, 1H), 1.37 - 1.29 (m, 2H), 1.24 - 1.13 (m, 2H). |
| **57** | 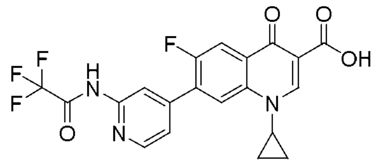 | 436.2 $[M+H]^+$ | $^1$H NMR (400 MHz, DMSO-d6) δ 8.82 (s, 1H), 8.65 (d, J = 5.1 Hz, 1H), 8.47 (d, J = 6.1 Hz, 1H), 8.27 (s, 1H), 8.21 (d, J = 10.1 Hz, 1H), 7.62 (d, J = 4.7 Hz, 1H), 4.00 - 3.93 (m, 1H), 1.36 - 1.21 (m, 4H). |
| **58** | 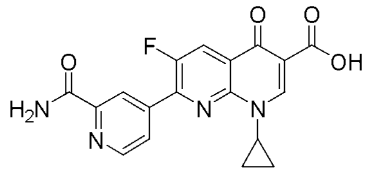 | 369.2 $[M+H]^+$ | $^1$H NMR (400 MHz, DMSO-d6) δ 14.21 (s, 1H), 8.91 (d, J = 4.2 Hz, 2H), 8.79 (s, 1H), 8.69 (d, J = 8.4 Hz, 1H), 8.35 - 8.21 (m, 2H), 7.82 (s, 1H), 4.03 - 3.88 (m, 1H), 1.39 - 1.16 (m, 4H). |
| **59** | 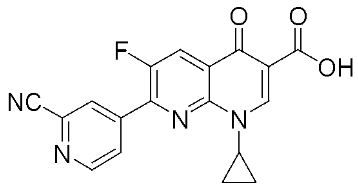 | 351.2 $[M+H]^+$ | $^1$H NMR (400 MHz, DMSO-d6) δ 14.25 (s, 1H), 9.05 (d, J = 5.1 Hz, 1H), 8.92 (s, 1H), 8.76 (d, J = 10.2 Hz, 1H), 8.68 (s, 1H), 8.45 (d, J = 5.2 Hz, 1H), 4.07 - 3.95 (m, 1H), 1.38 - 1.19 (m, 4H). |
| **60** | 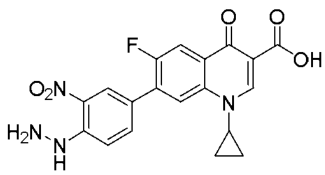 | 399.2 $[M+H]^+$ | $^1$H NMR (400 MHz, DMSO-d6) δ 14.88 (s, 1H), 9.42 (s, 1H), 8.78 (s, 1H), 8.46 - 8.34 (m, 2H), 8.12 (d, J = 10.8 Hz, 1H), 7.95 (d, J = 8.8 Hz, 1H), 7.79 (d, J = 9.2 Hz, 1H), 4.84 (s, 2H), 4.04 - 3.93 (m, 1H), 1.37 - 1.31 (m, 2H), 1.26 - 1.22 (m, 2H). |
| **61** | 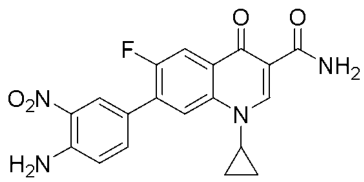 | 383.3 $[M+H]^+$ | $^1$H NMR (400 MHz, DMSO-d6) δ 9.13 (d, J = 4.1 Hz, 1H), 8.72 (s, 1H), 8.35 (s, 1H), 8.23 (d, J = 6.7 Hz, 1H), 8.04 (d, J = 11.1 Hz, 1H), 7.80 (d, J = 8.9 Hz, 1H), 7.73 (s, 2H), 7.55 (d, J = 4.0 Hz, 1H), 7.21 (d, J = 8.9 Hz, 1H), 3.90 - 3.83 (m, 1H), 1.36 - 1.25 (m, 2H), 1.20 - 1.10 (m, 2H). |
| **62** | 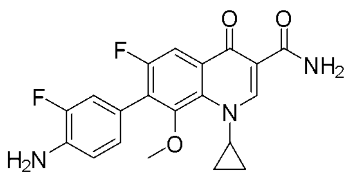 | 386.2 $[M+H]^+$ | $^1$H NMR (400 MHz, DMSO-d6) δ 9.06 (d, J = 4.0 Hz, 1H), 8.74 (s, 1H), 7.82 (d, J = 9.7 Hz, 1H), 7.54 (d, J = 4.1 Hz, 1H), 7.19 (d, J = 12.4 Hz, 1H), 7.08 (d, J = 8.1 Hz, 1H), 6.90 (t, J = 8.9 Hz, 1H), 5.51 (s, 2H), 4.19 - 4.08 (m, 1H), 3.38 (s, 3H), 1.19 - 1.02 (m, 4H). |
| **63** | 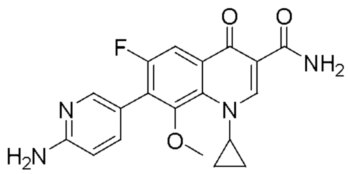 | 369.1 $[M+H]^+$ | $^1$H NMR (400 MHz, DMSO-d6) δ 9.06 (d, J = 4.1 Hz, 1H), 8.75 (s, 1H), 8.07 (s, 1H), 7.84 (d, J = 9.7 Hz, 1H), 7.56 (d, J = 4.9 Hz, 2H), 6.59 (d, J = 8.5 Hz, 1H), 6.30 (s, 2H), 4.16 - 4.09 (m, 1H), 3.41 (s, 3H), 1.17 - 1.11 (m, 2H), 1.07 - 1.01 (m, 2H). |

(continued)

| Example No. | Structure | LC-MS | 1H NMR |
|---|---|---|---|
| **64** | 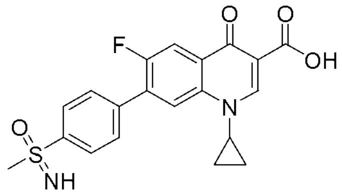 | 401.2 [M+H]+ | 1H NMR (400 MHz, DMSO-d6) δ 14.77 (s, 1H), 8.80 (s, 1H), 8.42 (d, J = 5.8 Hz, 1H), 8.18 (d, J = 10.3 Hz, 1H), 8.13 (d, J = 8.1 Hz, 2H), 7.96 (d, J = 7.6 Hz, 2H), 4.37 (s, 1H), 4.03 - 3.90 (m, 1H), 3.16 (s, 3H), 1.36 - 1.22 (m, 4H). |
| **65** | 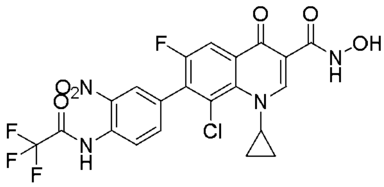 | 529.1 [M+H]+ | 1H NMR (400 MHz, DMSO-d6) δ 11.93 (s, 1H), 11.35 (d, J = 1.4 Hz, 1H), 9.33 (d, J = 1.7 Hz, 1H), 8.84 (s, 1H), 8.14 (d, J = 5.1 Hz, 1H), 8.11 (d, J = 8.8 Hz, 1H), 7.86 - 7.80 (m, 2H), 4.39 - 4.32 (m, 1H), 1.24 - 1.18 (m, 2H), 1.10 - 1.04 (m, 2H). |
| **66** | 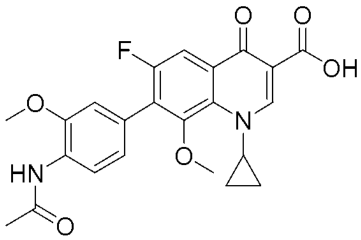 | 441. 1 [M+H]+ | 1H NMR (400 MHz, DMSO-d6) δ 14.70 (s, 1H), 9.31 (s, 1H), 8.80 (s, 1H), 8.16 (d, J = 8.5 Hz, 1H), 7.91 (d, J = 8.9 Hz, 1H), 7.20 (s, 1H), 7.08 (d, J = 8.0 Hz, 1H), 4.28 - 4.19 (m, 1H), 3.87 (s, 3H), 3.43 (s, 3H), 2.14 (s, 3H), 1.19 - 1.13 (m, 4H). |
| **67** | 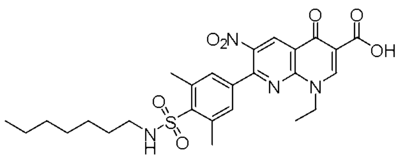 | 545.2 [M+H]+ | 1H NMR (400 MHz, DMSO-d6) δ 14.70 (s, 1H), 9.31 (s, 1H), 8.80 (s, 1H), 8.16 (d, J = 8.5 Hz, 1H), 7.91 (d, J = 8.9 Hz, 1H), 7.20 (s, 1H), 7.08 (d, J = 8.0 Hz, 1H), 4.28 - 4.19 (m, 1H), 3.87 (s, 3H), 3.43 (s, 3H), 2.14 (s, 3H), 1.19 - 1.13 (m, 4H). |
| **68** | 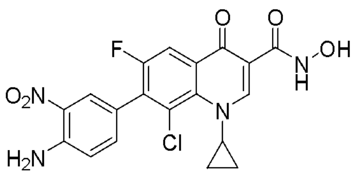 | 433.0 [M+H]+ | 1H NMR (400 MHz, DMSO-d6) δ 11.93 (s, 1H), 11.35 (d, J = 1.4 Hz, 1H), 9.33 (d, J = 1.7 Hz, 1H), 8.84 (s, 1H), 8.14 (d, J = 5.1 Hz, 1H), 8.11 (d, J = 8.8 Hz, 1H), 7.86 - 7.80 (m, 2H), 4.39 - 4.32 (m, 1H), 1.24 - 1.18 (m, 2H), 1.10 - 1.04 (m, 2H). |
| **69** | 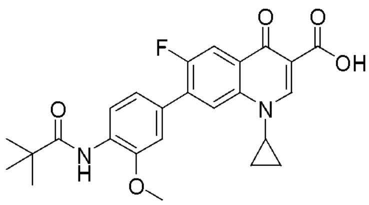 | 453.1 [M+H]+ | 1H NMR (400 MHz, DMSO-d6) δ 14.88 (s, 1H), 9.16 (s, 1H), 8.69 (s, 1H), 8.17 (d, J = 8.0 Hz, 1H), 7.90 (d, J = 7.6 Hz, 1H), 7.62 - 7.20 (m, 3H), 4.02 - 3.72 (m, 4H), 1.50 - 1.04 (m, 13H). |
| **70** | 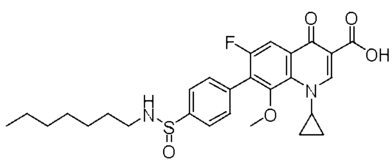 | 515.3 [M+H]+ | 1H NMR (400 MHz, DMSO-d6) δ 14.85 (s, 1H), 8.71 (s, 1H), 8.01 (d, J = 8.0 Hz, 1H), 7.76 - 7.42 (m, 4H), 4.93 - 4.69 (m, 1H), 4.16 - 3.81 (m, 4H), 3.13 - 2.87 (m, 2H), 1.66 - 1.44 (m, 2H), 1.40 - 0.80 (m, 15H). |
| **71** | 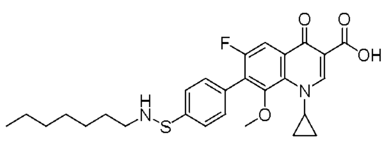 | 499.2 [M+H]+ | 1H NMR (400 MHz, DMSO-d6) δ 14.85 (s, 1H), 8.71 (s, 1H), 8.03 (d, J = 7.8 Hz, 2H), 7.61 - 7.32 (m, 4H), 4.62 - 4.40 (m, 2H), 4.16 - 3.79 (m, 4H), 2.87 - 2.66 (m, 2H), 1.68 - 0.79 (m, 17H). |

(continued)

| Example No. | Structure | LC-MS | $^1$H NMR |
|---|---|---|---|
| **72** | 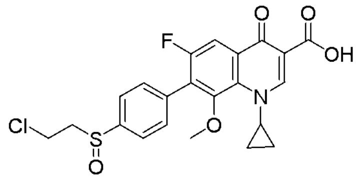 | 464.1 [M+H]$^+$ | $^1$H NMR (400 MHz, DMSO-d6) δ 14.65 (s, 1H), 8.82 (s, 1H), 7.97 (d, J = 9.2 Hz, 1H), 7.91 (d, J = 8.4 Hz, 2H), 7.79 (d, J = 8.0 Hz, 2H), 4.27 - 4.17 (m, 1H), 4.03 - 3.94 (m, 1H), 3.89 (dt, J = 11.2, 5.2 Hz, 1H), 3.58 - 3.46 (m, 1H), 3.39 (s, 3H), 3.36 (d, J = 5.2 Hz, 1H), 1.25 - 1.14 (m, 4H). |
| **73** | 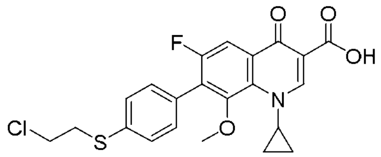 | 448.1 [M+H]$^+$ | $^1$H NMR (400 MHz, DMSO-d6) δ 14.69 (s, 1H), 8.80 (s, 1H), 7.93 (d, J = 9.2 Hz, 1H), 7.55 (q, J = 8.4 Hz, 4H), 4.28 - 4.08 (m, 1H), 3.83 (t, J = 7.2 Hz, 2H), 3.47 (t, J = 7.2 Hz, 2H), 3.39 (s, 3H), 1.25 - 1.14 (m, 4H) |
| **74** | 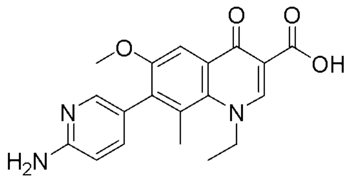 | 354.2 [M+H]$^+$ | $^1$H NMR (400 MHz, DMSO-d6) δ 14.78 (s, 1H), 8.81 (s, 1H), 7.89 - 7.80 (m, 2H), 7.28 (dd, J = 7.6, 1.5 Hz, 1H), 6.59 (d, J = 7.3 Hz, 1H), 6.50 - 6.44 (m, 2H), 4.25 - 4.18 (m, 2H), 3.93 (s, 3H), 2.34 (s, 3H), 1.44 (t, J = 7.9 Hz, 3H). |
| **75** | 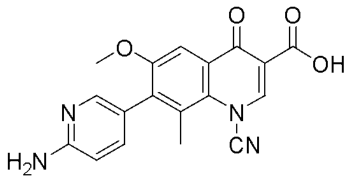 | 351.1 [M+H]$^+$ | $^1$H NMR (400 MHz, DMSO-d6) δ 8.15 (s, 1H), 7.85 (d, J = 1.6 Hz, 1H), 7.76 (s, 1H), 7.24 (dd, J = 7.5, 1.5 Hz, 1H), 6.61 (d, J = 7.5 Hz, 1H), 6.22 (d, J = 7.0 Hz, 1H), 6.04 (d, J = 7.0 Hz, 1H), 3.93 (s, 3H), 2.29 (s, 3H). |
| **76** | 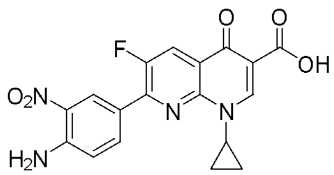 | 385.2 [M+H]$^+$ | $^1$H NMR (400 MHz, DMSO-d6) δ 14.78 (s, 1H), 9.05 (s, 1H), 8.23 (t, J = 1.8 Hz, 1H), 7.80 - 7.70 (m, 2H), 7.40 - 7.36 (m, 3H), 4.08 - 4.02 (m, 1H), 1.32 - 1.13 (m, 4H). |
| **77** | 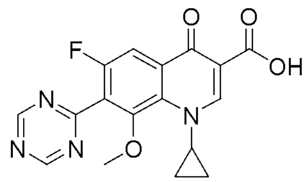 | 357.3 [M+H]$^+$ | $^1$H NMR (400 MHz, DMSO-d6) δ 14.88 (s, 1H), 9.28 (s, 2H), 8.71 (s, 1H), 8.23 (d, J = 8.0 Hz, 1H), 4.17 - 3.79 (m, 4H), 1.39 - 0.84 (m, 4H). |
| **78** | 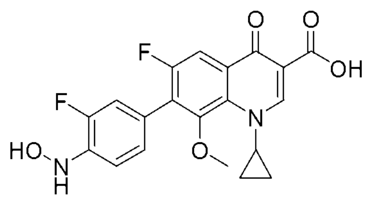 | 403.4 [M+H]$^+$ | $^1$H NMR (400 MHz, DMSO-d6) δ 14.88 (s, 1H), 9.12 (d, J = 3.9 Hz, 1H), 8.72 (s, 1H), 8.00 (d, J = 8.0 Hz, 1H), 7.35 - 6.95 (m, 4H), 4.17 - 3.79 (m, 4H), 1.39 - 0.83 (m, 4H). |
| **79** | 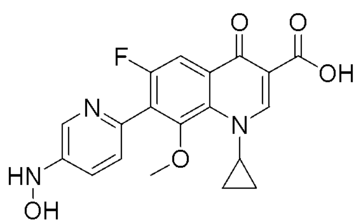 | 386.1 [M+H]$^+$ | $^1$H NMR (400 MHz, DMSO-d6) δ 14.88 (s, 1H), 9.22 (d, J = 4.9 Hz, 1H), 8.71 (s, 1H), 8.45 (d, J = 4.9 Hz, 1H), 8.28 - 8.01 (m, 2H), 7.66 - 7.36 (m, 2H), 4.19 - 3.76 (m, 4H), 1.33 - 0.84 (m, 4H). |

(continued)

| Example No. | Structure | LC-MS | 1H NMR |
|---|---|---|---|
| **80** | 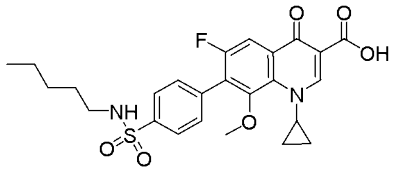 | 503.3 [M+H]+ | 1H NMR (400 MHz, DMSO-d6) δ 14.63 (s, 1H), 8.82 (s, 1H), 8.01 - 7.94 (m, 3H), 7.79 (d, J = 8.1 Hz, 2H), 7.72 (t, J = 5.7 Hz, 1H), 4.28 - 4.16 (m, 1H), 3.39 (s, 3H), 2.82 (q, J = 6.8 Hz, 2H), 1.43 - 1.27 (m, 10H), 0.87 - 0.74 (m, 3H). |
| **81** | 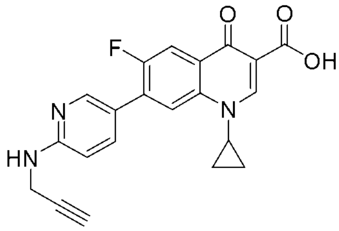 | 378.5 [M+H]+ | 1H NMR (400 MHz, DMSO-d6) δ 14.82 (s, 1H), 9.01 (s, 1H), 8.81 (d, J = 5.1 Hz, 1H), 8.47 (d, J = 6.4 Hz, 1H), 8.19 (d, J = 10.5 Hz, 1H), 7.89 (s, 1H), 7.74 - 7.60 (m, 2H), 4.05 - 3.88 (m, 1H), 2.41 (s, 3H), 1.36 - 1.24 (m, 4H). |
| **82** | 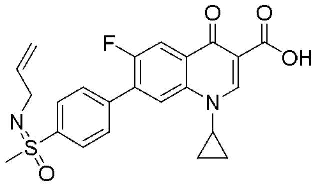 | 441.4 [M+H]+ | 1H NMR (400 MHz, DMSO-d6) δ 14.88 (s, 1H), 8.69 (s, 1H), 8.26 (d, J = 8.0 Hz, 1H), 7.88 - 7.74 (m, 3H), 7.58 (dq, J = 8.0, 1.7 Hz, 3H), 7.37 (d, J = 5.0 Hz, 1H), 5.88 (ddt, J = 14.1, 12.4, 6.1 Hz, 2H), 5.24 (dq, J = 13.4, 0.9 Hz, 3H), 4.26 (dt, J = 6.3, 1.0 Hz, 3H), 4.10 - 3.98 (m, 1H), 3.23 (s, 3H), 1.45 - 1.03 (m, 6H). |
| **83** | 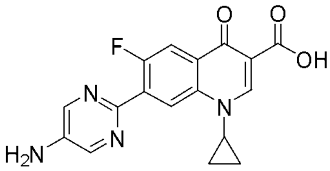 | 341.3 [M+H]+ | 1H NMR (400 MHz, DMSO-d6) δ 14.78 (s, 1H), 8.67 (s, 1H), 8.40 - 8.33 (m, 3H), 7.57 (d, J = 5.0 Hz, 1H), 5.60 (s, 1H), 3.98 - 3.91 (m, 1H), 1.31 - 1.10 (m, 4H). |
| **84** | 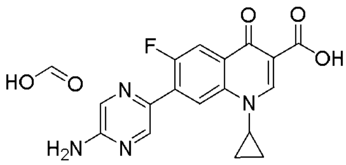 | 341.2 [M+H]+ | 1H NMR (400 MHz, DMSO-d6) δ 14.88 (s, 1H), 8.77 (d, J = 5.1 Hz, 2H), 8.56 (s, 1H), 8.10 (d, J = 9.9 Hz, 2H), 7.04 (s, 2H), 3.98 - 3.88 (m, 1H), 1.36 - 1.19 (m, 4H). |
| **85** | 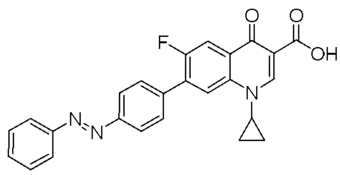 | 428.5 [M+H]+ | 1H NMR (400 MHz, DMSO-d6) δ 14.88 (s, 1H), 8.69 (s, 1H), 8.24 (d, J = 7.9 Hz, 1H), 7.99 - 7.83 (m, 4H), 7.78 - 7.65 (m, 2H), 7.59 - 7.39 (m, 4H), 7.35 (d, J = 5.0 Hz, 1H), 3.98 - 3.90 (m, 1H), 1.32 - 1.06 (m, 4H). |
| **86** | 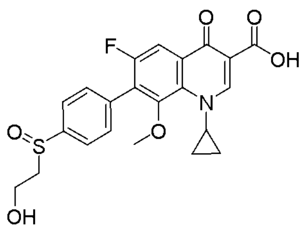 | 446.1 [M+H]+ | 1H NMR (400 MHz, DMSO-d6) δ 14.64 (s, 1H), 8.82 (s, 1H), 7.96 (d, J = 9.1 Hz, 1H), 7.87 (d, J = 8.1 Hz, 2H), 7.76 (d, J = 8.1 Hz, 2H), 5.10 (t, J = 5.0 Hz, 1H), 4.30 - 4.11 (m, 1H), 3.95 - 3.76 (m, 1H), 3.72 - 3.63 (m, 1H), 3.39 (s, 3H), 3.17 - 3.07 (m, 1H), 3.01 - 2.92 (m, 1H), 1.25 - 1.14 (m, 4H). |

(continued)

| Example No. | Structure | LC-MS | $^1$H NMR |
|---|---|---|---|
| **87** | 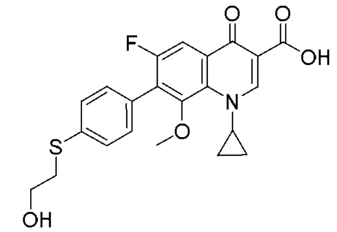 | 430.1 [M+H]$^+$ | $^1$H NMR (400 MHz, DMSO-d6) δ 14.69 (s, 1H), 8.80 (s, 1H), 7.92 (d, J = 9.2 Hz, 1H), 7.63 - 7.20 (m, 4H), 5.01 (t, J = 5.6 Hz, 1H), 4.32 - 4.05 (m, 1H), 3.64 (dd, J = 12.4, 6.8 Hz, 2H), 3.39 (s, 3H), 3.14 (t, J = 6.8 Hz, 2H), 1.27 - 1.12 (m, 4H). |
| **88** | 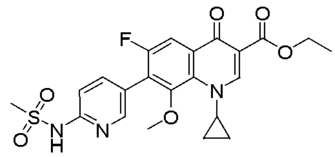 | 476.3 [M+H]$^+$ | $^1$H NMR (400 MHz, DMSO-d6) δ 10.93 (s, 1H), 8.57 (s, 1H), 8.41 (s, 1H), 7.91 (d, J = 7.7 Hz, 1H), 7.80 (d, J = 9.5 Hz, 1H), 7.11 (d, J = 8.6 Hz, 1H), 4.24 (q, J = 7.1 Hz, 2H), 4.09 - 4.01 (m, 1H), 3.41 (s, 3H), 3.35 (s, 3H), 1.29 (t, J = 7.1 Hz, 3H), 1.16 - 1.01 (m, 4H). |
| **89** | 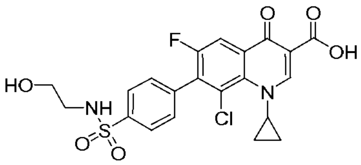 | 481.2 [M+H]$^+$ | $^1$H NMR (400 MHz, DMSO-d6) δ 14.25 (s, 1H), 8.94 (s, 1H), 8.18 (d, J = 8.5 Hz, 1H), 7.99 (d, J = 8.4 Hz, 2H), 7.79 (t, J = 5.9 Hz, 1H), 7.70 (d, J = 8.2 Hz, 2H), 4.75 (t, J = 5.6 Hz, 1H), 4.43 - 4.36 (m, 1H), 3.42 (q, J = 6.1 Hz, 2H), 2.89 (q, J = 6.2 Hz, 2H), 1.24 - 1.10 (m, 4H). |
| **90** | 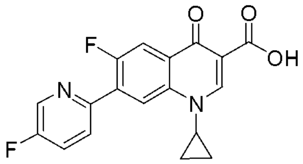 | 343.3 [M+H]$^+$ | 1H NMR (400 MHz, DMSO-d6) δ 14.78 (s, 1H), 8.87 (d, J = 2.8 Hz, 1H), 8.83 - 8.75 (m, 2H), 8.19 (d, J = 11.2 Hz, 1H), 8.09 (s, 1H), 7.99 (dd, J = 8.4 Hz, 5.6 Hz, 1H), 4.01 - 3.93 (m, 1H), 1.33-1.23 (m, 4H). |
| **91** | 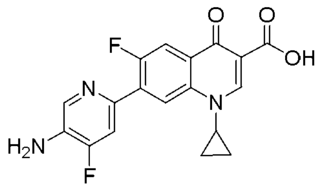 | 358.1 [M+H]$^+$ | $^1$H NMR (400 MHz, DMSO-d6) δ 14.68 (s, 1H), 8.67 (s, 1H), 8.23- 8.14 (m, 2H), 7.83 - 7.79 (m, 3H), 7.32 - 7.26 (m, 1H), 3.87 (p, J = 7.0 Hz, 1H), 1.32 - 1.19 (m, 4H). |
| **92** | 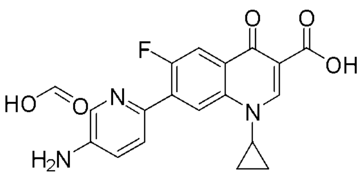 | 340.0 [M+H]$^+$ | $^1$H NMR (400 MHz, DMSO-d6) δ 14.92 (s, 1H), 8.82 (d, J = 6.6 Hz, 1H), 8.76 (s, 1H), 8.17 (d, J = 2.6 Hz, 1H), 8.06 (d, J = 11.5 Hz, 1H), 7.73 (d, J = 7.1 Hz, 1H), 7.06 (dd, J = 8.6, 2.6 Hz, 1H), 5.91 (s, 2H), 3.98 - 3.88 (m, 1H), 1.34 - 1.19 (m, 4H). |
| **93** | 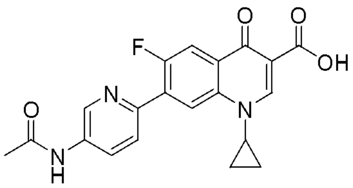 | 382.3 [M+H]$^+$ | $^1$H NMR (400 MHz, DMSO-d6) δ 10.44 (s, 1H), 8.97 (d, J = 2.3 Hz, 1H), 8.84 (d, J = 6.5 Hz, 1H), 8.79 (s, 1H), 8.23 (dd, J = 8.7, 2.5 Hz, 1H), 8.14 (d, J = 11.1 Hz, 1H), 7.98 (dd, J = 8.7, 1.8 Hz, 1H), 3.98 - 3.94 (m, 1H), 2.14 (s, 3H), 1.37 - 1.21 (m, 4H). |
| **94** | 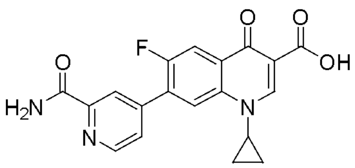 | 368.2 [M+H]$^+$ | $^1$H NMR (400 MHz, DMSO-d6) δ 14.73 (s, 1H), 8.85 (d, J = 5.0 Hz, 1H), 8.82 (s, 1H), 8.51 (d, J = 6.3 Hz, 1H), 8.35 (s, 1H), 8.28 - 8.19 (m, 2H), 7.97 (d, J = 4.8 Hz, 1H), 7.81 (s, 1H), 4.05 - 3.92 (m, 1H), 1.37 - 1.29 (m, 2H), 1.28 - 1.22 (m, 2H). |

(continued)

| Example No. | Structure | LC-MS | $^1$H NMR |
|---|---|---|---|
| **95** | 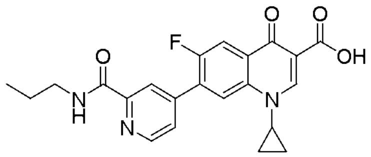 | 410.5 [M+H]+ | $^1$H NMR (400 MHz, DMSO-d6) δ 14.74 (s, 1H), 8.94 (t, J = 6.0 Hz, 1H), 8.86 (d, J = 5.0 Hz, 1H), 8.82 (s, 1H), 8.51 (d, J = 6.1 Hz, 1H), 8.34 (s, 1H), 8.22 (d, J = 10.4 Hz, 1H), 7.97 (d, J = 5.0 Hz, 1H), 4.07 - 3.93 (m, 1H), 3.30 - 3.27 (m, 2H), 1.58 (dd, J = 14.6, 7.2 Hz, 2H), 1.36 - 1.21 (m, 4H), 0.90 (t, J = 7.4 Hz, 3H). |
| **96** | 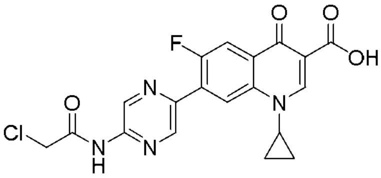 | 417.9 [M+H]+ | $^1$H NMR (400 MHz, DMSO-d6) δ 14.89 (s, 1H), 10.51 (s, 1H), 8.68 (s, 1H), 8.52 - 8.38 (m, 3H), 7.65 (d, J = 5.1 Hz, 1H), 4.31 (d, J = 5.6 Hz, 2H), 4.05 - 3.95 (m, 1H), 1.32 - 1.06 (m, 4H). |
| **97** | 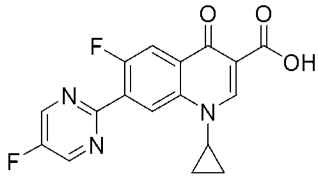 | 344.2 [M+H]+ | $^1$H NMR (400 MHz, DMSO-d6) δ 14.88 (s, 1H), 8.67 (s, 1H), 8.52 (d, J = 7.9 Hz, 2H), 8.37 (d, J = 7.9 Hz, 1H), 7.60 (d, J = 5.1 Hz, 1H), 4.06 - 3.99 (m, 1H), 1.31 - 1.22 (m, 2H), 1.18 - 1.08 (m, 2H). |
| **98** | 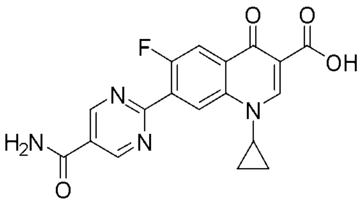 | 369.0 [M+H]+ | $^1$H NMR (400 MHz, DMSO-d6) δ 14.68 (s, 1H), 9.31 - 9.26 (m, 2H), 8.67 (s, 1H), 8.33 - 8.25 (m, 3H), 7.61 (d, J = 5.0 Hz, 1H), 4.02 - 3.96 (m, 1H), 1.33 - 1.25 (m, 2H), 1.21 - 1.08 (m, 2H). |
| **99** | 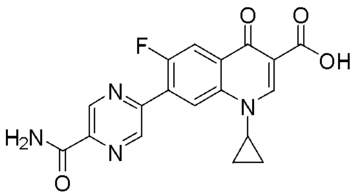 | 369.0 [M+H]+ | $^1$H NMR (400 MHz, DMSO-d6) δ 14.70 (s, 1H), 9.52 (s, 1H), 8.96 (d, J = 2.1 Hz, 1H), 8.67 (s, 1H), 8.43 (d, J = 7.9 Hz, 1H), 7.86 (d, J = 12.3 Hz, 1H), 7.80 - 7.68 (m, 3H), 3.91 - 3.90 (m, 1H), 1.28 - 1.20 (m, 2H), 1.16 - 1.08 (m, 2H). |
| **100** | 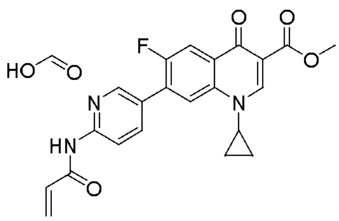 | 408.3 [M+H]+ | $^1$H NMR (400 MHz, DMSO-d6) δ 10.96 (s, 1H), 8.69 (s, 1H), 8.56 (s, 1H), 8.43 (s, 1H), 8.38 (d, J = 8.7 Hz, 1H), 8.24 (d, J = 6.4 Hz, 1H), 8.18 (d, J = 8.8 Hz, 1H), 8.00 (d, J = 10.7 Hz, 1H), 6.66 (dd, J = 17.0, 10.2 Hz, 1H), 6.36 (dd, J = 17.0, 1.8 Hz, 1H), 5.83 (dd, J = 10.2, 1.7 Hz, 1H), 3.81 - 3.76 (m, 4H), 1.30 - 1.12 (m, 4H). |
| **101** | 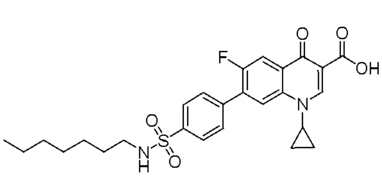 | 501.0 [M+H]+ | $^1$H NMR (400 MHz, DMSO-d6) δ 14.78 (s, 1H), 8.80(s, 1H), 8.44 (d, J = 10.4 Hz 1H), 8.19 (d, J = 10.4 Hz 1H), 8.00-7.94 (m, 4H), 7.77-7.72 (m, 1H),4.00-3.93 (m, 1H), 2.81 (q, J = 13.0 Hz, J =6.8 Hz, 2H), 1.43-1.30(m, 4H), 1.26-1.16 (m, 10H), 0.82 (t, J = 6.9 Hz, 3H). |

(continued)

| Example No. | Structure | LC-MS | $^1H$ NMR |
|---|---|---|---|
| **102** | 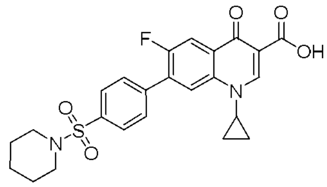 | 471.1 $[M+H]^+$ | $^1H$ NMR (400 MHz, DMSO-d6) δ 14.88 (s, 1H), 8.68 (s, 1H), 8.26 (d, J = 8.1 Hz, 1H), 7.85 - 7.75 (m, 2H), 7.63 - 7.53 (m, 2H), 7.39 (d, J = 5.0 Hz, 1H), 4.06 - 3.98 (m, 1H), 2.97 (t, J = 7.1 Hz, 4H), 1.65 - 1.12 (m, 10H). |
| **103** | 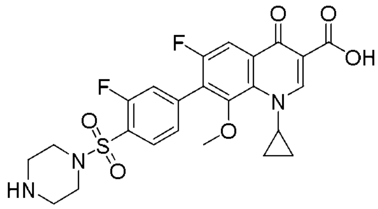 | 520.2 $[M+H]^+$ | $^1H$ NMR (400 MHz, DMSO-d6) δ 14.85 (s, 1H), 8.71 (s, 1H), 8.01 (d, J = 8.2 Hz, 1H), 7.92 (dd, J = 7.4, 5.0 Hz, 1H), 7.55 (dt, J = 7.9, 1.7 Hz, 1H), 7.36 (dt, J = 7.8, 1.8 Hz, 1H), 4.10 - 4.00 (m, 1H), 3.85 (s, 3H), 3.54 - 3.27 (m, 5H), 3.07- 3.94 (m, 4H), 1.32 - 1.01 (m, 4H). |
| **104** | 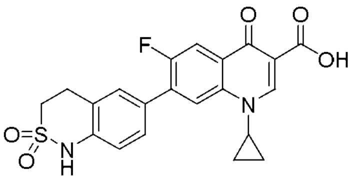 | 429.1 $[M+H]^+$ | $^1H$ NMR (400 MHz, DMSO-d6) δ 14.87 (s, 1H), 10.11 (s, 1H), 8.69 (s, 1H), 8.27 (d, J = 8.1 Hz, 1H), 7.47 - 7.36 (m, 2H), 7.33 (d, J = 5.0 Hz, 1H), 7.16 (d, J = 7.3 Hz, 1H), 4.09 - 4.01 (m, 1H), 3.42 (t, J = 7.0 Hz, 2H), 3.12 (td, J = 7.2, 1.1 Hz, 2H), 1.30 - 1.08 (m, 4H). |
| **105** | 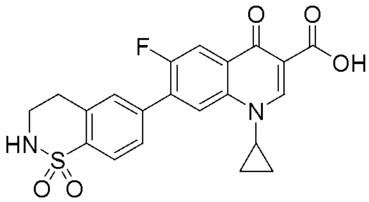 | 429.0 $[M+H]^+$ | $^1H$ NMR (400 MHz, DMSO-d6) δ 14.67 (s, 1H), 8.59 (s, 1H), 8.37 (d, J = 8.1 Hz, 1H), 7.99 (d, J = 7.6 Hz, 1H), 7.66 (dt, J = 7.6, 1.8 Hz, 1H), 7.37 (d, J = 5.0 Hz, 1H), 7.29 - 7.15 (m, 2H), 4.10 - 4.02 (m, 1H), 3.37 - 3.23 (m, 2H), 3.15 - 2.99 (m, 2H), 1.30 - 1.17 (m, 3H), 1.12 - 1.09 (m, 3H). |
| **106** | 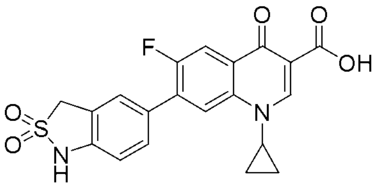 | 415.0 $[M+H]^+$ | $^1H$ NMR (400 MHz, DMSO-d6) δ 14.78 (s, 1H), 10.02 (s, 1H), 8.79 (s, 1H), 8.20 (d, J = 7.9 Hz, 1H), 7.43 (dt, J = 2.3, 1.1 Hz, 1H), 7.33 - 7.24 (m, 2H), 6.97 (d, J = 7.3 Hz, 1H), 4.48 (d, J = 1.2 Hz, 2H), 4.06 - 3.98 (m, 1H), 1.30 - 1.19 (m, 2H), 1.12 - 1.04 (m, 2H). |
| **107** | 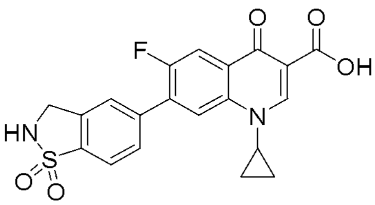 | 415.2 $[M+H]^+$ | $^1H$ NMR (400 MHz, DMSO-d6) δ 14.78 (s, 1H), 8.81 (s, 1H), 8.44 (d, J = 6.4 Hz, 1H), 8.20 (d, J = 10.2 Hz, 1H), 8.05 (d, J = 7.9 Hz, 1H), 7.91 (d, J = 9.6 Hz, 2H), 7.13 (s, 1H), 4.53 (s, 2H), 4.00 - 3.91 (m, 1H), 1.35 - 1.30 (m, 2H), 1.27 - 1.22 (m, 2H). |
| **108** | 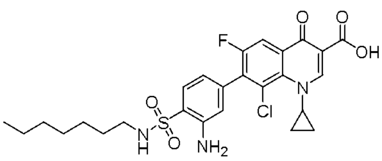 | 550.2 $[M+H]^+$ | $^1H$ NMR (400 MHz, DMSO-d6) δ 8.92 (s, 1H), 8.14 (d, J = 8.4 Hz, 1H), 7.65 (d, J = 8.2 Hz, 1H), 6.82 (s, 1H), 6.64 (d, J = 8.3 Hz, 1H), 6.51 (s, 1H), 6.11 (s, 2H), 4.45 - 4.34 (m, 1H), 2.80 (dd, J = 10.7, 5.9 Hz, 2H), 1.38 - 1.33 (m, 2H), 1.24 - 1.08 (m, 12H), 0.83 (t, J = 7.0 Hz, 3H). |
| **109** | 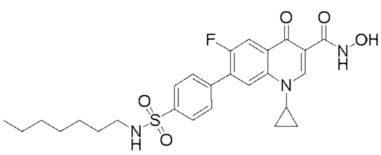 | 516.4 $[M+H]^+$ | $^1H$ NMR (400 MHz, DMSO-d6) δ 11.57 (s, 1H), 9.27 (d, J = 1.8 Hz, 1H), 8.72 (s, 1H), 8.32 (d, J = 6.4 Hz, 1H), 8.11 (d, J = 10.6 Hz, 1H), 7.95 (q, J = 8.4 Hz, 4H), 7.72 (t, J = 5.8 Hz, 1H), 3.93 - 3.82 (m, 1H), 2.81 (dd, J = 13.0, 6.7 Hz, 2H), 1.46 - 1.16 (m, 14H), 0.82 (t, J = 6.9 Hz, 3H). |

(continued)

| Example No. | Structure | LC-MS | $^1H$ NMR |
|---|---|---|---|
| **110** | 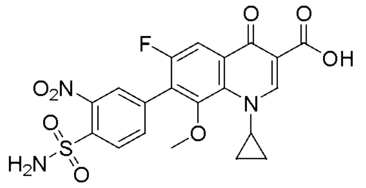 | 478.2 [M+H]+ | $^1H$ NMR (400 MHz, DMSO-d6) δ 14.57 (s, 1H), 8.83 (s, 1H), 8.25 (d, J = 8.1 Hz, 2H), 8.09 (d, J = 8.2 Hz, 1H), 8.01 (d, J = 8.9 Hz, 3H), 4.28 - 4.16 (m, 1H), 3.47 (s, 3H), 2.00 - 1.13 (m, 4H). |
| **111** | 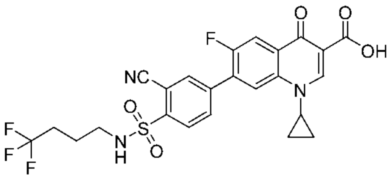 | 538.2 [M+H]+ | $^1H$ NMR (400 MHz, DMSO-d6) δ 14.80 (s, 1H), 8.66 (s, 1H), 8.31 (d, J = 8.1 Hz, 1H), 8.08 - 7.94 (m, 2H), 7.79 - 7.70 (m, 1H), 7.55 (d, J = 5.0 Hz, 1H), 7.43 (t, J = 8.9 Hz, 1H), 4.11 - 4.01 (m, 1H), 2.87 (dt, J = 8.9, 7.1 Hz, 2H), 2.41 - 2.22 (m, 2H), 1.79 - 1.63 (m, 2H), 1.30 - 1.20 (m, 4H). |
| **112** | 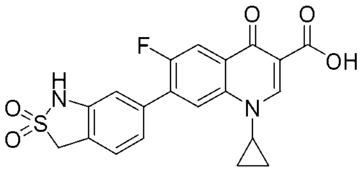 | 415.2 [M+H]+ | $^1H$ NMR (400 MHz, DMSO-d6) δ 8.65 (s, 1H), 8.12 - 8.03 (m, 2H), 7.53 (d, J = 5.0 Hz, 1H), 7.43 (t, J = 1.8 Hz, 1H), 7.33 - 7.25 (m, 1H), 7.25 - 7.16 (m, 1H), 4.44 (d, J = 0.9 Hz, 2H), 4.04 - 3.98 (m, 1H), 1.33 - 1.17 (m, 4H). |
| **113** | 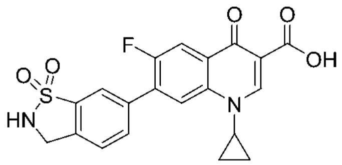 | 415.1 [M+H]+ | $^1H$ NMR (400 MHz, DMSO-d6) δ 14.79 (s, 1H), 8.80 (s, 1H), 8.44 (d, J = 6.3 Hz, 1H), 8.25 - 8.13 (m, 2H), 8.01 (dd, J = 13.5, 6.2 Hz, 2H), 7.79 (d, J = 8.0 Hz, 1H), 4.52 (d, J = 4.3 Hz, 2H), 4.05 - 3.93 (m, 1H), 1.36 - 1.21 (m, 4H). |
| **114** | 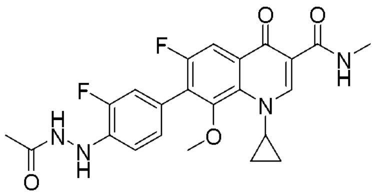 | 457.3 [M+H]+ | $^1H$ NMR (400 MHz, DMSO-d6) δ 9.65 (d, J = 6.7 Hz, 1H), 8.82 (s, 1H), 8.48 (d, J = 6.7 Hz, 1H), 8.33 (q, J = 3.5 Hz, 1H), 8.07 (d, J = 7.9 Hz, 1H), 7.32 - 7.19 (m, 3H), 4.05 - 3.90 (m, 4H), 3.02 (d, J = 3.5 Hz, 3H), 2.00 (s, 3H), 1.32 - 1.18 (m, 4H). |
| **115** | 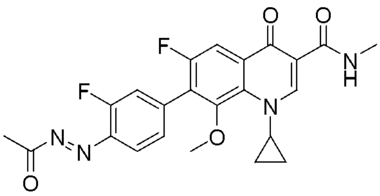 | 455.2 [M+H]+ | $^1H$ NMR (400 MHz, DMSO-d6) δ 8.72 (s, 1H), 8.23 (q, J = 3.5 Hz, 1H), 8.17 (d, J = 7.9 Hz, 1H), 7.69 (dd, J = 7.5, 4.9 Hz, 1H), 7.49 (ddt, J = 17.0, 7.8, 2.0 Hz, 2H), 4.05 - 3.92 (m, 4H), 3.02 (d, J = 3.5 Hz, 3H), 2.33 (s, 3H), 1.30 - 1.13 (m, 4H). |
| **116** | 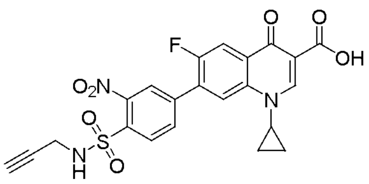 | 486.1 [M+H]+ | $^1H$ NMR (400 MHz, DMSO-d6) δ 14.77 (s, 1H), 8.63 (s, 1H), 8.22 (d, J = 7.9 Hz, 1H), 8.18 - 8.09 (m, 2H), 7.91 - 7.79 (m, 2H), 7.42 (d, J = 5.1 Hz, 1H), 4.06 - 3.91 (m, 3H), 2.46 (t, J = 2.9 Hz, 1H), 1.30 - 1.17 (m, 4H). |
| **117** | 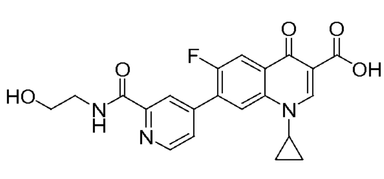 | 412.3 [M+H]+ | $^1H$ NMR (400 MHz, DMSO-d6) δ 14.80 (s, 1H), 8.98 (d, J = 7.3 Hz, 1H), 8.67 (s, 1H), 8.56 (t, J = 8.5 Hz, 1H), 8.30 - 8.22 (m, 2H), 7.76 - 7.70 (m, 1H), 7.44 (d, J = 5.0 Hz, 1H), 4.55 (t, J = 6.3 Hz, 1H), 4.09 - 4.00 (m, 1H), 3.59 - 3.46 (m, 4H), 1.34 - 1.20 (m, 4H). |

(continued)

| Example No. | Structure | LC-MS | $^1$H NMR |
|---|---|---|---|
| **118** | 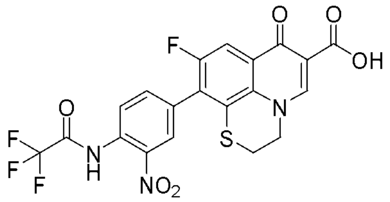 | 498.0 [M+H]$^+$ | $^1$H NMR (400 MHz, DMSO-d6) δ 14.81 (s, 1H), 11.92 (s, 1H), 8.99 (s, 1H), 8.19 (d, J = 1.6 Hz, 1H), 7.96 (d, J = 9.0 Hz, 1H), 7.91 - 7.84 (m, 2H), 4.80 - 4.71 (m, 2H), 3.45 - 3.40 (m, 2H). |
| **119** | 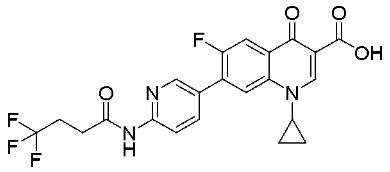 | 464.3 [M+H]$^+$ | $^1$H NMR (400 MHz, DMSO-d6) δ 14.81 (s, 1H), 10.90 (s, 1H), 8.78 (s, 1H), 8.70 (s, 1H), 8.43 (d, J = 6.4 Hz, 1H), 8.27 (d, J = 8.4 Hz, 1H), 8.22 - 8.11 (m, 2H), 4.02 - 3.82 (m, 1H), 2.76 (t, J = 7.6 Hz, 2H), 2.67 - 2.56 (m, 2H), 1.35 - 1.21 (m, 4H). |
| **120** | 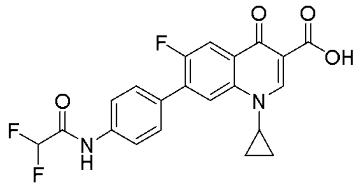 | 417.3 [M+H]$^+$ | $^1$H NMR (400 MHz, DMSO-d6) δ 14.86 (s, 1H), 11.01 (s, 1H), 8.79 (s, 1H), 8.37 (d, J = 6.3 Hz, 1H), 8.13 (d, J = 10.5 Hz, 1H), 7.89 (d, J = 8.3 Hz, 2H), 7.78 (d, J = 7.9 Hz, 2H), 6.44 (t, J = 53.6 Hz, 1H), 4.02 - 3.89 (m, 1H), 1.40 - 1.30 (m, 2H), 1.27 - 1.19 (m, 2H). |
| **121** | 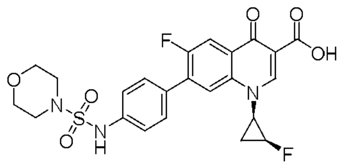 | 506.1 [M+H]$^+$ | $^1$H NMR (400 MHz, DMSO-d6) δ 14.74 (s, 1H), 10.38 (s, 1H), 8.88 (s, 1H), 8.30 (d, J = 6.4 Hz, 1H), 8.14 (d, J = 10.4, 4.6 Hz, 1H), 7.71 (d, J = 7.2 Hz, 2H), 7.40 (d, J = 8.6 Hz, 2H), 5.53 - 5.29 (m, 1H), 4.05 - 3.95 (m, 1H), 3.63 - 3.55 (m, 4H), 3.18 - 3.11 (m, 4H), 2.10 - 1.97 (m, 1H), 1.86 - 1.73 (m, 1H). |
| **122** | 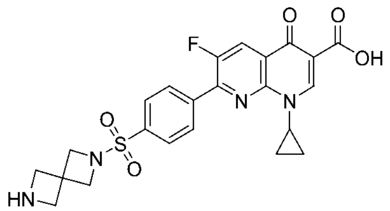 | 485.2 [M+H]$^+$ | $^1$H NMR (400 MHz, DMSO-d6) δ 9.05 (s, 1H), 8.05 - 7.74 (m, 5H), 4.05 - 3.98 (m, 1H), 3.49 - 3.18 (m, 8H), 2.24 - 2.18 (m, 1H), 1.51 - 1.16 (m, 4H). |
| **123** | 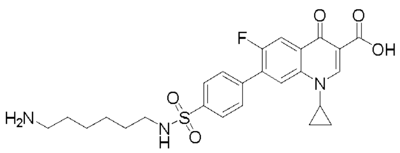 | 502.6 [M+H]$^+$ | $^1$H NMR (400 MHz, DMSO-d6) δ 8.81 (s, 1H), 8.43 (d, J = 6.3 Hz, 1H), 8.20 (d, J = 9.7 Hz, 1H), 7.97 (s, 4H), 3.98 - 3.83 (m, 1H), 2.81 (t, J = 7.0 Hz, 2H), 2.72 - 2.69 (m, 2H), 1.47 - 1.43 (m, 4H), 1.34 - 1.32 (m, 2H), 1.28 - 1.24 (m, 6H). |
| **124** | 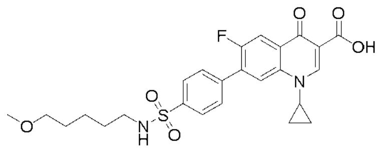 | 503.2 [M+H]$^+$ | $^1$H NMR (400 MHz, DMSO-d6) δ 14.79 (s, 1H), 8.81 (s, 1H), 8.44 (d, J = 6.4 Hz, 1H), 8.19 (d, J = 10.3 Hz, 1H), 8.00 - 7.94 (m, 4H), 7.76 (t, J = 5.8 Hz, 1H), 3.99 - 3.95 (m, 1H), 3.25 (t, J = 6.4 Hz, 2H), 3.18 (s, 3H), 2.81 (dd, J = 13.0, 6.6 Hz, 2H), 1.42 (m, 4H), 1.29 (m, 6H). |
| **125** | 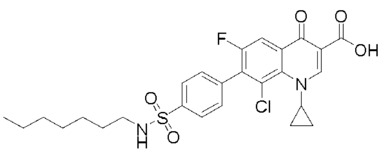 | 535.1 [M+H]$^+$ | $^1$H NMR (400 MHz, DMSO-d6) δ 14.23 (s, 1H), 8.93 (s, 1H), 8.17 (d, J = 8.5 Hz, 1H), 7.97 (d, J = 8.4 Hz, 2H), 7.75 - 7.67 (m, 3H), 4.45 - 4.35 (m, 1H), 2.84 (dd, J = 13.0, 6.7 Hz, 2H), 1.36 - 1.14 (m, 14H), 0.84 (t, J = 7.0 Hz, 3H). |

(continued)

| Example No. | Structure | LC-MS | $^1$H NMR |
|---|---|---|---|
| **126** | 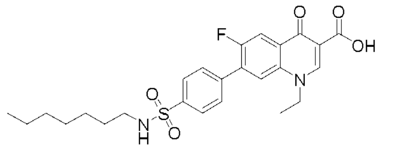 | 489.2 [M+H]$^+$ | $^1$H NMR (400 MHz, DMSO-d6) δ 14.84 (s, 1H), 8.89 (s, 1H), 8.26 (d, J = 8.1 Hz, 1H), 7.94 - 7.85 (m, 2H), 7.75 - 7.66 (m, 2H), 7.38 - 7.26 (m, 2H), 4.50 - 4.36 (m, 2H), 2.80 - 2.66 (m, 2H), 1.58 - 1.22 (m, 13H), 0.97 - 0.82 (m, 3H). |
| **127** | 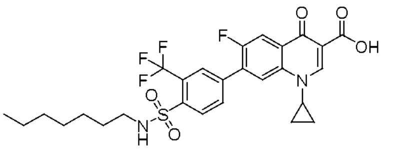 | 569.3 [M+H]$^+$ | $^1$H NMR (400 MHz, DMSO-d6) δ 14.75 (s, 1H), 8.75 (s, 1H), 8.48 (d, J = 8.0 Hz, 1H), 8.19 - 8.12 (m, 1H), 7.79 (d, J = 7.4 Hz, 1H), 7.58 - 7.39 (m, 3H), 4.12 - 4.06 (m, 1H), 2.83 - 2.68 (m, 2H), 1.56 - 1.10 (m, 14H), 0.99 - 0.87 (m, 3H). |
| **128** | 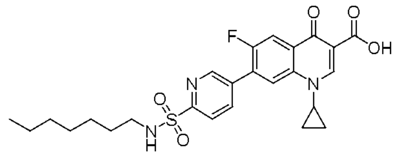 | 502.3 [M+H]$^+$ | $^1$H NMR (400 MHz, DMSO-d6) δ 14.74 (s, 1H), 9.09 (s, 1H), 8.81 (s, 1H), 8.50 (d, J = 40.6 Hz, 2H), 8.22 (d, J = 10.1 Hz, 1H), 8.13 (d, J = 7.7 Hz, 1H), 7.98 (t, J = 5.6 Hz, 1H), 3.98 - 3.94 (m, 1H), 2.98 (dd, J = 12.8, 6.5 Hz, 2H), 1.46 - 1.34 (m, 4H), 1.27 - 1.18 (m, 10H), 0.83 (t, J = 6.8 Hz, 3H). |
| **129** | 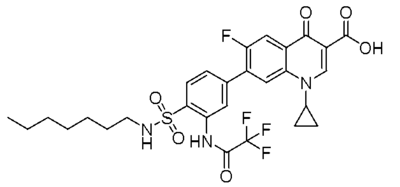 | 612.2 [M+H]$^+$ | $^1$H NMR (400 MHz, DMSO-d6) δ 14.75 (s, 1H), 10.96 (s, 1H), 8.81 (s, 1H), 8.44 (d, J = 6.0 Hz, 1H), 8.32 (s, 1H), 8.20 (d, J = 10.4 Hz, 1H), 8.09 (d, J = 8.4 Hz, 1H), 8.05 (t, J = 5.6 Hz, 1H), 7.90 (d, J = 8.4 Hz, 1H), 4.01-3.94 (m, 1H), 2.87 (dd, J = 12.8 Hz, 6.8 Hz, 2H), 1.45-1.37 (m, 2H), 1.35-1.30 (m, 2H), 1.27-1.16 (m, 10H), 0.82 (t, J = 6.8 Hz, 3H). |
| **130** | 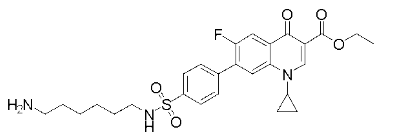 | 530.2 [M+H]$^+$ | $^1$H NMR (400 MHz, DMSO-d6) δ 8.55 (s, 1H), 8.42 (s, 1H), 8.23 (d, J = 6.5 Hz, 1H), 7.97 (dt, J = 14.7, 9.6 Hz, 5H), 4.25 (q, J = 7.0 Hz, 2H), 3.80 - 3.75 (m, 1H), 2.80 (t, J = 7.0 Hz, 2H), 2.71 - 2.63 (m, 2H), 1.48 - 1.34 (m, 4H), 1.32 - 1.22 (m, 9H), 1.16 (t, J = 8.0 Hz, 2H). |
| **131** | 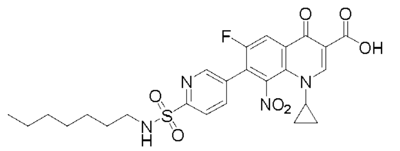 | 547.1 [M+H]$^+$ | $^1$H NMR (400 MHz, DMSO-d6) δ 13.87 (s, 1H), 8.94 - 8.77 (m, 2H), 8.51 (d, J = 8.4 Hz, 1H), 8.28 - 8.17 (m, 1H), 8.12 (d, J = 8.2 Hz, 1H), 8.03 - 7.96 (m, 1H), 3.81 - 3.72 (m, 1H), 3.09 - 2.90 (m, 2H), 1.46 - 0.77 (m, 17H). |
| **132** | 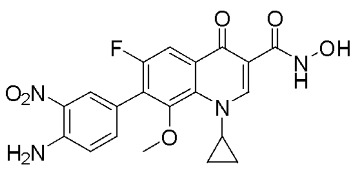 | 429.1 [M +H] $^+$ | $^1$H NMR (400 MHz, DMSO-d6) δ 11.50 (s, 1H), 9.27 (s, 1H), 8.73 (s, 1H), 8.17 (s, 1H), 7.87 (d, J = 10.0 Hz, 1H), 7.72 (s, 2H), 7.58 (d, J = 8.8 Hz, 1H), 7.18 (d, J = 8.8 Hz, 1H), 4.20-4.13 (m, 1H), 3.43 (s, 3H), 1.20-1.05 (m, 4H). |
| **133** | 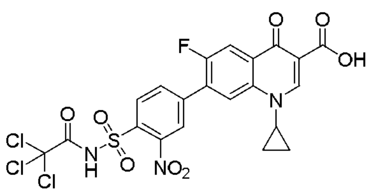 | 593.8 [M+H]$^+$ | $^1$H NMR (400 MHz, DMSO-d6) δ 14.81 (s, 1H), 10.90 (s, 1H), 8.78 (s, 1H), 8.70 (s, 1H), 8.43 (d, J = 6.5 Hz, 1H), 8.27 (d, J = 8.7 Hz, 1 H), 8.17 (dd, J = 15.4, 9.4 Hz, 2H), 4.02 - 3.82 (m, 1H), 2.76 (t, J = 7.5 Hz, 2H), 2.67 - 2.56 (m, 2H), 1.36 - 1.22 (m, 4H). |

(continued)

| Example No. | Structure | LC-MS | $^1$H NMR |
|---|---|---|---|
| **134** | 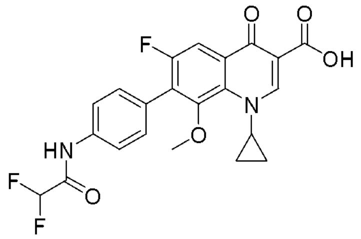 | 446.9 [M+H]$^+$ | $^1$H NMR (400 MHz, DMSO-d6) δ 14.70 (s, 1H), 10.96 (s, 1H), 8.81 (s, 1H), 7.93 (d, J = 9.2 Hz, 1H), 7.86 (d, J = 8.7 Hz, 2H), 7.57 (d, J = 7.7 Hz, 2H), 6.43 (t, J = 53.7 Hz, 1H), 4.30 - 4.17 (m, 1H), 3.39 (s, 3H), 1.22 - 1.10 (m, 4H). |
| **135** | 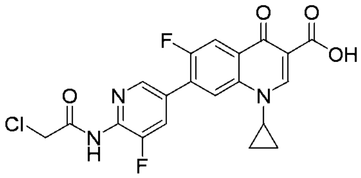 | 434.0 [M+H]$^+$ | $^1$H NMR (400 MHz, DMSO-d6) δ 14.76 (s, 1H), 10.89 (s, 1H), 8.80 (s, 1H), 8.66 (s, 1H), 8.52 (d, J = 6.4 Hz, 1H), 8.28 (d, J = 10.8 Hz, 1H), 8.19 (d, J = 10.4 Hz, 1H), 4.44 (s, 2H), 4.00 - 3.90 (m, 1H), 1.39 - 1.32 (m, 2H), 1.27 - 1.21 (m, 2H). |
| **136** | 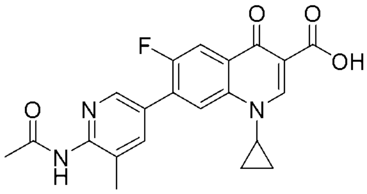 | 396.2 [M+H]$^+$ | $^1$H NMR (400 MHz, DMSO-d6) δ 14.79 (s, 1H), 10.18 (s, 1H), 8.79 (s, 1H), 8.60 (s, 1H), 8.43 (d, J = 6.0 Hz, 1H), 8.18 (t, J = 10.4 Hz, 1H), 8.04 (s, 1H), 4.01 - 3.92 (m, 1H), 2.28 (s, 3H), 2.12 (s, 3H), 1.38 - 1.31 (m, 2H), 1.26 - 1.21 (m, 2H). |
| **137** | 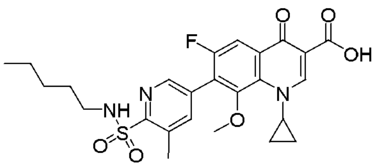 | 518.4 [M+H]$^+$ | $^1$H NMR (400 MHz, DMSO-d6) δ 14.58 (s, 1H), 8.83 (s, 1H), 8.67 (s, 1H), 8.15 (s, 1H), 8.03 (dd, J = 15.7, 7.5 Hz, 2H), 4.27 - 4.17 (m, 1H), 3.44 (s, 3H), 3.10 (dd, J = 13.1, 6.7 Hz, 2H), 2.66 (s, 3H), 1.53 - 1.43 (m, 2H), 1.29 - 1.15 (m, 8H), 0.85 (t, J = 6.5 Hz, 3H). |
| **138** | 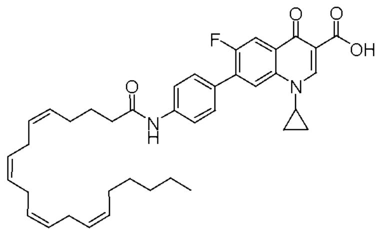 | 625.7 [M+H]$^+$ | $^1$H NMR (400 MHz, DMSO-d6) δ 14.80 (s, 1H), 9.64 (s, 1H), 8.65 (s, 1H), 8.29 (d, J = 7.9 Hz, 1H), 7.77 - 7.67 (m, 2H), 7.39 - 7.29 (m, 3H), 5.58 - 5.30 (m, 8H), 3.95 - 3.80 (m, 1H), 2.53 - 2.30 (m, 8H), 2.17 - 1.97 (m, 4H), 1.77 - 1.62 (m, 2H), 1.50 - 1.08 (m, 10H), 0.98 - 0.81 (m, 3H). |
| **139** | 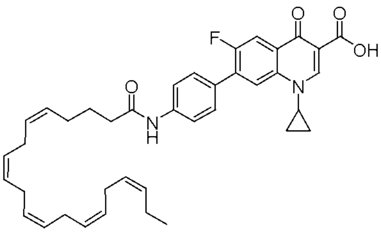 | 623.3 [M+H]$^+$ | $^1$H NMR (400 MHz, DMSO-d6) δ 14.75 (s, 1H), 9.74 (s, 1H), 8.65 (s, 1H), 8.29 (d, J = 7.9 Hz, 1H), 7.70 - 7.46 (m, 5H), 5.62 - 5.28 (m, 10H), 3.96 - 3.75 (m, 1H), 2.81 - 2.22 (m, 10H), 2.18 - 1.95 (m, 4H), 1.51 - 1.08 (m, 6H), 1.04 - 0.87 (m, 3H). |
| **140** | 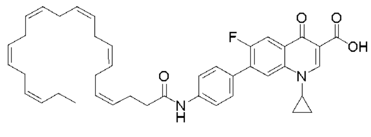 | 649.4 [M+H]$^+$ | $^1$H NMR (400 MHz, DMSO-d6) δ 14.80 (s, 1H), 9.66 (s, 1H), 8.67 (s, 1H), 8.29 (d, J = 7.9 Hz, 1H), 7.76 - 7.67 (m, 2H), 7.39 - 7.29 (m, 3H), 5.61 - 5.42 (m, 12H), 4.03 - 3.96 (m, 1H), 2.63 - 2.15 (m, 16H), 2.15 - 1.99 (m, 2H), 1.30 - 1.18 (m, 4H), 1.01 - 0.89 (m, 3H). |

(continued)

| Example No. | Structure | LC-MS | 1H NMR |
|---|---|---|---|
| **141** | 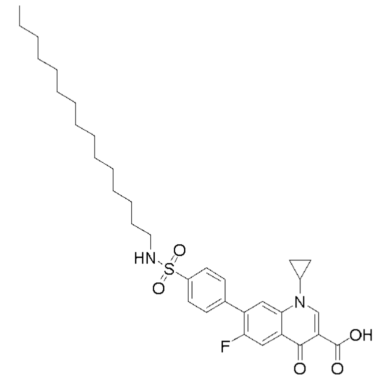 | 613.4 $[M+H]^+$ | $^1$H NMR (400 MHz, DMSO-d6) δ 14.85 (s, 1H), 8.65 (s, 1H), 8.28 (d, J = 7.9 Hz, 1H), 7.94 - 7.84 (m, 2H), 7.63 - 7.52 (m, 2H), 7.41 - 7.35 (m, 2H), 3.99 - 3.92 (m, 1H), 2.79 - 2.66 (m, 2H), 1.58 - 1.15 (m, 26H), 0.97 - 0.81 (m, 3H). |
| **142** | 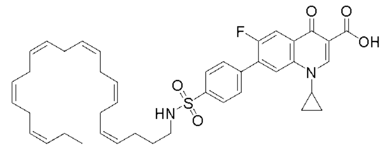 | 699.5 $[M+H]^+$ | $^1$H NMR (400 MHz, DMSO-d6) δ 14.82 (s, 1H), 8.63 (s, 1H), 8.27 (d, J = 8.1 Hz, 1H), 7.95 - 7.86 (m, 2H), 7.64 - 7.54 (m, 2H), 7.39 - 7.24 (m, 2H), 5.62 - 5.30 (m, 12H), 4.10 - 4.02 (m, 1H), 2.96 - 2.93 (m, 2H), 2.74 - 2.40 (m, 10H), 2.15 - 1.98 (m, 4H), 1.68 - 1.60 (m, 2H), 1.31 - 1.20 (m, 4H), 0.95 (t, J = 7.8 Hz, 3H). |
| **143** | 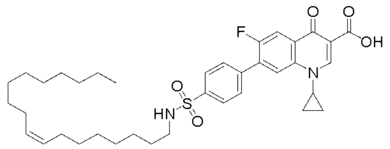 | 653.3 $[M+H]^+$ | $^1$H NMR (400 MHz, DMSO-d6) δ 14.75 (s, 1H), 8.68 (s, 1H), 8.29 (d, J = 7.9 Hz, 1H), 7.94 - 7.84 (m, 2H), 7.61 - 7.57 (m, 2H), 7.39 - 7.27 (m, 2H), 5.38 -5.33 (m, 2H), 4.11 - 4.02 (m, 1H), 2.77 - 2.70 (m, 2H), 2.05 - 1.98 (m, 4H), 1.63 - 1.56 (m, 2H), 1.38 - 1.17 (m, 26H), 0.97 - 0.83 (m, 3H). |
| **144** | 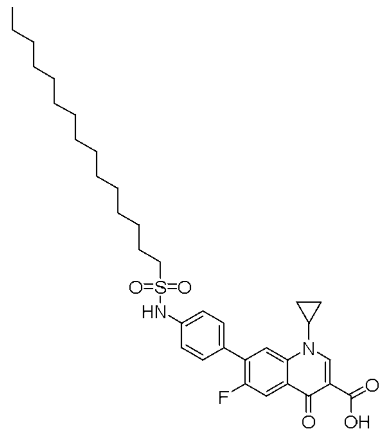 | 613.8 $[M+H]^+$ | $^1$H NMR (400 MHz, DMSO-d6) δ 14.80 (s, 1H), 9.72 (s, 1H), 8.63 (s, 1H), 8.22 (d, J = 7.9 Hz, 1H), 7.49 - 7.37 (m, 5H), 4.02 - 3.96 (m, 1H), 3.01 (t, J = 7.1 Hz, 2H), 1.78 -1.73 (m, 2H), 1.43 - 1.16 (m, 28H), 0.97 - 0.83 (m, 3H). |
| **145** | 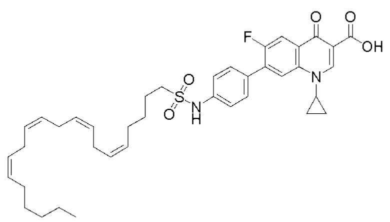 | 675.4 $[M+H]^+$ | $^1$H NMR (400 MHz, DMSO-d6) δ 14.77 (s, 1H), 9.67 (s, 1H), 8.45 (s, 1H), 8.28 (d, J = 7.9 Hz, 1H), 7.49 - 7.36 (m, 5H), 5.50 - 5.32 (m, 8H), 4.08 - 4.00 (m, 1H), 3.02 (t, J = 7.1 Hz, 2H), 2.55 - 2.38 (m, 6H), 2.06 - 1.75 (m, 6H), 1.56 - 1.17 (m, 12H), 0.98 - 0.81 (m, 3H). |
| **146** | 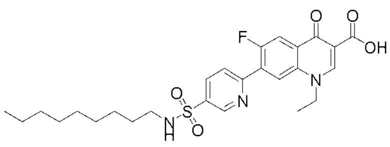 | 518.3 $[M+H]^+$ | $^1$H NMR (400 MHz, DMSO-d6) δ 14.68 (s, 1H), 8.97 - 8.89 (m, 2H), 8.35 - 8.21 (m, 2H), 7.74 (dd, J = 7.6, 2.1 Hz, 1H), 7.38 - 7.23 (m, 2H), 4.50 - 4.36 (m, 2H), 2.87 - 2.74 (m, 2H), 1.47 - 1.23 (m, 17H), 0.96 - 0.83 (m, 3H). |

(continued)

| Example No. | Structure | LC-MS | $^1$H NMR |
|---|---|---|---|
| **147** | 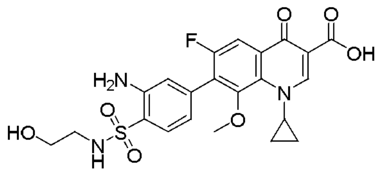 | 492.5 [M+H]$^+$ | $^1$H NMR (400 MHz, DMSO-d6) δ 14.65 (s, 1H), 8.80 (s, 1H), 7.93 (d, J = 7.9 Hz, 1H), 7.67 - 7.63 (m, 2H), 6.96 (s, 1H), 6.78 - 6.75 (m, 1H), 6.11 (s, 1H), 4.73 - 4.71 (m, 1H), 4.23 - 4.21 (m, 1H), 3.47 (s, 3H), 3.42 - 3.40 (m, 2H), 2.85 - 2.83 (m, 2H), 1.18 - 1.14 (m, 4H). |
| **148** | 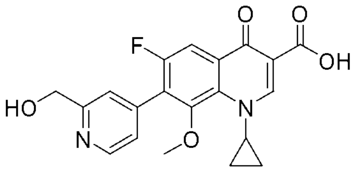 | 385.3 [M+H]$^+$ | $^1$H NMR (400 MHz, DMSO-d6) δ 14.59 (s, 1H), 8.82 (s, 1H), 8.68 (d, J = 4.8 Hz, 1H), 7.97 (d, J = 9.2 Hz, 1H), 7.62 (s, 1H), 7.45 (d, J = 4.2 Hz, 1H), 5.56 (s, 1H), 4.68 (s, 2H), 4.25 - 4.20 (m, 1H), 3.45 (s, 3H), 1.25 - 1.06 (m, 4H). |
| **149** | 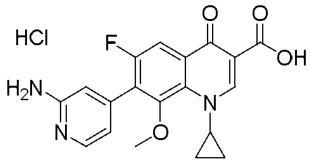 | 370.2 [M+H]$^+$ | $^1$H NMR (400 MHz, DMSO-d6) δ 14.49 (s, 1H), 14.08 (s, 1H), 8.83 (s, 1H), 8.32 - 8.10 (m, 3H), 8.00 (d, J = 9.3 Hz, 1H), 7.16 (s, 1H), 7.02 (d, J = 6.5 Hz, 1H), 4.27 - 4.17 (m, 1H), 3.59 (s, 3H), 1.18 - 1.13 (m, 4H). |
| **150** | 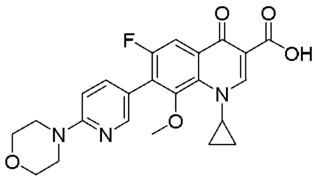 | 440.4 [M+H]$^+$ | $^1$H NMR (400 MHz, DMSO-d6) δ 8.80 (s, 1H), 8.32 (s, 1H), 7.92 (d, J = 9.3 Hz, 1H), 7.80 (d, J = 9.7 Hz, 1H), 7.05 (d, J = 9.0 Hz, 1H), 4.27 - 4.17 (m, 1H), 3.78 - 3.68 (m, 4H), 3.62 - 3.53 (m, 4H), 3.43 (s, 3H), 1.22 - 1.10 (m, 4H). |
| **151** | 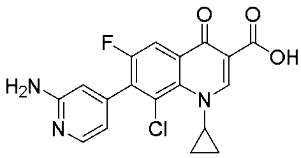 | 375.1 [M+H]$^+$ | $^1$H NMR (400 MHz, DMSO-d6) δ 14.48 (s, 1H), 8.86 (s, 1H), 8.12 - 8.04 (m, 2H), 7.27 - 7.19 (m, 1H), 6.82 - 6.72 (m, 3H), 4.25 - 4.18 (m, 1H), 1.30 - 1.10 (m, 4H). |

**In vitro antibacterial activity assay**

The method used to indicate the activity of the compounds in this example is to record the minimum inhibitory concentration (MIC) of the compounds against bacteria. The specific experimental method is as follows:

1. The bacterial liquid cultured from a single colony was taken, and diluted into autoclaved CAMHB broth in a ratio of 1:1000, and mixed well.
2. The prepared drug solution was filtered through a sterile 0.22μm syringe filter and placed in a sterile EP tube for later use.
3. A 96-well plate was taken, and 20μL of drug solution (400μg/mL) was added to each well in the first column of the 96-well plate. Then, 180μL of diluted bacterial liquid was added and mixed well (Perform 3 replicate rows for each drug). The same process was performed for the hydrochloride drug.
4. 100 μL of diluted bacterial liquid was added to each well in the remaining 11 columns of the 96-well plate. Drugs are the same as above.
5. Serial dilution: 100μL of liquid was taken from the first column to the second column, mixed well, then 100μL of liquid was taken from the second column to the third column, mixed well, and so on, sequentially diluting down to the last column (column 12). After mixing column 12 well, 100μL of liquid thereof was discarded. The drug concentration in each well was decreased by two-fold serially (the concentration in the first well is 40μg/mL).
6. Blank CAMHB broth without added bacteria liquid was added to 3 blank wells in the 96-well plate, as negative control.
7. The diluted bacterial liquid was added to 3 blank wells in the 96-well plate, as positive control.
8. Incubated at 37°C for 16 hours.

9. Results reading: the minimum inhibitory concentration is the minimum drug concentration at which no bacterial growth is visible to the naked eye and without turbidity.

The specific antibacterial results are shown in Tables 1, 2 and 3.

Table 1

| Compounds | G+ bacteria MIC (μg/mL) | |
|---|---|---|
| | *S. aureus* ATCC 43300 | *E. feacium* ATCC 700221 |
| **Ciprofloxacin** | 0.1-1 | >8 |
| **Moxifloxacin** | ≤0.1 | >8 |
| **Example 8** | ≤0.1 | 1-8 |
| **Example 9** | ≤0.1 | 1-8 |
| **Example 15** | ≤0.1 | >8 |
| **Example 18** | ≤0.1 | 1-8 |
| **Example 20** | ≤0.1 | >8 |
| **Example 34** | ≤0.1 | 1-8 |
| **Example 37** | ≤0.1 | 1-8 |
| **Example 54** | ≤0.1 | 1-8 |

Table 2

| Compounds | G- bacteria MIC (μg/mL) | | |
|---|---|---|---|
| | *E. coli* ATCC 25922 | *A. baumannii* ATCC 17978 | *Paeruginosa* ATCC 27853 |
| **Ciprofloxacin** | 0.1-1 | 0.1-1 | 0.1-1 |
| **Moxifloxacin** | ≤0.1 | ≤0.1 | 1-8 |
| **Example 8** | ≤0.1 | 0.1-1 | 0.1-1 |
| **Example 9** | ≤0.1 | ≤0.1 | 0.1-1 |
| **Example 18** | ≤0.1 | 0.1-1 | 0.1-1 |
| **Example 34** | ≤0.1 | ≤0.1 | ≤0.1 |
| **Example 35** | ≤0.1 | 0.1-1 | 0.1-1 |
| **Example 40** | ≤0.1 | ≤0.1 | 0.1-1 |
| **Example 48** | ≤0.1 | 0.1-1 | 0.1-1 |
| **Example 55** | ≤0.1 | ≤0.1 | 0.1-1 |

Table 3

| Compounds | MIC (μg/mL) | |
|---|---|---|
| | *S. aureus* ATCC 43300 | *E. coli* ATCC 25922 |
| **Example 56** | 0.1-1 | 0.1-1 |
| **Example 57** | 0.1-1 | 0.1-1 |
| **Example 64** | 0.1-1 | 0.1-1 |
| **Example 66** | 0.1-1 | 0.1-1 |
| **Example 74** | 0.1-1 | 0.1-1 |
| **Example 77** | 0.1-1 | 0.1-1 |
| **Example 80** | ≤0.1 | 0.1-1 |

(continued)

| Compounds | MIC (μg/mL) | |
|---|---|---|
| | *S. aureus* ATCC 43300 | *E. coli* ATCC 25922 |
| **Example 83** | 0.1-1 | 0.1-1 |
| **Example 86** | ≤0.1 | 0.1-1 |
| **Example 89** | ≤0.1 | 0.1-1 |
| **Example 91** | 0.1-1 | 0.1-1 |
| **Example 93** | 0.1-1 | 0.1-1 |
| **Example 96** | ≤0.1 | ≤0.1 |
| **Example 107** | ≤0.1 | 0.1-1 |
| **Example 110** | ≤0.1 | ≤0.1 |
| **Example 111** | 0.1-1 | 0.1-1 |
| **Example 113** | ≤0.1 | ≤0.1 |
| **Example 116** | 0.1-1 | 0.1-1 |
| **Example 128** | ≤0.1 | 1-8 |
| **Example 135** | ≤0.1 | ≤0.1 |
| **Example 147** | ≤0.1 | 0.1-1 |
| **Example 148** | 1-8 | ≤0.1 |
| **Example 151** | ≤0.1 | ≤0.1 |

In addition, as further described herein, the methods and antibacterial compounds disclosed herein can be used to inhibit the growth of multiple types of bacteria. For example, in some examples, the bacteria include but are not limited to Gram-positive bacteria, such as *Staphylococcus aureus, Enterococcus faecalis,* and Gram-negative bacteria, such as *Escherichia coli, Acinetobacter baumannii, Pseudomonas aeruginosa,* and combinations thereof.

The antibacterial compounds and methods disclosed in the present invention may possess various advantageous properties and applications. For example, in some embodiments, the antibacterial compounds disclosed in the present invention have excellent activity against Gram-positive bacteria. In some embodiments, the antibacterial compounds disclosed in the present invention have inhibitory activity against both of Gram-positive and Gram-negative bacteria. In some embodiments, the antibacterial compounds disclosed in the present invention have anticancer and antibacterial activity. In some embodiments, the antibacterial compounds disclosed in the present invention are difficult to incur drug resistance.

**Resistance test of bacteria against compounds**

This example provides a resistance test of bacteria against compounds, and the specific experimental method is as follows.

1. Drug preparation: methicillin-resistant *Staphylococcus aureus* MRSA strain with the MIC of 0.5μg/mL was taken as an example, 6 concentration gradients of tylosin of 20μg/mL, 10μg/mL, 8μg/mL, 4μg/mL, 2μg/mL, and 1μg/mL were prepared in sequence. It was ensured that among the 6 final concentrations of the drug diluted into the wells, some were higher than the original MIC of the bacteria and some were lower than the original MIC of the bacteria.
2. Preparation of bacterial liquid: 10 mL of culture medium was taken and added to a sterilized plate using a pipette, then 100 μL of the above-mentioned passaged bacterial liquid was added, and mixed well for later use.
3. The above-mentioned diluted bacterial liquid was taken and added to a 96-well plate using a pipette, 180 μL per well, 3 wells per group, 6 groups in total. 20 μL of drug solution was added to each well (note: it is necessary to replace the pipette tip each time the drug is added), so that the final drug concentrations were 2 μg/mL, 1 μg/mL, 0.8 μg/mL, 0.4 μg/mL, 0.2 μg/mL, 0.1 μg/mL, respectively. The well with 200 μL of diluted bacterial liquid was used as the positive control well, and the well with 200 μL of culture medium was used as the negative control well. The periphery of 96-well plate was fixed with medical tape, and placed in a shaker and cultured at 37°C for 24 hours, and the lowest drug concentration well without bacterial growth was an induced strain MIC well.

4. Each time the MIC of the induced strain changes, 50 μL of the bacterial liquid in the well was taken and added to 5 mL of culture medium, and then tylosin was added to make the final drug concentration reach MIC, followed by subcultured and the strain was preserved in time with 50% sterilized glycerol. In addition, the drug concentrations of the six groups were appropriately increased according to the change of the MIC of the induced strain, and the bacterial liquid in the well was taken and the above steps (2) and (3) were repeated for the next induction, until the MIC reaches 128 μg/mL.

In this experiment, drug resistance tests were conducted on Example 34, wherein the positive control was selected from the marketed drugs ciprofloxacin (CIP) and vancomycin (VAN). The test object was *Staphylococcus aureus (S. aureus)*. The specific results are shown in FIG1.

As can be seen from the figure, for *Staphylococcus aureus* ATCC43300, Example 34 was superior to the marketed drugs ciprofloxacin and vancomycin in terms of the initial MIC. In the subsequent culturalization, the resistance of bacteria against ciprofloxacin increased rapidly. In the experiment with a period of 21 days, it can be seen that at the end of the experiment, the MIC of ciprofloxacin has reached 128 μg/mL; the MIC of vancomycin has reached 4 μg/mL; and the MIC of Example 34 is only 2 μg/mL.

**In vitro anticancer activity test**

The anticancer activity of the compounds was tested using an *in vitro* cancer cell tests. The specific test method is as follows:

1. Cell recovery and culture method: the cryovial was taken out from the liquid nitrogen container firstly, soaked directly in 37°C warm water, and shaked from time to time to let it thaw as soon as possible. Then, the cryovial was transferred to a clean bench, and the lid was open. The cell suspension was taken into a centrifuge tube using a rubber-tipped dropper and more than 10 times the culture medium was added, mixed well, and centrifuged at 50g for 5 minutes. Then, the supernatant was discarded, complete culture medium containing 10% fetal bovine serum was added to resuspend the cells, the cells was counted, the cell density was adjusted, and then inoculated into a culture bottle. The culture bottle was placed in a cell culture incubator at 5% $CO_2$ and 37°C. The complete culture medium was replaced once the next day and the culture was continued.
2. Cell passaging method: adherent cells in the logarithmic growth phase were placed in a clean bench, the original culture medium was discarded, and washed three times with 1×PBS. An appropriate amount of 0.25% trypsin digestion solution was added to moisten the cells, and then the trypsin solution was aspirated. The cells were placed in a cell culture incubator at 5% $CO_2$ and 37°C for digestion for a suitable time, and observed under a microscope. When 90% of the cells become round, the cells were moved immediately to the clean bench and complete culture medium was added to terminate digestion. Then, the cells were dislodged by pipetting up and down with a rubber-tipped dropper to single cells. The cells were counted, and the cell density was adjusted. The cells were inoculated into a suitable culture bottle or dish, added enough complete culture medium, and placed in a cell culture incubator at 5% $CO_2$ and 37°C for static culture.
For suspension cells in the logarithmic phase, the original culture medium was directly centrifuged at 50g for 5 minutes, the supernatant was discarded, and the cells were washed once with PBS and centrifuged. After discarding the supernatant, the complete culture medium was added and the cells were dispersed using a rubber-tipped dropper. The cells are counted, and the cell density was adjusted. The cells were inoculated into appropriate culture bottles or culture dishes, and sufficient complete culture medium was added, and the cells were cultured in a cell culture incubator at 5% $CO_2$ and 37°C.
3. Cell proliferation assay: cells in good state in logarithmic phase were taken and appropriate amount of the cells was inoculated into 96-well plates according to the cell passage treatment method, with each well containing 180 μL complete medium. After the cells growth was recovered and the number of cells in each well is 5000±500, a drug was added for treatment.
After drug treatment during the experimental time, MTT solution with a final concentration of 0.5 mg/mL was added to each well, and placed in a cell culture incubator at 5% $CO_2$ and 37°C to allow a fully reaction of MTT with cells. After 4 hours, the solution in the 96-well plate was discarded, 100 μL of DMSO was added, and shaked to dissolve the formazan precipitated at the bottom of the plate, and the absorbance value of samples was detected at a wavelength of 570 nm using a microplate reader. Calculation method of cell viability rate:

$$\text{Viability rate} = \text{A sample group} \div \text{A control group} \times 100\%$$

4. Cell photography step: cells in good state in logarithmic phase were taken and an appropriate amount of cells was

inoculated into 96-well plates according to the cell passage treatment method, with each well containing 180 μL complete medium. After the cells growth was recovered and the number of cells in each well is 5000 ± 500, a drug was added for treatment.

After drug treatment during the experimental time, the cells were photographed under a biological microscope.
Hela cell is an artificially cultured cell with unlimited proliferation ability. They have existed for 68 years since their creation until 2019. In the medical field, Hela cell is widely used in tumor research, biological experiments or cell culture, and has become a very important tool in medical research.
Table 4 lists some compounds that have inhibitory effects against both of bacteria and cancer cells. In the table, the inhibitory effects against cancer cells are shown: the average proliferation inhibition rate (%) against cancer cells at the dosage of 40 μM.

Table 4

| **Example** | *E. coli* ATCC 25922 MIC (μg/mL) | *S. aureus* ATCC 43300 MIC (μg/mL) | Hela Average inhibition rate% |
|---|---|---|---|
| **Ciprofloxacin** | ≤1 | ≤1 | ≤10 |
| **17** | ≥1 | ≤1 | ≥50 |
| **24** | ≥1 | ≤1 | ≥50 |
| **34** | ≤1 | ≤1 | ≥50 |
| **45** | ≥1 | ≤1 | ≥50 |
| **48** | ≤1 | ≤1 | ≥20 |
| **68** | ≥1 | ≤1 | ≥20 |
| **69** | ≥1 | ≤1 | ≥50 |
| **80** | ≤1 | ≤1 | ≥50 |
| **86** | ≤1 | ≤1 | ≥20 |
| **89** | ≤1 | ≤1 | ≥20 |
| **93** | ≤1 | ≤1 | ≥20 |
| **101** | ≥1 | ≤1 | ≥50 |
| **107** | ≤1 | ≤1 | ≥20 |
| **113** | ≤1 | ≤1 | ≥20 |
| **127** | ≥1 | ≤1 | ≥20 |
| **128** | ≥1 | ≤1 | ≥20 |
| **132** | ≥1 | ≤1 | ≥50 |
| **135** | ≤1 | ≤1 | ≥50 |
| **148** | ≤1 | ≥1 | ≥20 |
| **151** | ≤1 | ≤1 | ≥20 |

In addition, the inhibitory effects of relevant molecules against other different types of cancer cells were also tested, and all of the molecules showed strong activity. The cells also include: human Burkitt's lymphoma cell Raji, human acute T-lymphoblastic leukemia cell Jurkat, human acute myeloid leukemia cell MOLM-13, human acute monocytic leukemia cell THP-1, human non-small cell lung cancer cell A549, human gastric cancer cell MGC-803, human renal cancer cell OS-RC-2, human colon cancer cell HCT 116, pancreatic cancer cell PNAC-1, human liver cancer cell HepG2, human liver cancer cell BLE-7402, human malignant melanoma cell A375, human epidermal carcinoma cell A431, human prostate cancer cell PC-3, human bladder transitional cell carcinoma cell T24, human ovarian adenocarcinoma cellSK-OV-3, human glioma cell SNB-19, human breast cancer cell MCF-7, human osteosarcoma cell U2-OS, human breast cancer cell MFM223, human breast ductal carcinoma cell T-47D. $IC_{50}$ is a concentration exhibiting 50% inhibition. The lower the concentration exhibiting 50% inhibition, the more sensitive the inhibitor. The present experiment conducted an *in*

*vitro* antitumor cell tests on Example 34, and the positive reference was the marketed drug sunitinib.

Table 5 showed the $IC_{50}$ assay results of Example 34, Example 135, sunitinib, ciprofloxacin, reference compound 1 and reference compound 2 against different cancer cell lines.

The present invention provides a phenylquinolone compound, which has antibacterial activity and also exhibits excellent anticancer activity. Herein, reference compound 1 disclosed in patent CN103764631A in the name of Otsuka Company and reference compound 2 disclosed in patent CN109422726A are used as references.

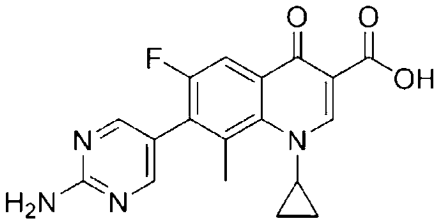

**reference compound 1**

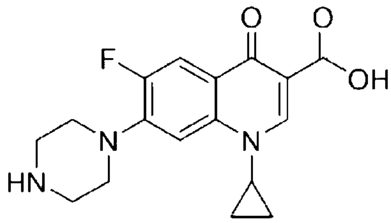

**reference compound 2**

Table 5 $IC_{50}$ values (μM) of Example 34 and Example 135 against different cancer cells

| $IC_{50}$/μM | Example **34** | Example **135** | Sunitinib | Reference compound 1 | Reference compound 2 (Ciprofloxacin) |
|---|---|---|---|---|---|
| Hela | 1-10 | 1-10 | 12±2.0 | ND | >100 |
| Raji | 1-10 | 1-10 | 20.3±2.0 | ≥40 | >100 |
| Jurkat | 1-10 | 1-10 | 8.5±1.2 | ND | >100 |
| MOLM-13 | 1-10 | 1-10 | ND | ND | >100 |
| THP-1 | 1-10 | 1-10 | ND | ND | >100 |
| A549 | 1-10 | 1-10 | 12.9 ±10.2 | ND | >100 |
| MGC-803 | 1-10 | 1-10 | 17.5±2.4 | ND | >100 |
| OS-RC-2 | 1-10 | 1-10 | ND | ND | >100 |
| HCT 116 | 1-10 | 1-10 | 20.7±4.6 | ND | >100 |
| PNAC-1 | 1-10 | 1-10 | 22.4±5.2 | ND | >100 |
| HepG2 | 1-10 | 1-10 | 9.6±2.1 | ND | >100 |
| BLE-7402 | 1-10 | 1-10 | ND | ND | >100 |
| A375 | 1-10 | 1-10 | 19.2+4.6 | ND | >100 |
| A431 | 1-10 | 1-10 | ND | ND | >100 |
| PC-3 | 1-10 | 1-10 | 14.7±6.6 | ND | >100 |
| T24 | 1-10 | 1-10 | ND | ND | >100 |
| SK-OV-3 | 1-10 | 1-10 | 12.2±5.8 | ND | >100 |
| SNB-19 | 1-10 | 1-10 | 6.5±1.2 | ND | >100 |
| MCF-7 | 1-10 | 1-10 | 3.2±0.3 | ND | >100 |
| U2-OS | 1-10 | 1-10 | 4.7±1.1 | ND | >100 |
| MFM223 | 1-10 | 1-10 | 10.2±3.8 | ND | >100 |
| T-47D | 1-10 | 1-10 | 6.4±1.7 | ND | >100 |

**Tests of antibacterial effects and drug resistance effects in animal model**

Experiment procedure:

Kunming mice (20±2 g) were anesthetized with chloral hydrate and shaved, and then 50 μL of 4×$10^7$ CFU/mL of methicillin-resistant *Staphylococcus aureus* was subcutaneously injected. One day later, 100 μL of 0.02, 2 and 200 mg/kg of Example 34 were subcutaneously injected respectively, and 100 μL of normal saline was injected for the control group. The drug was administered for consecutive 4 days, and mice were euthanized on the 5th day, and the area of abscess was measured.

Experimental results:

Large-scale necrosis of skin tissue was showed in mice of the control group (Figure 2). Compared with the control group, 0.02 and 2 mg/kg of Example 29 reduced the necrosis area by 78.09% and 81.21% respectively (Figure 2). When the high concentration of 200 mg/kg was administrated, certain bleeding symptom in body surface appeared at the subcutaneous administration site.

When the concentrations of Example 34 were 4 μg/mL and 400 μg/mL (corresponding to 0.02 mg/kg, 200 mg/kg), Example 29 exhibited good antibacterial effect.

**Tests of systemic blood infection animal model**

Experiment procedure:

(1) Specific pathogen-free mice were divided into six groups, with 5 mice in each group, namely, blank group, model group, low-dose treatment group (0.25 mg/kg), medium-dose treatment group (1 mg/kg), high-dose treatment group (4 mg/kg) and positive drug group (oral gavage administration of ciprofloxacin hydrochloride, 4 mg/kg).
(2) Sterile phosphate buffered saline (PBS) was used to prepare a 1×$10^7$ CFU/mL *Staphylococcus aureus* ATCC43300 bacterial suspension for later use; the drug dosage required for mice was calculated for each group, and solutions of Example 34 and solvent for gavage (400 or 200 μL of Tween 80 was mixed with Example 34, grinded with an agate mortar until it became transparent, and then 0.5% sodium carboxymethyl cellulose solution was added to reach the required concentration and volume of the test drug) were prepared for later use.
(3) Except for the blank group, 100 μL of the bacterial suspension prepared in step 2 was injected into the tail vein of each mouse.
(4) 2 hours after the challenge, the drugs prepared in step 2 were administered by oral gavage according to the groups, 3 times a day, 200uL each time, with an interval of 8 hours, for a total of 7 days. Mice were administered by oral gavage with the drug solvent for the model group and the blank group.
(5) 8 h after the last administration, mice were euthanized by cervical dislocation, disinfected with 75% alcohol, and then necropsied under sterile conditions. The kidneys and livers were harvested, observed and photographed, and the weight was recorded.
(6) The kidneys and livers were placed in a sterile glass grinder and sterile PBS in a volume ten times the weight of the organs was added and grinded. The grinding solutions were taken out, 10-fold serially diluted, smeared to a TSA solid culture medium, and incubated at 37°C for 16 hours, then the colonies were counted. The number of colonies of the organs was calculated to evaluate the therapeutic effect.

Experimental results:

(1) Analysis of weight changes of relevant organs
After dissecting the mice, it was found that the weights of the livers and kidneys of the mice in each group of the experiment had no significant changes, comparing with the negative control group (Figure 3).
(2) The number of colonies of organs

After analysis, it was found that Example 34 of the present invention exhibited a very significant antibacterial effect in the liver and kidney compared with the positive drug ciprofloxacin. At 1 mg/kg, there was a significant statistical difference from the control group. At 4 mg/kg, the drug of the present invention showed a huge difference, showing a good antibacterial effect, while the effects of the negative control and positive control ciprofloxacin were not apparent (Figure 4).

**Claims**

**1.** A phenylquinolone compound represented by formula (I) or a pharmaceutically acceptable salt, stereoisomer,

deuteride, or prodrug thereof,

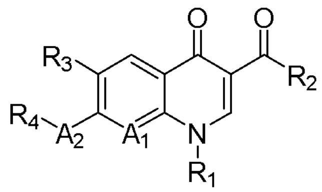

(I)

wherein:

$A_1$ is nitrogen atom N or $CR_a$; in which $R_a$ is hydrogen atom, halogen atom, C1-C3 alkyl, C1-C2 alkoxy, amino, nitro, or $R_1$ and $R_a$ are bonded to form a 6-membered heterocycle which is optionally substituted with short chain alkyl or unsubstituted;
$A_2$ is absent, then $R_4$-$A_2$- is $R_4$-;
$R_1$ is hydrogen atom, cyano, C1-C2 short chain alkyl substituted with 0 or 2 halogen atom(s), C3-C6 cycloalkyl substituted with 0 or 1 halogen atom, phenyl substituted with 1 or 2 halogen atom(s), or 2-pyridinyl substituted with 1 to 3 substituent(s) selected from: halogen or amino; or $R_1$ and $R_a$ are bonded to form a 6-membered heterocycle which is optionally substituted with short chain alkyl or unsubstituted;
$R_2$ is hydroxy, C1-C2 alkoxy, or -$NR_bR_c$; in which $R_b$, $R_c$ are each independently selected from hydrogen atom, C1-C2 alkyl or hydroxy, and are not both hydroxy;
$R_3$ is halogen, nitro or C1-C2 alkoxy;
$R_4$ is selected from: substituted aryl; the substitution is mono- to tetra-substitution, including combinations of one or more of ortho-, meta- and para-substitutions; the substituent is selected from: halogen, nitro -$NO_2$, cyano -CN, hydroxylaminyl -NH-OH, C1-C3 alkoxy substituted with 0 to 3 halogen atom(s), C1-C9 alkyl substituted with 0 to 3 halogen atom(s), sulfinyl -SO-$R_d$, aminylsulfinyl -SO-$NR_eR_e'$, sulfonyl -$SO_2$-$R_f$, aminylsulfonyl -$SO_2$-$NR_gR_g'$, methyliminosulfinyl -S(=O)(=$NR_h$)$CH_3$, sulfenyl -S-$R_i$, aminyl -$NR_jR_j'$, carbamoyl -CO-$NR_kR_k'$, hydrazinyl -NH-$NR_lR_l'$, diazenyl - N=$NR_mR_m'$; wherein, at least one substituent is selected from sulfinyl -SO-$R_d$, aminylsulfinyl -SO-$NR_eR_e'$, sulfonyl -$SO_2$-$R_f$, aminylsulfonyl -$SO_2$-$NR_gR_g'$, methyliminosulfinyl -S(=O)(=$NR_h$)$CH_3$, aminyl -$NR_jR_j'$, hydrazinyl -NH-$NR_lR_l'$, diazenyl -N=$NR_mR_m'$;
wherein, $R_d$ is selected from: C1-C9 linear or branched alkyl, C3-C6 cycloalkyl, halo C1-C9 alkyl, C1-C9 alkyl substituted with hydroxy, C1-C9 alkyl substituted with amino, C1-C9 alkyl substituted with -$SR_d''$, C2-C9 alkenyl, C2-C9 alkynyl, morpholine, piperazine, piperidine, $OR_d'$; $R_d'$, $R_d''$ are each independently selected from: hydrogen atom, C1-C9 linear or branched alkyl, C3-C6 cycloalkyl, or halo C1-C9 alkyl; C atom in the carbon chain of alkyl may be replaced by heteroatom at any position as normal valence permits;
$R_e$, $R_e'$ are each independently selected from: hydrogen atom, C1-C9 linear or branched alkyl, C3-C6 cycloalkyl, halo C1-C9 alkyl, C1-C9 alkyl substituted with hydroxy, C1-C9 alkyl substituted with amino, C1-C9 alkyl substituted with -$SR_e''$, C2-C9 alkenyl, C2-C9 alkynyl, C1-C5 acyl (H or C1-C4 alkyl)-C(=O)-, halo C2-C5 acyl or C2-C5 alkoxycarbonyl; $R_e''$ is hydrogen atom, C1-C9 linear or branched alkyl, C3-C6 cycloalkyl, or halo C1-C9 alkyl; C atom in the carbon chain of alkyl may be replaced by heteroatom at any position as normal valence permits;
$R_f$ is selected from: C1-C9 linear or branched alkyl, C3-C6 cycloalkyl, halo C1-C9 alkyl, C1-C9 alkyl substituted with hydroxy, C1-C9 alkyl substituted with amino, C1-C9 alkyl substituted with - $SR_f''$, morpholine, piperazine, piperidine, $OR_f'$; $R_f'$, $R_f''$ are each independently selected from: hydrogen atom, C1-C9 linear or branched alkyl, C3-C6 cycloalkyl, or halo C1-C9 alkyl; C atom in the carbon chain of alkyl may be replaced by heteroatom at any position as normal valence permits;
$R_g$, $R_g'$ are each independently selected from: hydrogen atom, C1-C25 linear or branched alkyl containing 0-10 unsaturated bond(s), C3-C6 cycloalkyl, halo C1-C9 alkyl, C1-C9 alkyl substituted with hydroxy, C1-C9 alkyl substituted with amino, C1-C9 alkyl substituted with -$SR_g''$, C2-C25 alkenyl, C2-C9 alkynyl, C1-C5 acyl (H or C1-C4 alkyl)-C(=O)-, halo C2-C5 acyl or C2-C5 alkoxycarbonyl; $R_g''$ is hydrogen atom, C1-C9 linear or branched alkyl; C atom in the carbon chain of alkyl may be replaced by heteroatom at any position as normal valence permits; or-$NR_gR_g'$ is

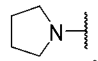,

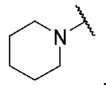, 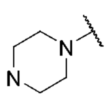, 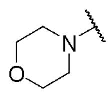, 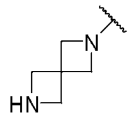;

$R_h$ is selected from: hydrogen atom, C1-C9 linear or branched alkyl containing 0-10 unsaturated bond(s), C3-C6 cycloalkyl, halo C1-C9 alkyl, C2-C9 alkenyl, C2-C9 alkynyl, C1-C5 acyl (H or C1-C4 alkyl)-C(=O)-, halo C2-C5 acyl or C2-C5 alkoxycarbonyl; C atom in the carbon chain of alkyl may be replaced by heteroatom at any position as normal valence permits;

$R_i$ is selected from: hydrogen atom, C1-C9 linear or branched alkyl, C3-C6 cycloalkyl, halo C1-C9 alkyl, C1-C9 alkyl substituted with hydroxy, C1-C5 acyl, halo C2-C5 acyl, $-NR_i'R_i''$; $R_i'$, $R_i''$ are each independently selected from hydrogen atom, C1-C9 linear or branched alkyl, C3-C6 cycloalkyl, halo C1-C9 alkyl, C1-C5 acyl (H or C1-C4 alkyl)-C(=O)-, halo C2-C5 acyl or C2-C5 alkoxycarbonyl; C atom in the carbon chain of alkyl may be replaced by heteroatom at any position as normal valence permits;

Rj is hydrogen atom, $R_j'$ is halo C2-C5 acyl or C1-C25 alkylsulfonyl;

$R_k$, $R_k'$ are each independently selected from: hydrogen atom, C1-C9 linear or branched alkyl, C3-C6 cycloalkyl, halo C1-C9 alkyl, C1-C9 alkyl substituted with hydroxy, C2-C9 alkenyl, C2-C9 alkynyl, hydroxy, C1-C5 acyl (H or C1-C4 alkyl)-C(=O)-, halo C2-C5 acyl, C2-C5 alkoxycarbonyl or C1-C9 alkylsulfonyl; C atom in the carbon chain of alkyl may be replaced by heteroatom at any position as normal valence permits;

$R_l$, $R_l'$ are each independently selected from: hydrogen atom, C1-C9 linear or branched alkyl, C3-C6 cycloalkyl, halo C1-C9 alkyl, C2-C9 alkenyl, C2-C9 alkynyl, hydroxy, C1-C5 acyl (H or C1-C4 alkyl)-C(=O)-, halo C2-C5 acyl, or C2-C5 alkoxycarbonyl; C atom in the carbon chain of alkyl may be replaced by heteroatom at any position as normal valence permits;

$R_m$, $R_m'$ are each independently selected from: hydrogen atom, phenyl, C1-C9 linear or branched alkyl, C3-C6 cycloalkyl, halo C1-C9 alkyl, C2-C9 alkenyl, C2-C9 alkynyl, hydroxy, C1-C5 acyl (H or C1-C4 alkyl)-C(=O)-, halo C2-C5 acyl, or C2-C5 alkoxycarbonyl; C atom in the carbon chain of alkyl may be replaced by heteroatom at any position as normal valence permits;

the aryl is selected from: phenyl, 2-pyridinyl, 3-pyridinyl, 4-pyridinyl,

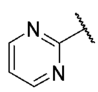, 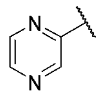,

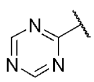, 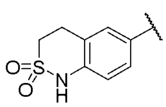, 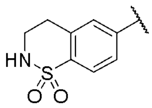, 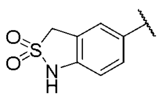, 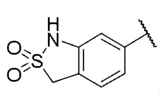, 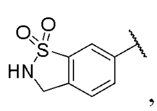, 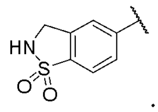.

**2.** The phenylquinolone compound according to claim 1, or a pharmaceutically acceptable salt, stereoisomer, deuteride, or prodrug thereof, **characterized in that**, the C3-C6 cycloalkyl in $R_4$ is C4-C5 heterocycloalkyl; and particularly selected from:

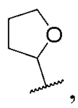, 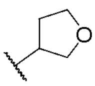, 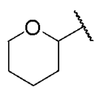, 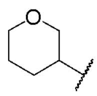, 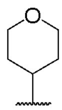.

**3.** The phenylquinolone compound according to claim 1, or a pharmaceutically acceptable salt, stereoisomer, deuteride, or prodrug thereof, **characterized in that**, the phenylquinolone compound further includes: the aryl in $R_4$ is 4-pyridinyl, or

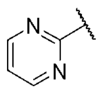.

**4.** The phenylquinolone compound according to claim 1, or a pharmaceutically acceptable salt, stereoisomer, deuteride, or prodrug thereof, **characterized in that**, the phenylquinolone compound is shown as follows:

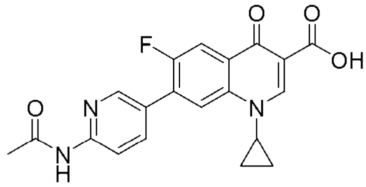 , 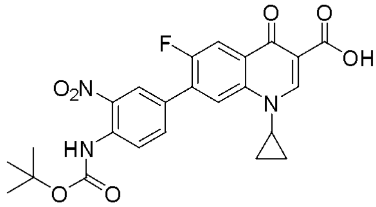 , 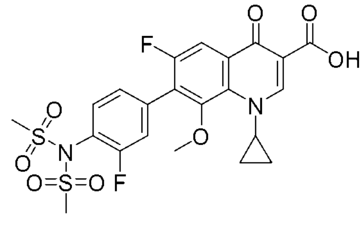 ,

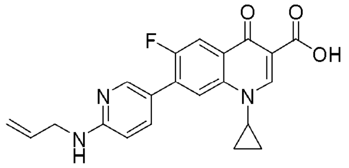 , 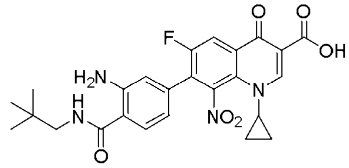 , 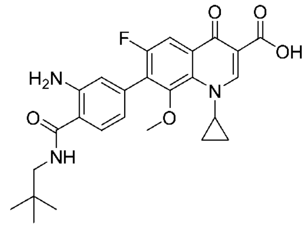 ,

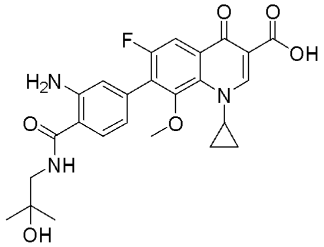 , 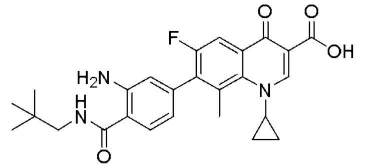 , 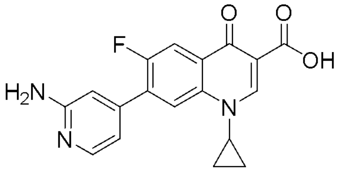 ,

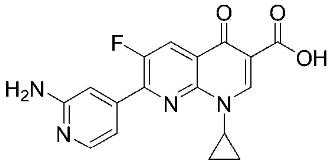 , 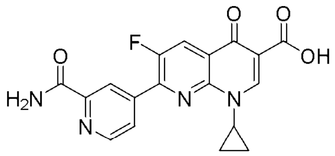 , 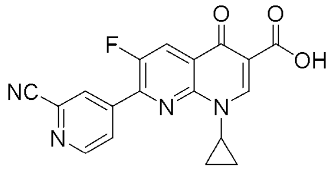 ,

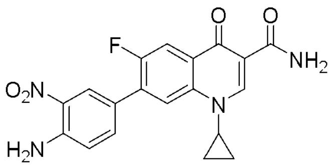 , 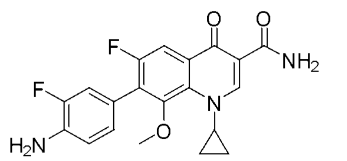 , 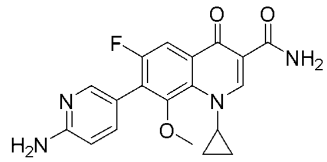 ,

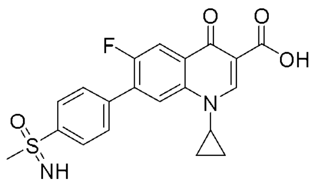 , 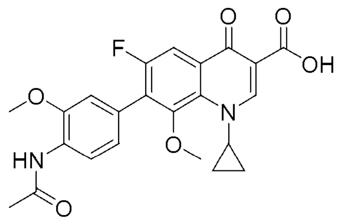 , 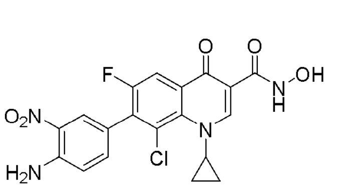 ,

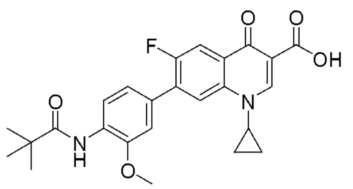 , 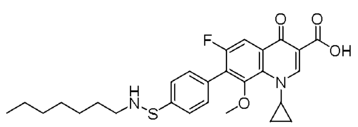 , 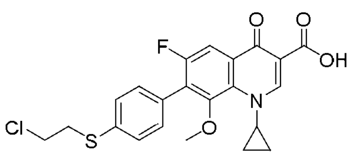 ,

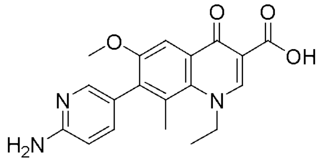 , 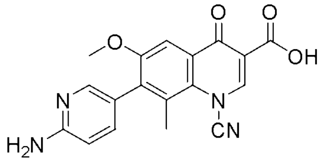 , 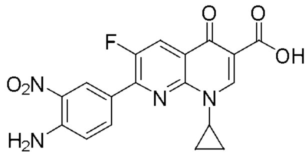 ,

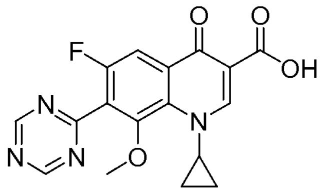 , 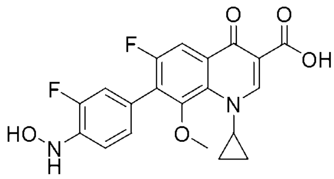 , 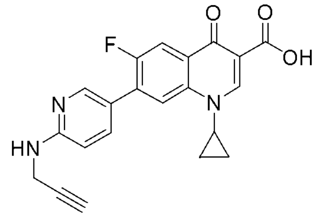 ,

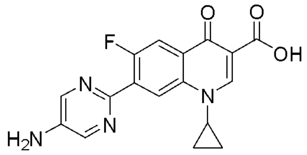 , 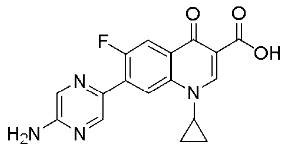 , 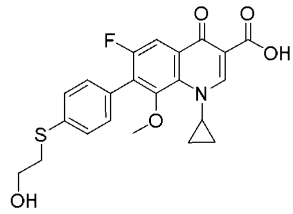 ,

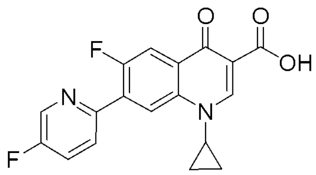 , 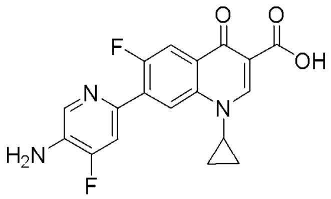 , 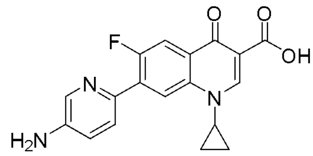 ,

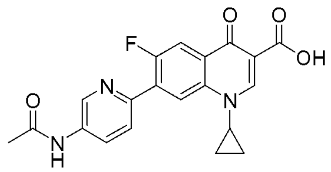 , 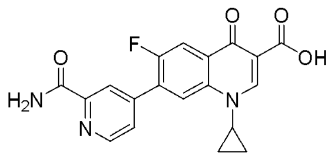 , 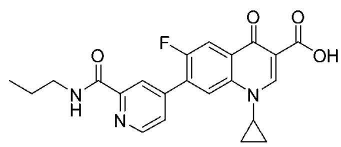 ,

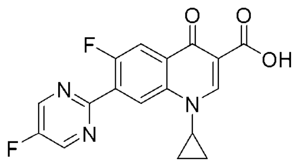 , 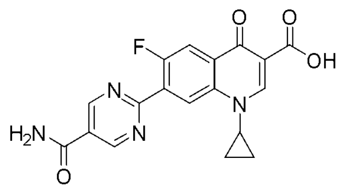 , 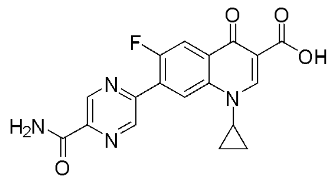 ,

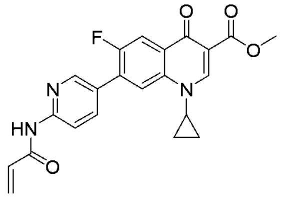 , 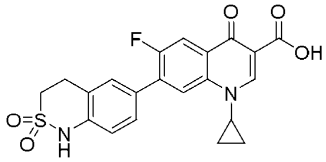 , 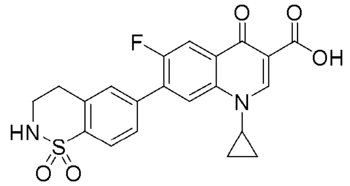 ,

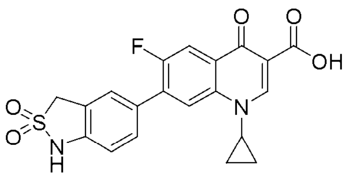 , 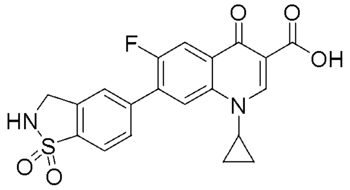 , 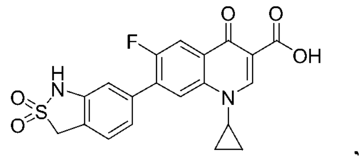 ,

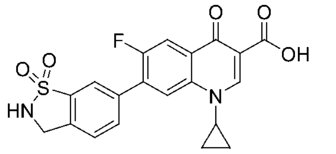 , 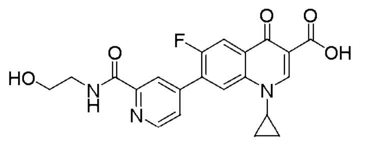 , 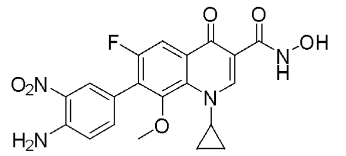 ,

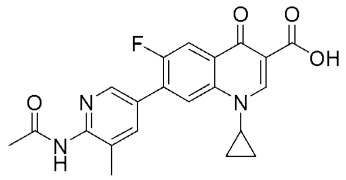 , 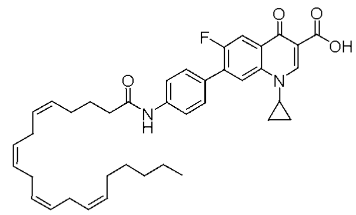 , 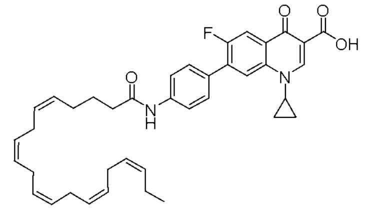 ,

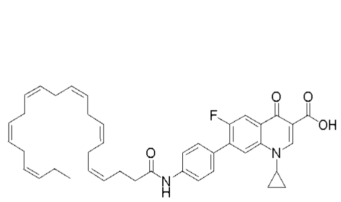 , 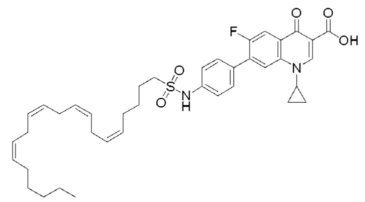 , 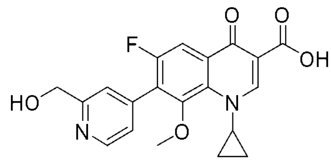 ,

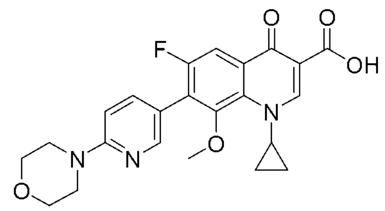 , 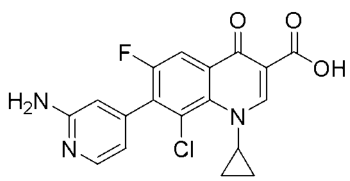 .

5. The phenylquinolone compound according to claim 1, or a pharmaceutically acceptable salt, stereoisomer, deuteride, or prodrug thereof, **characterized in that**, the pharmaceutically acceptable salt includes inorganic salt or organic salt; wherein, the inorganic salt includes sodium salt, potassium salt, magnesium salt, hydrochloride, hydrobromide, hydroiodate, perchlorate, sulfate, bisulfate, nitrate, phosphate, acid phosphate; the organic salt is selected from formate, acetate, trifluoroacetate, propionate, pyruvate, hydroxyacetate, oxalate, malonate, succinate, glutarate, fumarate, maleate, lactate, malate, citrate, tartrate, methanesulfonate, ethanesulfonate, benzenesulfonate, salicylate, p-toluenesulfonate, ascorbate.

6. A use of the phenylquinolone compound according to claim 1 or a pharmaceutically acceptable salt, stereoisomer, deuteride, or prodrug thereof in the manufacture of an antibacterial medicament or an anticancer medicament.

7. An antibacterial agent comprising the phenylquinolone compound according to claim 1 or a pharmaceutically acceptable salt, stereoisomer, deuteride, or prodrug thereof.

8. An antibacterial medicament comprising the phenylquinolone compound according to claim 1 or a pharmaceutically acceptable salt, stereoisomer, deuteride, or prodrug thereof.

9. An anticancer medicament comprising the phenylquinolone compound according to claim 1 or a pharmaceutically acceptable salt, stereoisomer, deuteride, or prodrug thereof.

**10.** The medicament according to claim 8 or claim 9, **characterized in that**, the medicament further includes one or more of the following pharmaceutical excipient(s): propellants, solubilizers, cosolvents, emulsifiers, color agents, adhesives, disintegrants, fillers, lubricants, wetting agents, osmotic regulators, stabilizers, glidants, flavoring agents, preservatives, suspending agents, coating materials, fragrances, anti-binders, permeation promoters, pH adjusters, buffers, plasticizers, foaming agents, defoamers, thickeners, inclusion agents, moisturizers, flocculants and anti-flocculation agents, filter aids and release blockers.

**11.** The medicament according to claim 8 or claim 9, **characterized in that**, the medicament further includes a pharmaceutical carrier selected from microcapsules, microspheres, nanoparticles and liposomes.

**12.** The medicament according to claim 9, **characterized in that**, the cancer includes: cervical cancer, Burkitt's lymphoma, acute T-lymphoblastic leukemia, non-small cell lung cancer, gastric cancer, colon cancer, pancreatic cancer, liver cancer, malignant melanoma, prostate cancer, ovarian adenocarcinoma, glioma, breast cancer, osteosarcoma, breast ductal carcinoma.

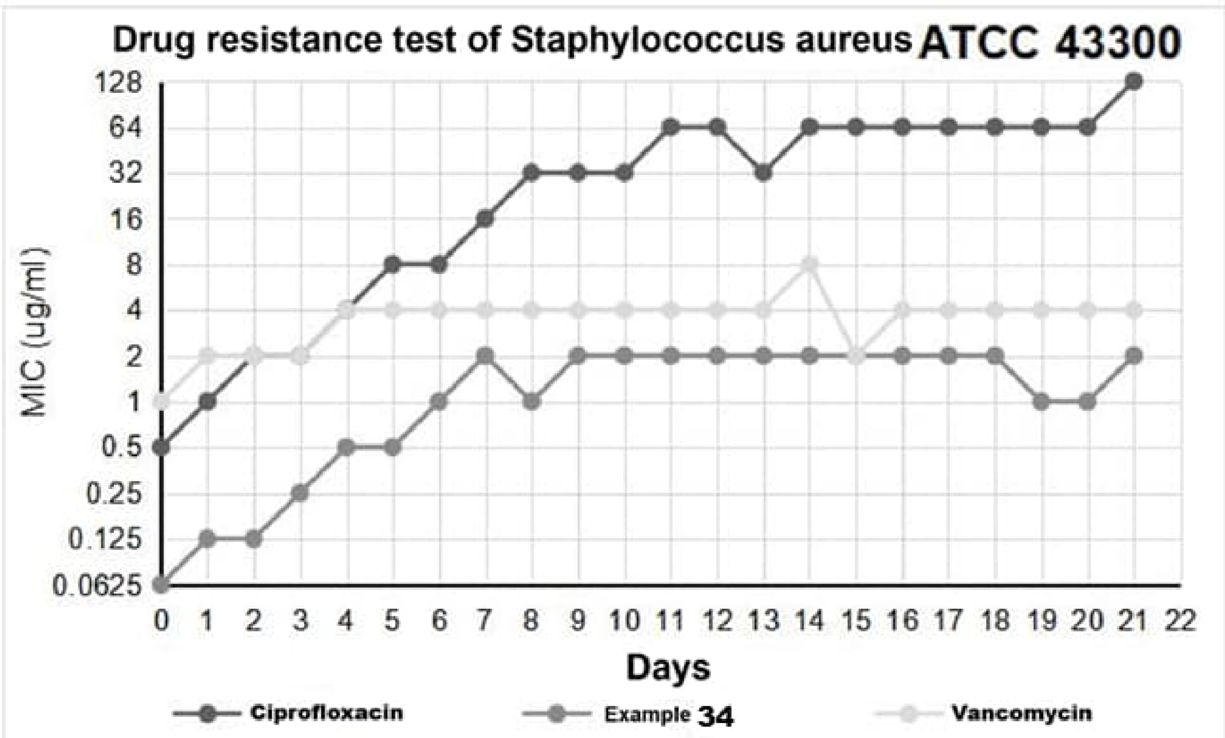
Drug resistance test of Staphylococcus aureus ATCC 43300
MIC (ug/ml)
128
64
32
16
8
4
2
1
0.5
0.25
0.125
0.0625
0 1 2 3 4 5 6 7 8 9 10 11 12 13 14 15 16 17 18 19 20 21 22
Days
Ciprofloxacin
Example 34
Vancomycin

Fig. 1

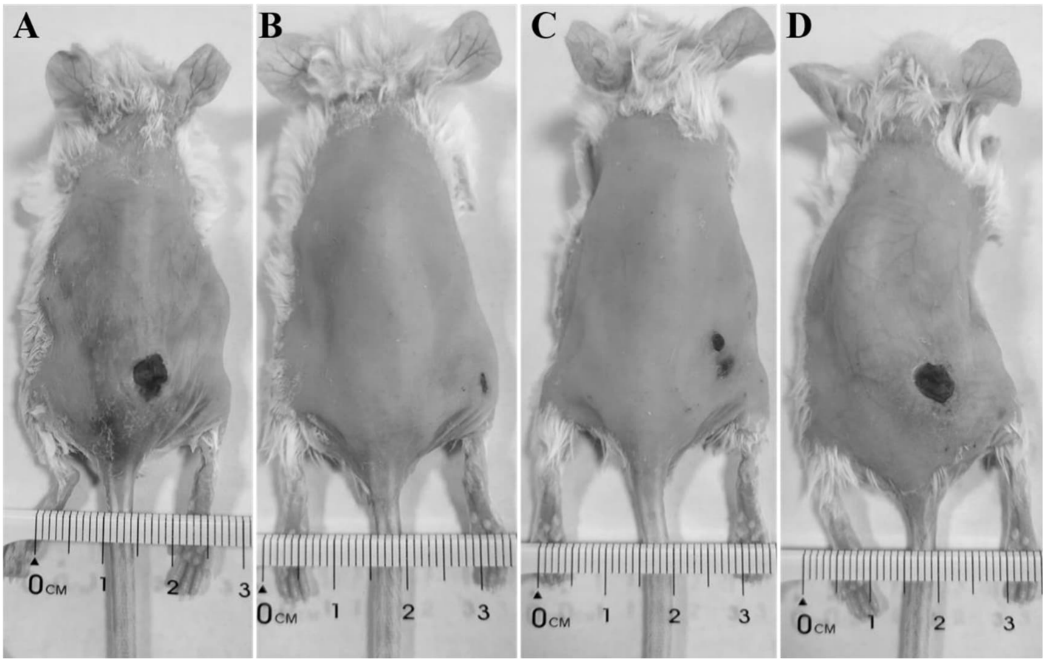
A
B
C
D

Fig. 2

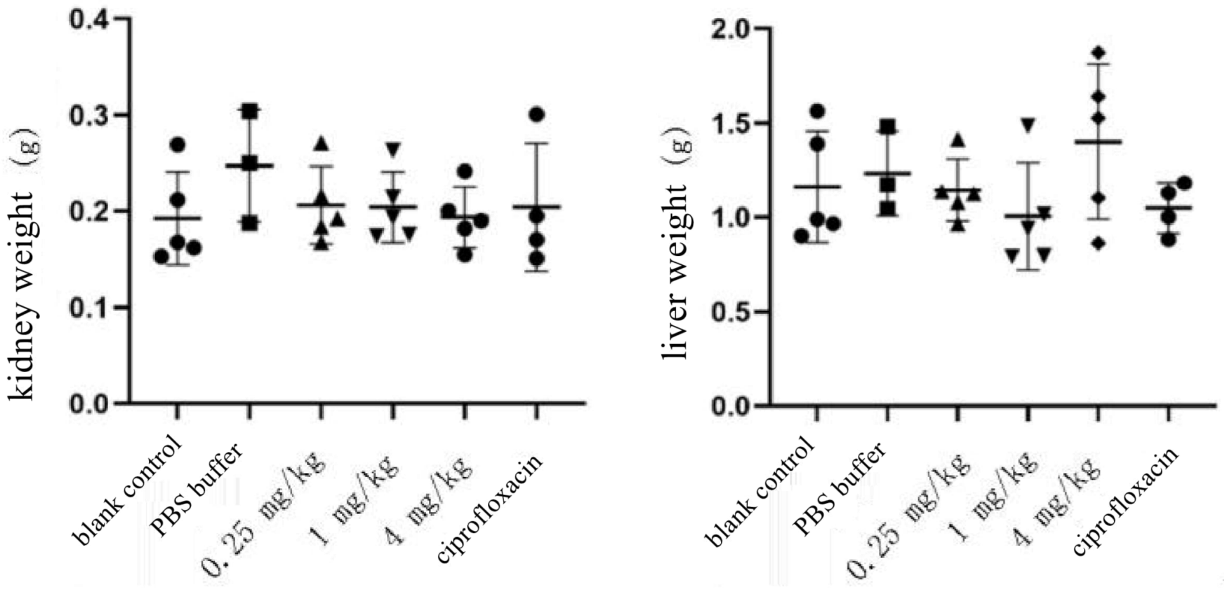
kidney weight（g）
0.4
0.3
0.2
0.1
0.0
blank control
PBS buffer
0. 25 mg/kg
1 mg/kg
4 mg/kg
ciprofloxacin
liver weight（g）
2.0
1.5
1.0
0.5
0.0

Fig. 3

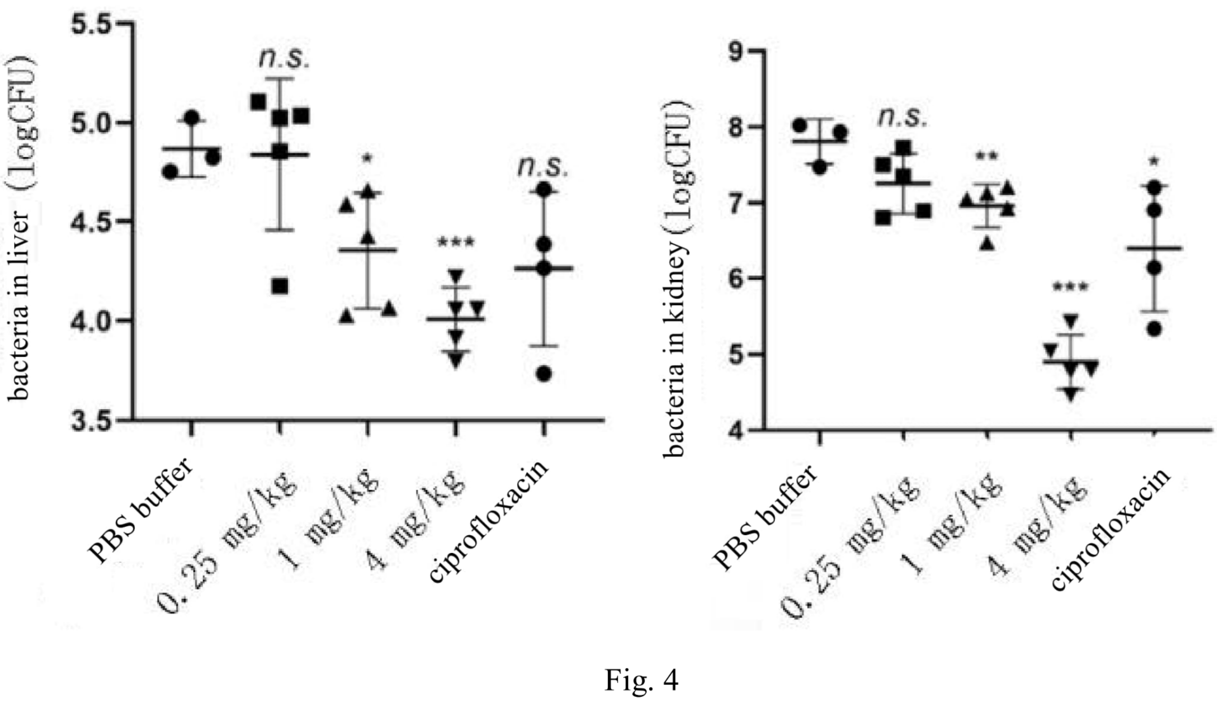
bacteria in liver（1ogCFU)
5.5
5.0
4.5
4.0
3.5
n.s.
*
***
n.s.
PBS buffer
0. 25 mg/kg
1 mg/kg
4 mg/kg
ciprofloxacin
bacteria in kidney(1ogCFU)
9
8
7
6
5
4
n.s.
**
***
*

Fig. 4

**INTERNATIONAL SEARCH REPORT**

International application No.
**PCT/CN2024/078241**

**A. CLASSIFICATION OF SUBJECT MATTER**

C07D215/56(2006.01)i; C07D471/04(2006.01)i; A61K31/47(2006.01)i; A61K31/435(2006.01)i; A61P31/00(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D A61K A61P

Documentation searched other than minimum documentation to the extent that such documents are included in the fields searched

Electronic data base consulted during the international search (name of data base and, where practicable, search terms used)

CNABS; DWPI; WPABS; CJFD; CNKI; CNTXT; ENTXT; ENTXTC; STN-REGISTRY; STN-CAPLUS; PATENTICS; 超星读秀, DUXIU; ISI-Web of Science: 喹诺酮, 喹啉, 苯, 抗菌, 细菌, 抗癌, 抗肿瘤, 癌, 肿瘤, carbostyril, quinolone, +phenyl+, antibiotic, antibiosis, bacteria, bacterium, anticancer, antitumour, antitumor, cancer, tumour, tumor

**C. DOCUMENTS CONSIDERED TO BE RELEVANT**

| Category* | Citation of document, with indication, where appropriate, of the relevant passages | Relevant to claim No. |
|---|---|---|
| X | CN 103764631 A (OTSUKA PHARMA CO., LTD.) 30 April 2014 (2014-04-30)<br>description, page 116, table, and paragraph 161, and claims 1 and 24-27 | 1-8, 10, 11 |
| X | EP 0184384 A1 (PFIZER) 11 June 1986 (1986-06-11)<br>description, embodiments 3 and 7, page 82, tables I, and claim 8 | 1-8, 10, 11 |
| A | CN 102040591 A (SHANDONG XUANZHU PHARMACEUTICAL TECHNOLOGY CO., LTD.) 04 May 2011 (2011-05-04)<br>entire document | 1-12 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 April 2024** | **20 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/078241**

| Patent document cited in search report | Publication date (day/month/year) | Patent family member(s) | Publication date (day/month/year) |
|---|---|---|---|
| CN 103764631 A | 30 April 2014 | US 2015239865 A1 | 27 August 2015 |
| | | US 9440951 B2 | 13 September 2016 |
| | | KR 20140139132 A | 04 December 2014 |
| | | EA 030867 B1 | 31 October 2018 |
| | | MY 165004 A | 28 February 2018 |
| | | JP 2014525420 A | 29 September 2014 |
| | | JP 6106174 B2 | 29 March 2017 |
| | | US 2020055823 A1 | 20 February 2020 |
| | | CA 2845459 A1 | 07 March 2013 |
| | | CA 2845459 C | 27 August 2019 |
| | | BR 112014000665 A2 | 10 January 2017 |
| | | BR 112014000665 B1 | 18 February 2020 |
| | | KR 20140087059 A | 08 July 2014 |
| | | KR 101996697 B1 | 04 July 2019 |
| | | US 2019077762 A1 | 14 March 2019 |
| | | US 2016340314 A1 | 24 November 2016 |
| | | NZ 620447 A | 26 February 2016 |
| | | EP 3318557 A2 | 09 May 2018 |
| | | EP 3318557 A3 | 11 July 2018 |
| | | JP 2017039733 A | 23 February 2017 |
| | | AU 2012303954 A1 | 20 February 2014 |
| | | AU 2012303954 B2 | 22 June 2017 |
| | | EP 2751083 A1 | 09 July 2014 |
| | | EP 2751083 A4 | 10 June 2015 |
| | | EP 2751083 B1 | 27 December 2017 |
| | | JP 2016027046 A | 18 February 2016 |
| | | JP 6039025 B2 | 07 December 2016 |
| | | EA 034787 B1 | 20 March 2020 |
| | | TW 201630900 A | 01 September 2016 |
| | | TWI 583680 B | 21 May 2017 |
| | | WO 2013029548 A1 | 07 March 2013 |
| | | IL 230559 B | 31 March 2019 |
| | | TW 201718506 A | 01 June 2017 |
| | | TW 201319053 A | 16 May 2013 |
| | | KR 20130095831 A | 28 August 2013 |
| | | KR 101560073 B1 | 13 October 2015 |
| | | ES 2660973 T3 | 26 March 2018 |
| | | US 2017362180 A1 | 21 December 2017 |
| | | US 2021032207 A1 | 04 February 2021 |
| | | JP 2018150324 A | 27 September 2018 |
| | | AU 2017203756 A1 | 22 June 2017 |
| | | AU 2017203756 B2 | 07 February 2019 |
| | | MX 371361 B | 27 January 2020 |
| | | US 2014179675 A1 | 26 June 2014 |
| | | US 2015005287 A2 | 01 January 2015 |
| | | US 9067887 B2 | 30 June 2015 |
| | | CN 105017151 A | 04 November 2015 |
| | | CN 105017151 B | 02 April 2019 |
| | | CN 103764631 B | 15 February 2017 |
| | | CN 105712976 A | 29 June 2016 |

Form PCT/ISA/210 (patent family annex) (July 2022)

**INTERNATIONAL SEARCH REPORT**
**Information on patent family members**

International application No.
**PCT/CN2024/078241**

| Patent document cited in search report | | | Publication date (day/month/year) | Patent family member(s) | | | Publication date (day/month/year) |
|---|---|---|---|---|---|---|---|
| EP | 0184384 | A1 | 11 June 1986 | DK | 562985 | D0 | 05 December 1985 |
| | | | | DK | 562985 | A | 07 June 1986 |
| | | | | HU | 195780 | B | 28 July 1988 |
| | | | | KR | 860004857 | A | 14 July 1986 |
| | | | | KR | 880000429 | B1 | 23 March 1988 |
| | | | | PH | 23499 | A | 16 August 1989 |
| | | | | PT | 81614 | B | 21 April 1988 |
| | | | | ES | 549601 | A0 | 16 April 1987 |
| | | | | IE | 853077 | L | 06 June 1986 |
| | | | | IE | 58270 | B1 | 25 August 1993 |
| | | | | FI | 854829 | A0 | 05 December 1985 |
| | | | | FI | 854829 | A | 07 June 1986 |
| | | | | FI | 83643 | B | 30 April 1991 |
| | | | | FI | 83643 | C | 12 August 1991 |
| | | | | EP | 0184384 | B1 | 02 August 1989 |
| | | | | AU | 5079285 | A | 12 June 1986 |
| | | | | AU | 562237 | B2 | 04 June 1987 |
| | | | | GR | 852923 | B | 07 April 1986 |
| | | | | CA | 1262249 | A | 10 October 1989 |
| | | | | JP | S61143365 | A | 01 July 1986 |
| | | | | US | 4623650 | A | 18 November 1986 |
| | | | | JP | 91066301 | B | 16 October 1991 |
| CN | 102040591 | A | 04 May 2011 | CN | 102040591 | B | 11 December 2013 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103764631 A
- CN 109422726 A